(19) Europäisches Patentamt
European Patent Office
Office européen des brevets

(11) **EP 1 661 994 A1**

(12) **EUROPEAN PATENT APPLICATION**

(43) Date of publication:
**31.05.2006 Bulletin 2006/22**

(21) Application number: **05023460.8**

(22) Date of filing: **09.04.1999**

(51) Int Cl.:
*C12N 15/12* (2006.01) *C12N 5/10* (2006.01)
*C07K 14/71* (2006.01) *C07K 14/48* (2006.01)
*C07K 16/28* (2006.01) *G01N 33/50* (2006.01)
*G01N 33/566* (2006.01) *C12Q 1/68* (2006.01)
*A61K 38/17* (2006.01) *A01K 67/027* (2006.01)
*G06F 17/30* (2006.01) *G06F 17/50* (2006.01)
*G06F 19/00* (2006.01)

(84) Designated Contracting States:
**AT BE CH CY DE DK ES FI FR GB GR IE IT LI LU MC NL PT SE**

(30) Priority: **09.04.1998 GB 9807781**

(62) Document number(s) of the earlier application(s) in accordance with Art. 76 EPC:
**99915913.0 / 1 068 315**

(71) Applicant: **The University of Bristol**
**Clifton,**
**Bristol BS8 1TH (GB)**

(72) Inventors:
- **Robertson, Alan George Simpson**
**Cambridgeshire CB7 5XU (GB)**
- **Allen, Shelley Jane**
**Bristol**
**BS1 3NY (GB)**
- **Dawbarn, David**
**Bristol**
**BS1 3NY (GB)**

(74) Representative: **Dean, John Paul et al**
**Withers & Rogers LLP**
**Goldings House,**
**2 Hays Lane**
**London SE1 2HW (GB)**

Remarks:
This application was filed on 27.10.2005 as a divisional application to the application mentioned under INID code 62.

(54) **Therapeutic agent for NGF**

(57) This invention relates to the use of a domain of Trk as a therapeutic agent and for screening purposes and rational design of NGF mimetics.

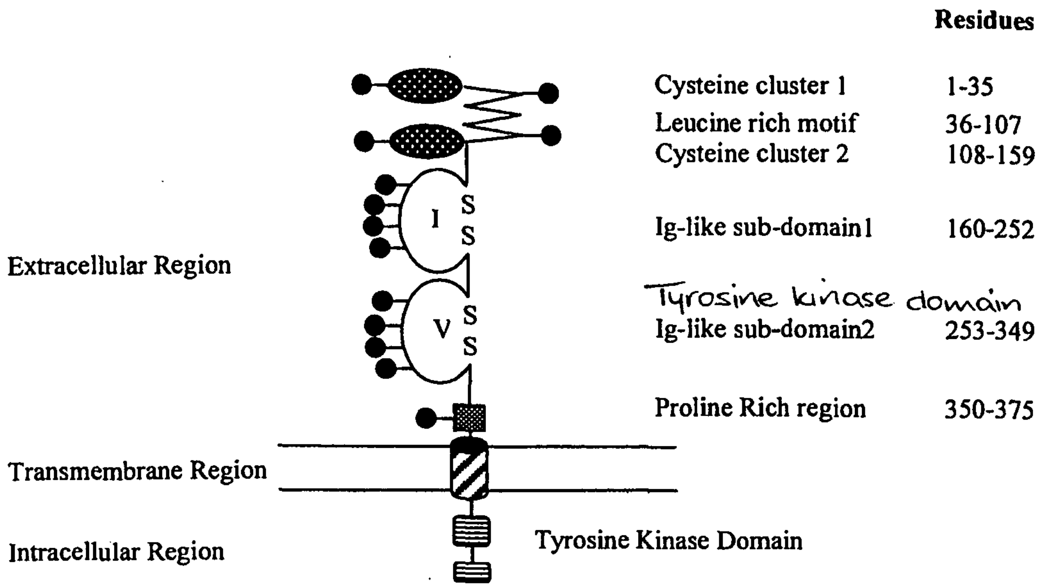


EP 1 661 994 A1

Fig. 1(B)

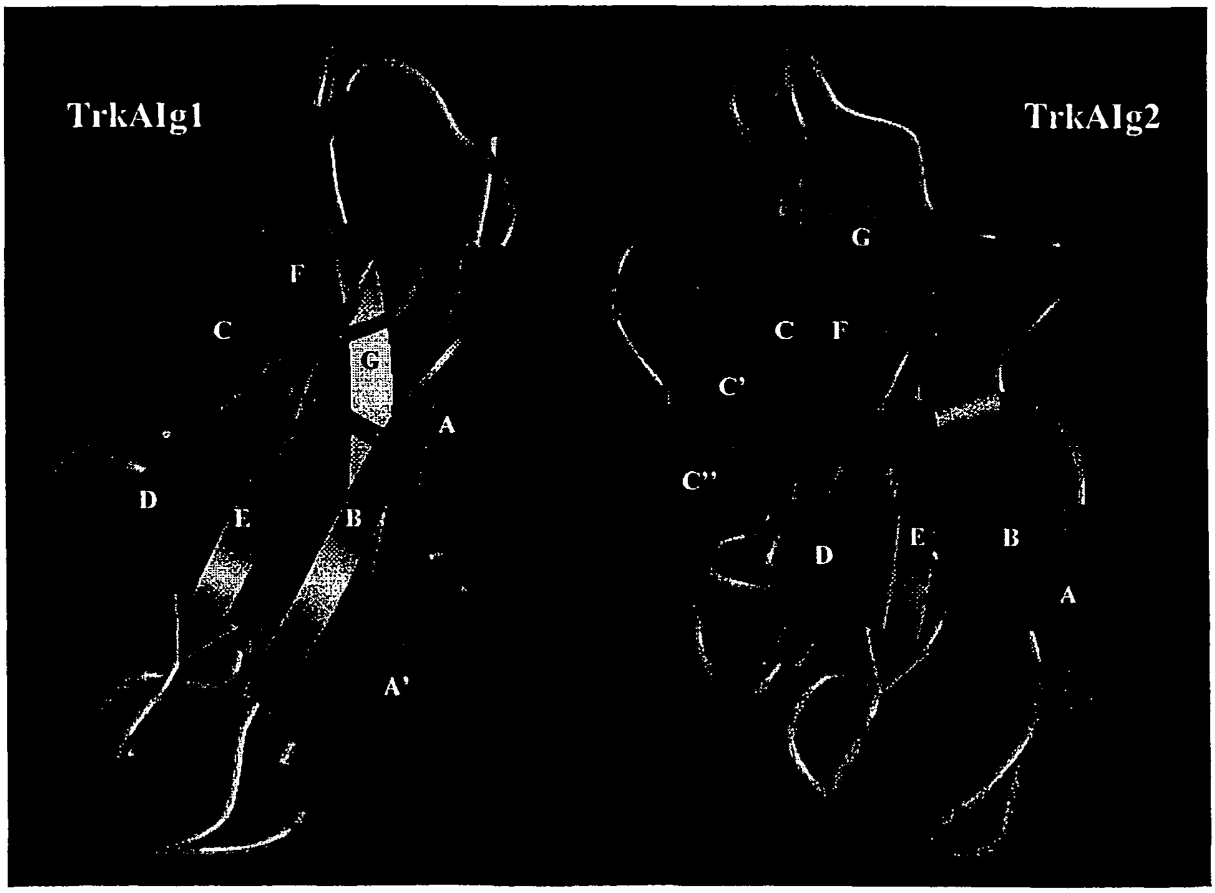

## Description

This invention relates to therapeutic agents and screening methods. In particular, the invention relates to the use of the Ig2 domain of the tyrosine kinase TrkA and fragments thereof in the treatment of disorders in which levels of neurotrophins, such as NGF, are elevated such as in pain disorders. It also relates to the use of the TrkAIg2 domain as a target for screening for compounds which act to antagonise or to mimic the actions of neurotrophins such as NGF. TrkAIg2 is defined here as including the TrkAIg-like sub-domain 2 together with the proline rich region (Fig. 1A).

Nerve Growth Factor (NGF) is a potent neurotrophic factor for forebrain cholinergic neurones and promotes the survival and differentiation of sympathetic and sensory neurones during development. In animal models it has been shown that administration of NGF is able to correct the effects of cholinergic atrophy in aged or lesioned animals. Purified mouse NGF has been used as a treatment for Alzheimer's disease. This treatment, however, requires invasive surgery and a long term solution would be the generation of small molecule agonists able to mimic the trophic actions of NGF. NGF usually exists as a dimer, however, for these purposes, the term NGF embraces monomeric dimeric, trimeric, or heterodimeric forms.

Evidence suggests that NGF may also act as a mediator of some persistent pain states (McMahon S.B. Series B-Biological Sciences, (1996), Vol.351, No.1338, 431- 440) by interacting with receptors on nociceptive primary afferents. In a variety of experimental inflammatory conditions NGF levels are rapidly increased in the inflamed tissue. Similarly, the systematic or local application of exogenous NGF produces a rapid and prolonged behavioural hyperalgesia in both animals and humans. In a number of animal models, much of the hyperalgesia associated with experimental inflammation is blocked by molecules which are able to sequester NGF, including antibodies. Therefore peripherally acting NGF - sequestering agents or NGF antagonists may potentially be used in treating some chronic pain states.

Peripheral inflammation is usually characterised by heightened pain sensitivity or hyperalgesia, which is the consequence of the release of inflammatory mediators, cytokines and growth factors. NGF seems to play a central role in pain mediation through its action on the TrkA receptors of a sub-group of the nociceptive sensory neurons of the dorsal root ganglion (DRG). In the adult this comprises some 40% of DRG cells. These neurons also express the peptides Substance P and calcitonin-gene related peptide (CGRP). By the action of NGF on TrkA receptors there results an increase in neuropeptide levels in these sensory neurons; in addition sodium and calcium channels are affected such that these neurons are increased in excitability. These actions may result in an increase in pain levels. Thus, NGF sequestering agents such as the TrkA extracellular domains may potentially be used to reduce these pain levels.

Under conditions of continual NGF up-regulation, chronic inflammation may lead to a persistent pain state. There are various models of chronic inflammation which involve exogenous administration of NGF or its upregulation. One such model (Woolf, C.J. *et* al. British Journal Of Pharmacology, (1997), Vol.121, No.3, 417- 424) is that induced by intraplantar injection of complete Freund's adjuvant in adult rats. This produces a localized inflammation of the hindpaw with elevation in the levels of TNF $\beta$, IL-1$\beta$ and NGF. TNF $\alpha$ injections have been reported to produce an increase in thermal and mechanical sensitivity which is attenuated by prior administration of anti-NGF antiserum. Carrageenan administration is known to cause a specific increase in NGF mRNA levels (of up to 500%) which is not seen for other neurotrophins such as NT-3 and BDNF.

In chronic inflammatory states the effects of consistently elevated levels of NGF may result in a long-term disabling pain state. Examples of this may be in some forms of bladder cystitis where raised levels of NGF have been found in biopsies (Lowe, E. M. *et al* British Journal Of Urology, (1997), Vol.79, No.4, 572-577 ). A rat model of human chronic cystitis, induced by administration of an irritant chemical can be treated, again by NGF sequestration, by administration of TrkA immunoadhesin (Dmitrieva, N. *et al* Neuroscience, (1997), Vol.78, No.2, 449-459 ). Systemic treatment with the NGF-sequestering molecule was able to partially and significantly reverse established inflammatory changes, by about 30-60%. The administration of exogenous NGF into the lumen of the urinary bladders of normal rats also has been shown to produce a rapid and marked bladder hyper-reflexia similar to that seen with experimental inflammation. It is also likely that chronically increased NGF levels may lead to both peripheral sensitization of nociceptors and central sensitization of dorsal horn neurons and perhaps even long-term sensory neuronal abnormalities (McMahon, S. B. Series B-Biological Sciences, (1996), Vol.351, No.1338, 431-440).

In arthritic synovial fluid, high levels of NGF have been observed. Transgenic arthritic mice have also been shown to have raised levels of NGF and an increase in the number of mast cells (Aloe, L. *et al* International Journal Of Tissue Reactions-Experimental And Clinical Aspects, (1993), Vol.15, No.4, 139-143). Purified NGF antibodies injected into arthritic transgenic mice cause a reduction in the number of mast cells, as well as a decrease in histamine and substance P levels within the synovium (Aloe, L. *et al.* Rheumatology International, (1995), Vol. 14, No.6,249-252).

It seems likely also that the postherpetic neuralgia (PHN), associated with the disorder shingles, may involve upregulation of NGF protein. Varicella-zoster virus (VZV) is an $\alpha$ herpes virus responsible for two human diseases: chicken pox in childhood (varicella), and shingles. The virus remains latent in dorsal root ganglia and may re-emerge later in life, taking advantage of the decline in immune function that occurs with aging. Reactivation causes herpes zoster, commonly known as shingles. The incidence of herpes zoster increases with advancing age. Pain, allodynia, and sensory loss in the affected dermatome are the central manifestations of the disorder. Severe pain is the major cause of acute and chronic morbidity in patients with herpes zoster. The chronic and often debilitating pain, PHN, is the most common complication of herpes zoster. Up to 50% of elderly patients who have had shingles may develop PHN. Antiviral agents appropriately administered systemically greatly relieve the pain of acute shingles, also antidepressants maybe useful; conventional analgesics however are generally of little use, though in a few patients some relief has been obtained with opioids, particularly methadone. The difficulty with testing the effects of anti-NGF treatment is that the model for shingles is not possible in the rat, there is only a cat model. However, it may be possible to investigate such treatments in human subjects, with the potential for reduction of NGF levels and alleviation of associated pain.

Chronic inflammatory conditions are widespread and current therapies are severely limited. For instance it is estimated that arthritis affects 37.9 million people and interstitial cystitis 450,00 people in the United States. In a study of rheumatoid arthritis, more than 80% of the patients were in severe pain despite the fact that the majority were taking analgesics. Similarly, there is no effective therapy for interstitial cystitis, which is characterised by painful bladder symptoms.

NGF is one of a family of neurotrophins involved in the development and maintenance of the peripheral and central nervous system. NGF may be isolated from various sources, most particularly from male mice salivary glands. It may be isolated first as 75 NGF, named for its sedimentation coefficient, which is a complex of β-NGF and γNGF. 2.5S NGF may be obtained from this. 2.5S NGF is known to be responsible for the neurotrophic biological activity of the complex. 2.5S NGF is βNGF but often partially proteolysed at the amino and carboxy termini. The other members include for example BDNF, NT-3 and NT-4. All of the neurotrophins bind to a common receptor p75NGFR. Each also binds to one of a homologous family of tyrosine kinase receptors: NGF binds to TrkA, BDNF and NT-4 bind to TrkB, and NT-3 binds to TrkC. NT-3 can also bind TrkA and TrkB with reduced affinity.

Although the three dimensional structure of the TrkA extracellular domain is unknown, distinct structural motifs in the sequence have been characterised (Figure 1A). The Trk extracellular domain comprises three tandem leucine rich motifs (LRM), flanked by two cysteine cluster regions, followed by two immunoglobulin-like (Ig-like) domains. Based on sequence homology with the neural cell adhesion molecule and the platelet derived growth factor (PDGF) receptor, the Ig-like domains have previously been classified as belonging to the C2 class of the immunoglobulin superfamily (IgSF) (Williams, AF, and Barclay AN (1988) Ann Rev Immunol 6, 381-405). Numerous studies have defined neurotrophin residues which interact with p75NGFR and Trk receptors but little is known about the Trk residues which are involved in binding the neurotrophins.

Recently two groups have shown that the Ig-like domains of the Trk receptors play important roles in the binding of neurotrophin ligands and receptor activation. Perez P. *et al* (Molecular and Cellular Neuroscience **6**: 97-105 (1995)) concluded that both of the Ig-like domains are important for the binding of NGF to TrkA. Urfer, R. *et al* (EMBO J. **14** p2795-2805 (1995)) concluded that the second Ig-like sub-domain and proline rich region, Ig2 (Figure 1A) provide the main contacts for NGF binding.

The extracellular domain of TrkA is 375 amino acids long. The inventors have recently shown that a protein comprising the two immunoglobulin-like domains and proline-rich region (amino acids 160-375) alone are able to bind NGF with a similar affinity to that of the complete extracellular domain (Holden P. H *et al* (1997) Nature Biotchnology **15:** 668-672). This region has been defined here as TrkAIg1,2. Surprisingly, the inventors have found that an even smaller domain of TrkA referred to as TrkAIg2 (amino acids 253-375) is able to bind NGF with a similar affinity to the complete extracellular domain or the TrkAIg1,2 region and is thus responsible primarily for its binding properties.

The inventors have demonstrated that the recombinant Ig-like domains are able to bind neurotrophins such as NGF with high affinity and inhibit the biological activity of NGF *in vitro* and *in vivo.* In particular, TrkAIg2 as defined by amino acids 253-375, (Figure 1A) is the major contributor to NGF binding. The inventors have used molecular modelling techniques to model the TrkAIg1 and TrkAIg2 domains. Surprisingly, they find that TrkIg2 - like sub-domain 2 is not of the C2 class but of the V set of Ig-like domains (Figure 1B).

This gives rise to several uses for TrkAIg2 and polypeptides derived therefrom. Structural data from co-crystals of TrkAIg2-NGF will identify the residues in TrkA which are involved in binding NGF. This will enable rational design of neutrophin, particularly NGF, mimetics. Immobilised TrkAIg2 can be used as a target for phage display libraries as well as combinatorial chemical libraries and fungal extracts. This will allow for selection of molecules able to bind TrkA and thus either act as agonists or antagonists at the receptor. A third use of TrkAIg2 is as a therapeutic agent for a number of chronic pain states. NGF is particularly important for peripheral sensory neurones, evidence suggests that NGF may act as a mediator of some persistent pain states by interacting with receptors on nociceptive primary afferents and that peripherally acting NGF antagonists may be of use in treating some chronic pain states such as rheumatoid arthritis, interstitial cystitis and shingles.

A first aspect of the invention provides a polypeptide comprising the amino acid sequence of residues 22 to 119 of Fig. 4(B) or a portion of the amino acid sequence of Figure 4(B), and which binds a neurotrophin. Preferably, the polypeptide consists of the whole sequence of amino acids 22-144 of Figure 4(B). The polypeptide may be TrkAIg1,2 or a portion thereof. Such a polypeptide may be produced by chemical or biological means.

We exclude the full coding sequence of natural TrkA.

The polypeptide may be derived from animal cells. More preferably, the polypeptide is selected from mammalian cells, and in particular, may be selected from human cells. Alternatively, the polypeptide may be selected from avian cells including chicken cells or reptile or amphibian or fish or insect.

Preferably, the neurotrophin is NGF, NT-3, or a neurotrophin which binds p75 NGFR. Such a neurotrophin may exist in a monomeric, dimeric, trimeric or heterodimeric form, and may be from a mammalian, such as a human.

A second aspect of the invention provides a DNA sequence encoding a polypeptide according to a first aspect of the invention; or variants of such a DNA sequence due to the degeneracy of the genetic code, or insertion or deletion mutants thereof that encode a polypeptide according to a first aspect of the invention, and DNA sequences which hybridise to such a DNA sequence. This DNA sequence may be inserted into a plasmid or other vector such as pET15b.

A further aspect of the invention provides a complex comprising a polypeptide according to a first aspect of the invention in combination with at least one neurotrophin or neurotrophin subunit, such as NGF or NT-3.

A further aspect of the invention provides a method of producing a polypeptide according to a first aspect of the invention comprising introducing a DNA sequence according to a second aspect of the invention into a suitable host and cultivating that host whereby the TrkAIg2 is expressed. A suitable host may be selected from animal cells such as bacterial cells, insect cells and mammalian cells, particularly human cells.

Further, the TrkAIg2 may be conveniently used as a target for a high throughput screen for molecules which bind to the TrkA receptor using a polypeptide according to a first aspect of the invention. Such a method may involve the use of phage or peptide display libraries, combinatorial chemical libraries and fungal extracts, and ELISA techniques.

A futher aspect of the invention comprises comparative binding of a putative ligand to at least a portion of TrkAIg1 with its binding to at least a portion of TrkAIg2. Such methods may involve selecting molecules which bind to at least one solvent exposed loop of TrkAIg2, such as the E to F loop or C" to D loop as shown in Fig. 1(B). The molecules selected may enhance the binding of a polypeptide according to a first aspect of the invention, or at least a portion of TrkA in its natural state, to a neurotrophin.

A further aspect of the invention provides a method of combinatorial chemistry comprising generating compounds and screening the compounds using their binding affinities to a polypeptide according to a first aspect of the invention.

A further aspect of the invention comprises an antibody raised against a polypeptide according to a first aspect of the invention, particularly TrkAIg2.

A further aspect of the invention comprises a host cell containing a polypeptide according to a first aspect of the invention carried on a plasmid. Such as host cell may be mammalian (including human), bacterial, insect, yeast, avian, amphibian, fish or reptilian.

A further aspect of the invention comprises a diagnostic probe comprising a portion of a polypeptide according to a first aspect of the invention. The probe may be labelled with a fluorescent tag or radiolabel.

A further aspect of the invention comprises diagnostic tests, assays, or monitoring methods using a polypeptide according to a first aspect of the invention, particularly in the detection of elevated neurotrophin levels.

A further aspect of the invention comprises an organism engineered to express a polypeptide according to a first aspect of the invention.

A further aspect of the invention comprises a method of treating a subject with pain associated with increased neurotrophin polypeptide levels, the method comprising supplying to the subject a pharmaceutical composition comprising a polypeptide according to a first aspect of the invention, or an NGF analogue isolated or identified by a screeening procedure as described above.

The pain may be a symptom of ISU, interstitial cystitis, arthritis, shingles, peripheral inflammation, chronic inflammation, or postherpetic neuralgia.

A further aspect of the invention comprises a treating a subject of Alzheimer's disease comprising supplying to the subject a pharmaceutical composition comprising a polypeptide according to a first aspect of the invention, or a composition comprising a neurotrophin analogue isolated or identified by a screening procedure involving a polypeptide according to a first aspect of the invention.

A composition comprising a polypeptide according to a first aspect of the invention can be used to reduce free NGF levels in a subject.

All references above to neurotrophin embrace NGF and NT-3.

A further aspect of the invention includes a homology model having the coordinates shown in Fig. 21, and machine readable data storage medium on which such a homology model has been stored, and a computers programmed with, or arranged to provide such a homology model.

A further aspect of the invention provides crystalling TrkAIg2.

A further aspect of the invention provides compounds obtained by a method as mentioned above, using a computer as mentioned above, or using a machine readable data storage medium as mentioned above.

A further aspect of the invention comprises a crystal comprising a polypeptide according to a first aspect of the invention, particularly a TrkAIg2 polypeptide.

The invention will now be described, by way of example only, with reference to the accompanying drawings Figures 1 to 20 in which

Fig. 1 (A) is a schematic representation of the TrkA structure (the filled circles represent consensus glycosylation sites);

Fig. 1(B) shows a modelled structure for TrkAIg1 and TrkAIg2; the most important binding determinates probably occur in the loop connecting strands E and F (the EF loop).

Fig. 2(A) is a restriction map of the plasmid pET15b;

Fig. 2(B) shows the sequence of oligonucleotides used to amplify TrkAIg1,2.

Fig. 3 shows the nucleotide sequence of the insert of pET15b-TrkAIg1,2 and its derived amino acid sequence;

Fig. 4(A) shows the nucleotide sequence and derived amino acid sequence of his TrkAIg1;

Fig. 4(B) shows the nucleotide sequence and derived amino acid sequence of his TrkAIg2;

Fig. 4(C) shows the TrkAIg2 domain of a splice variant of TrkA including the six amino acid insert in the proline-rich region able to bind NT-3;

Fig. 5 is a gel illustrating expression of TrkAIg1,2, TrkAIg1 and TrkAIg2;

Fig. 6(A) is a gel illustrating purification of TrkAIg2;

Fig. 6(B) is a gel illustrating purification of TrkAIg1;

Fig. 7(A) shows an elution profile of TrkAIg1 from Poros 20HQ after refolding;

Fig. 7(B) shows an elution profile of TrkAIg2 from Poros 20HQ after refolding

Fig. 8 shows a Circular Dichroism spectrum of TrkAIg2. The molecular ellipticity (θ) is shown as a function of wavelength.

Fig. 9 shows competitive binding Assay for TrkAIg1,2 and TrkAIg2; The axis is given in logarithmic scale as 1 x $10^{-11}$ to 1 x $10^{-5}$ M.

Fig. 10 shows surface plasmon resonance (SPR) of NGF binding to Immobilised TrkAIg2;

Fig. 11 illustrates the results of binding experiments where TrkAIg2 (2μM) and TrkAIg1 (2μM) were incubated separately with a standard curve of βNGF (0-1000pM);

Fig. 12 illustrates the results of binding experiments where increasing concentrations of βNGF (1-200μM) were incubated separately with 2μM TrkAIg1 or 2μM TrkAIg2;

Fig. 13 shows the effect of TrkAIg2 on NGF dependent neurite outgrowth on PC 12 cells.

Fig. 14 A to F illustrates the effect of co-injected TrkAIg1,2 on NGF-induced plasma extravasation;

Fig. 15 illustrates the effect of 5 minute pre-treatment with TrkAIg1,2 on NGF- induced plasma extravasation;

Fig. 16 illustrates the effect of 40 minute pre-treatment with TrkAIg1,2 on NGF-induced plasma extravasation;

Fig. 17 illustrates the effect of co-injected TrkAIg1 on NGF-induced plasma extravasation;

Fig. 18 illustrates the effect of co-injection of TrkAIg2 on NGF-induced plasma extravasation;

Fig. 19 illustrates the effect of 5 minute pre-treatment with TrkAIg2 on NGF-induced plasma extravasation;

Fig. 20 illustrates the effect of 40 minute pre-treatment with TrkAIg2 on NGF-induced plasma extravasation.

Fig. 21 shows the coordinate data for the model of Fig. 1 (B).

**Structure prediction of the extracellular domain of TrkA and modelling of the Ig-like domains:**

Secondary structure analysis of the Ig-like regions using PredictProtein (Rost B. and Sander C. (1993) PNAS **90:** 7553-7562; Rost B. and Sander C. (1993) J. Mol. Biol. 232: 584-599; Rost B. and Sander C. (1994) Proteins **19:** 55-72) showed defined stretches of β-strands. The first Ig-like sub-domain, TrkAIg1, consists of residues 160-252 (Fig. 1A) in the mature extracellular domain of TrkA, while the second Ig-like sub-domain consists of residues 253-349 (Fig. 1A). There is also a proline rich region at residues 349-375 (Fig. 1A).

For TrkAIg1, two known proteins (parents) were identified as homologues from which a model could be built. These are 2NCM (domain 1 of mouse NCAM) and 1 VCA (domain 1 of human vascular cell adhesion molecule). Both domains are I-set Ig domains and have 32% and 29% sequence identity, respectively, with the target sequence. 2NCM was identified as the most suitable parent on which to base the model, apart from residues 38-50 connecting β-strand C to D where the smaller loop found in 1 VCA was used (Figure 1B).

For TrkAIg2, two parents were identified as homologues from which a model could be built. These are 1TNM (titin module M5) and 1HNG (CD2 domain 1). The homologues are quite distantly related at 21% and 14% sequence identity and belong to the Ig-set I family and the V set family respectively. However, certain key features of the Ig fold can be identified including a disulphide bridge and a Trp in the C strand. This is surprising since both homologues lack a disulphide bond. These homologues show higher sequence identity in different regions, hence a chimeric model was built using 1TNM as the main template and 1HNG being used to model residues 39-59 (Figure 1B) and the coordinate data is shown in Fig. 21.

Following slight manual interventions in the sequence alignment the inventors have elucidated a model containing 8 β-strands with strands (ABDE) in one sheet and (A'CFG) in the other sheet. Together they form the β-sandwich for TrkAIg1. For TrkAIg2, the A' strand is absent and two extra strands C' and C" are predicted with the β-sandwich formed by β-strands (ABDE) and (GFC'C"). For domain 1, the alignment mapped the disulphide between strands B and F across the β-sandwich to the same position as found in 2NCM. This disulphide also superimposed onto the 1 VCA disulphide between residues 23-71. Conversely for domain 2, a disulphide is predicted on the surface of the molecule bridging two adjacent β-strands, B and E, the second Cys aligns with a Ser in 1TNM. This disulphide bond arrangement is similar to the model predicted by Urfer *et al* (Urfer, R., Tsoulfas, P., O'Connell, L., Hongo, J.A., Zhao, W. and Presta, L.G. (1998). J. Biol.Chem. Urfer *et al. (supra)* **273:** 5829-5840) modelled on 1VCA domain 1 although our TrkAIg2 model predicts nine β-sheets, of the V-set, in contrast with the model with seven β-sheets in a I-set arrangement. The modelled structures are shown in Figure 1B and the co-ordinate data is shown in DATA.1.

In terms of the structural model built here for TrkAIg2 the parents used in model construction, titin module M5 (1tnm) and CD2 domain 1 (1hng) are clearly distant homologues, that can be identified by sensitive sequence search methods (Barton, G.J. (1993) Comput. Appl. Biosci. **9**: 729-734; Henikoff, S. and Henikoff, J.G. 1991. Nucleic Acids Research **19**: 6565-6572). The VCAM domain 1 used to model build TrkAIg2 by Urfer *et al.* (Urfer, R., Tsoulfas, P., O'Connell, L., Hongo, J.A., Zhao, W. and Presta, L.G. (1998) JBC 273: 5829-5840 is not significantly related by sequence, however, is homologous by virtue of being an Ig-fold. Relative to titin and VCAM (both I-set domains) the TrkAIg2 sequence has a significant insertion (~10 residues) between strands C and D. The region corresponding to positions 39-59 which includes this insert has more significant homology to CD2 domain 1 than other Ig domains. Furthermore, the predicted secondary structure (Rost B. and Sander C. (1993) PNAS **90**: 7553-7562) of TrkAIg2 in this region corresponds to the existence of two extra strands (C' and C") in accordance with the CD2 structure. This results in a predicted V-set domain as opposed to the I-set domain proposed by Urfer *et al.* (Urfer, R., Tsoulfas, P., O'Connell, L., Hongo, J.A., Zhao, W. and Presta, L.G. (1998). JBC 273: 5829-5840)

The importance of key residues in binding NGF can be understood by reference to our model and the extensive mutational analysis of TrkAIg2 by Urfer *et al.* (Urfer, R., Tsoulfas, P., O'Connell, L., Hongo, J.A., Zhao, W. and Presta, L.G. 1998. J. Biol.Chem. **273:** 5829-5840). The most important binding determinants in TrkAIg2 occur in the loop connecting strands E and F (the EF-loop) with single mutations T319A, H320A and N323A exhibiting greater than 100-fold reduction in binding. Reference to our structural model indicates that all three residues are in solvent exposed locations near the apex of the EF-loop. Minor contributors to loss in binding affinity also occur in the spatially adjacent AB-loop with mutations H258A, V261E, M263A and H264A. The first three residue locations are in solvent exposed locations on the surface of this loop. Only two other mutations exhibit greater than 50-fold reduction in binding affinity, these are P269E and H310A. These two residues are spatially adjacent to one another in our model and in close proximity to the disulphide bridge (C267-C312) connecting strands B and E. It is possible these residues play a direct role in binding NGF as suggested by Urfer *et al.* (Urfer, R., Tsoulfas, P., O'Connell, L., Hongo, J.A., Zhao, W. and Presta, L.G. 1998. J. Biol.Chem. **273:** 5829-5840). However an alternative explanation may be their importance in maintaining the structural integrity of the disulphide bridge. Unlike the conserved core disulphide bond of canonical Ig domains the solvent exposed disulphide bridge may not be important in stabilising the structure of the domain, however, the covalent link between strands B and E may be important in maintaining the conformation of the AB and EF loops in binding. Indeed the loss of the disulphide with mutations C267A or C312A results in a 10 to 30-fold reduction in binding, underlining the importance of the disulphide bridge in the binding mechanism.

An alternatively spliced form of TrkA containing a six amino acid insert (at amino acid position 224-225 (Fig. 3)) in the proline rich domain, VSFSPV, shows a higher affinity for NT3 and therefore may be important for ligand binding (Clary, D. O & Reichardt L. F. (1994) PAISA **91:** 11133-11137). This sequence is also found in the rat TrkA sequence and a similar sequence is found in the chicken TrkA. There is also a similar of polar residues in all of the TrkB sequences (Allen S. J. *et al.* (1994) Neuroscience **60**: 825-834).

It is therefore possible that this region may contribute to the binding of the neurotrophins or to the receptor's specificity.

The TrkAIg1,2 region is generally considered as comprising amino acids 160-375 of the mature extracellular domain of TrkA (Fig. 1A), TrkAIg1 or TrkAIg like sub-domain 1, as comprising amino acids 160-252 and including TrkAIg - like subdomain 2 as amino acids 253-349. TrkAIg2 here comprises amino acids 253-375 the proline rich region. In all cases the use of variants of TrkA and its sub domains such as those described above are embraced by the present invention.

**Construction of TrkAIg2 with the Insert from the Alternatively Spliced Variant:**

TrkAIg2 with the insert from the alternatively spliced variant was created by PCR mutagenesis. The mutagenesis was done in two stages. First the 5' and 3' fragments were amplified such that there is an overlap encoding the sequence of the alternative spliced form of TrkA. In the second stage, the PCR products of the 5' and 3' fragments were spliced together using the overlapping sequence and the two flanking primers. The first round of PCR involved oligo66816 (ATCATATGCC GGCCAGTGTG CAGCT) and oligo49234 (CCACTGGCGA GAAGGAGACA GGGATGGGGT CCTCGGGG) to produce the 5'-fragment and oligo49233 (GTCTCCTTCT CGCCAGTGGA CACTAACAGC ACATCTGG) and the T7 terminator primer (GCTAGTTATTGCTCAGCGG) to produce the 3'-fragment. The products were then purified and used as target for a second round of PCR using oligo66816 and T7terminator primer. The PCR product from the second round of PCR was then cloned into pET15b and expressed in the same way as TrkAIg2.

**Sub-cloning of TrkAIg1,2:**

From the secondary structure prediction data, it was decided to subclone the DNA encoding amino acids 160 to 375 (Fig.1A) of the extracellular domain of TrkA. Oligonucleotide primers (10692 and 10693) were designed that would provide appropriate restriction sites in order that the TrkAIg1,2 insert would be in-frame with the poly-histidine tag of the expression vector, pET15b (Novagen) and two stop codons to terminate translation. A map of pET15b and the sequence of the oligonucleotide primers is shown in Figure 2.

Amplification by PCR was then carried out using the primers oligo 10692 and oligo 10693 (Cruachem Ltd) and the full-length Human TrkA cDNA clone (a gift from David Kaplan, Montreal Neurological Institute, Canada) as target. The PCR product was then ligated into the plasmid pCRII (Invitrogen), to give pCRII-TrkAIg1,2. pCRII-TrkAIg1,2 was then digested with *Xho*I and the insert purified from a low-melting point agarose gel by phenol extraction and ligated into pET15b (Novagen) previously prepared by digesting with *Xho*I and dephosphorylating using Calf-Intestinal Alkaline Phosphatase (CIAP). After transformation into *Escherichia coli* XL1Blue (Stratagene), transformants were screened by PCR using the T7 promoter primer which anneals to pET15b and oligo10693. In this way, clones were identified which had the TrkAIg1,2 insert in the correct orientation for expression from the T7 promoter. The resulting clone, pET15b-TrkAIg1,2 was sequenced from the T7 promoter primer and the T7 terminator primer to ensure that the insert had ligated to the pET15b at the *Xho*I site. The DNA sequence of the insert of pET15b-TrkAIg1,2 and the derived amino acid sequence are shown in bold in Fig. 3 (amino acids 24-239, nucleotides 71-718). Enzymes and enzyme buffers were obtained from Boerhinger.

**Sub-cloning of TrkAIg1:**

An oligonucleotide primer was designed which would allow amplification of the TrkAIg1 domain using the left primer for TrkAIg1,2 such that the PCR product could be ligated into the XhoI site of pET15b in-frame with the poly-histidine tag.

**oligo36770** *Right Primer For TrkA Ig1;*

cg**ctcgag** tta tca GAAGGAGACGTTGACC
*Xho*I STOP STOP

Amplification by PCR was then carried out using oligo 10692 and oligo36770 with pET15b-TrkAIg1,2 as target. The PCR product was then ligated into pCRII (Invitrogen) to give pCRII-TrkAIg1 which was then digested with *Xho*I and subjected to low melting point agarose gel electrophoresis. The insert was then purified and ligated into pET15b previously digested with *Xho*I and treated with CIAP. After transformation into *E. coli* XL1Blue, transformants were screened by PCR using oligo10692 and the T7 terminator primer. The resulting clone pET15b-TrkAIg1, was then sequenced to ensure that the reading frame of TrkAIg1 was in-frame with the poly-histidine tag of pET15b. Figure 4a shows the nucleotide sequence (residues 71-349) and deduced amino acid sequence (residues 24-116) of TrkAIg1, in bold.

**Sub-cloning of TrkAIg2:**

An oligonucleotide primer was designed which would allow amplification of the TrkAIg2 domain using the T7 terminator primer of pET15b-TrkAIg1,2;

**oligo66816** *Left Primer For TrkA Ig2*;
at**catatg**CC GGCCAGTGTG CAGCT
*Nde*I

Amplification by PCR was then carried out using oligo66816 and the T7 terminator primer with pET15b-TrkAIg1,2 as the template DNA. The PCR product was then digested with *Nde*I and *Bam*HI and ligated into pET15b previously prepared by digestion with the same enzymes and treated with CIAP. Transformants were screened by PCR using the T7 promoter primer and oligo10693 and the positive clones were sequenced. Figure 4b shows, in bold, the nucleotide sequence (residues 65-433) and derived amino acid sequence (residues 22-144) of TrkAIg2.

**Hybridisation to TrkA DNA sequence**

DNA encoding TrkAIg1,2 or TrkAIg2 (sequences according to Figures 3, and 4B) may be used for a hybridization assay. A DNA sequence encoding TrkAIg1,2 or TrkAIg2 or portions of such a sequence may be obtained by reverse transcriptase PCR of genomic DNA or directly by PCR or restriction digest from the cDNA for TrkA. DNA or RNA which is complimentary to the DNA encoding TrkAIg1,2 or TrkAIg2 or portions of such a sequence, or a sequence which is similar in composition but contains a degeneracy of sequence, may be hybridized to the DNA prepared above. Such a sequence is referred to herein as a probe. Usually, the complimentary DNA or RNA is tagged by radioactive or non-radioactive substances.

One example of this is the northern analysis of TrkAIg2 using a radioactively labelled cDNA probe. A cDNA probe is random primed (Stratagene, CA) with $^{32}$P-dATP (6000Ci/mmol; Dupont NEN). The probe is then purified using a Nuctrap column (Stratagene), to a specific activity in the region of 2 x $10^6$ cpm/ng. Chinese hamster ovary cells (CHO) expressing TrkA are then homogenised in Ultraspec™ (Biotecx, Houston Texas) and total RNA extracted. The RNA is loaded onto a 1% denaturing agarose gel and seperated by electrophoresis, before being blotted onto Hybond N (Amersham, Cardiff, UK) overnight and baked for 2 hours at 80°C. These Hybond N filters are pre-hybridized for 4 hours at 65°C by revolving in hybridization buffer (6SSC, 5 x Denhardts, 0.5% SDS and 0.002% acid cleaved salmon sperm DNA), in a hybridization oven. The probe is then denatured for 5 minutes at 100°C, before being added to fresh hybridisation solution. Filters are then hybridized under these conditions of high stringency, overnight at 65°C. Stringency may be varied according to degeneracy of probe or homology of target. Lower temperatures such as 50°C, and higher salt concentrations, such as 20x55C, will allow for lower stringency. The presence of formamide decreases the affinity of nucleic acid binding and allows for variance in stringency. Such strategies are well described (e.g. Nucleic acid hybridisation, a practical approach edited by Hames and Higgins, IRL Press 1988). The next day, the filters are washed in 2 x SSC/ 0.5% SDS and washed twice for 30 minutes at 65°C in Hybaid with 2 x SSC/ 0.5% SDS. The filters are then dried and exposed to Hyperfilm (Hyperfilm MP, Amersham) overnight, at -70°C, and developed the following day. DNA probes which have bound to RNA encoding the TrkAIg2 sequence are visualised as exposed, black, areas of the autoradiographic film.

A further example of this is the detection of expression of TrkAIg1,2 or TrkAIg2, or a similar sequences in an expression library. A λGT10 human brain cDNA library (M Goedert, Cambridge) is used to infect *E. coli* c600 cells. These are plated onto 24cm x 24cm agar plates to give 10,000pfu per plate. A plaque lift is then carried out by laying Nylon membrane Hybond N (Amersham, Cardiff, UK) onto the agar plate for 1 minute. The filter is then placed, DNA side up, on denaturing solution (1.5N NaCl, 0.5N NaOH) for 30 sec, before being immersed for 2 minutes. The filter is then immersed into neutralising solution (1.5N NaCl, 0.5N Tris-HCl pH 8.0) for 5 min. Immersion is repeated in fresh neutralising solution. The filter is then rinsed briefly in 2 X SSC (0.3N NaCl, 0.03N $Na_3$Citrate, pH 7.0) and placed on filter paper which is baked at 80°C for 2 hours. Hybridization is carried out as described above. The position of DNA probes which have bound to plaques encoding the TrkA sequence is visualised as exposed, black, areas of the autoradiographic film. These exposed, black areas can be re-aligned to the plates to identify positive clones expressing sequences similar to TrkAIg1,2 or TrkAIg2 or a portion of such a DNA sequence.

Hybridisation may also occur using homologous PCR techniques. Specific or degenerate oligonucleotides corresponding to a region in the sequence for TrlcAIg1,2 may be used to amplify a portion of the sequence as described for example, in the section entitled 'sub-cloning of TrkAIg2'. Such hybridization assays may be used as tools to detect the presence of TrkAIg 1,2 or TrkAIg2 sequences, or portions thereof, in diagnostic kits.

**Expression of TrkAIg1,2, TrkAIg1 and TrkAIg2:**

Competent BL21(DE3) cells were transformed with the above vector and expression was carried out using a variation on the method described in the pET (Novagen) manual for difficult target proteins. Briefly, 2 ml of 2YT broth (containing 200mg/ml carbenecillin) was inoculated with a colony and grown at 37°C to mid log phase. Cells were not centrifuged and resuspended in 2YT (as in manual) but used directly to inoculate 50 ml of 2 YT broth (containing 500 mg/ml carbenecillin) and grown at 37°C to mid log phase. The cells were not harvested by centrifugation and resuspended but used directly to infect 5 litres of 2 YT (containing 500 μg/ml ampicillin). Once an $OD_{600}$ of 1 was reached the cell culture was induced by the addition of IPTG to a final concentration of 1 mM and the cells were grown for a further 2 hrs at 37°C. Figure 5 shows a 15% SDS PAGE gel of extracts of cultures of BL21(DE3) containing the various pET15b-TrkAIg constructs. Further analysis of the cell extracts revealed that for all of the constructs, the expressed TrkAIg protein was insoluble. Several attempts were made to express the TrkAIg protein in the soluble fraction, but were unsuccessful. However, the fact that the TrkAIg proteins were insoluble faciliated in their purification.

**Purification and Refolding of TrkAIg1,2:**

The harvested cells were resuspended in 10% glycerol, frozen at -70°C and the pellet was passed 3 times through an Xpress (BioX, 12 ton psi). The lysed cells were washed with 20 mM Tris-HCl (pH 8.0) and centrifuged for 30 min at 10,000 rpm at 4,°C until all soluble matter was removed, leaving inclusion bodies containing insoluble protein. The purified inclusion bodies were solubilised in 6M urea buffer (20 mM Tris-HCl pH 8.5, 1 mM β-mercaptoethanol) at approximately 0.1 mg/ml protein and incubated on ice with gentle shaking for 1 hour. Refolding was carried out by dialysis against 400x buffer (20 mM Tris-HCl, 100 mM NaCl, pH 8.5) for 24 hrs at 4°C, with one buffer change. The refolded TrkA-Ig1,2 protein was loaded onto a 1ml Resource Q (Pharmacia) column and eluted with a linear gradient of 0-1M NaCl in 20 mM Tris-HCl over 40mls at 2 mls per minute. The main peak as detected at 280 nm (using a UV detector) was collected and affinity purified according to the Novagen His column purification protocol using a 2.5 ml disposable column of His-bind resin (Novagen). Finally, the eluted protein was re-applied to the Resource Q column to remove imidazole. This was eluted with a 10 ml salt gradient of 0-1m NaCl in 20 mM Tris buffer pH 8.0.

**Purification of TrkAIg1 and TrkAIg2:**

The harvested cells were resuspended in 10% glycerol, frozen at -70°C and the pellet was passed 3 times through an Xpress (BioX). The extract was then centrifuged at 10,000 rpm, 4°C for 30min to pellet the insoluble inclusion bodies. The inclusion bodies were then washed in 50 ml 1%(v/v) Triton X-100, 10 mMTrisHCl pH8.0, 1 mM EDTA followed by 50 ml 1M NaCl 10mMTrisHCl pH8.0, 1 mM EDTA and finally 10 mM TrisHCl pH8.0, 1 mM EDTA. The inclusion bodies were then solubilised in 20 mM Na Phosphate, 30 mM Imidazole, 8 M Urea pH7.4. The solubilised inclusion bodies were then clarified by centrifugation before loading on a 5 ml HisTrap column (Pharmacia). The column was washed with 50 ml 20 mM NaPhosphate, 30 mM Imidazole, 8 M Urea pH7.4 and the purified TrkAIg1 and TrkAIg2 eluted with 25 ml 20 mM NaPhosphate, 300 mM Imidazole, 8 M Urea pH7.4 at 2 mls/minute (Figure 6(A) and 6(B)).

**Refolding of TrkAIg1 and TrkAIg2:**

The purified TrkAIg proteins were adjusted to a concentration of 0.1 mg/ml in 20 mM NaPhosphate, 30 mM Imidazole, 8 M Urea pH7.4 with the addition of 1 mM β-mercaptoethanol and dialysed against 20 mM TrisHCl, 50 mM NaCl, pH8.5 for TrkAIg2 and 20 mM TrisHCl, 50 mM NaCl pH9.0 for TrkAIg1 (2x100 volumes). The dialysed proteins were loaded onto a 1.6 ml Poros 20HQ column and eluted with a linear gradient of 0.05-1 M NaCl over 20 column volumes (Figure 7).

Three peaks were eluting from the Poros 20HQ column for TrkAIg2, all of which gave a band corresponding to TrkAIg2 (data not shown). Therefore the refolding process must result in three species of TrkAIg2, all of which have a different conformation. Displacement binding studies reveal that the first peak to elute binds NGF while the others do not. The first peak was therefore collected, glycerol added to a final concentration of 20% (v/v), and snap frozen in liquid nitrogen before storage at -70°C.

For TrkAIg1, only two peaks elute from the Poros 20HQ column with more protein in the flow through. Again SDS page of each peak and the flow through show that TrkAIg1 is the only protein present. Displacement binding assays of the two peaks show that neither of these species of TrkAIg1 bind to NGF (data not shown).

**Circular Dichroism Studies on TrkAIg2**

To determine the secondary structure content of the folded protein, far-UV circular dichroism (CD) measurements were made. The CD of proteins is primarily the CD of the amide chromophore, which begins absorbing far into the UV region with the first band at about 220 nm. Antiparallel β-sheet structures typically display a negative Cotton effect with a minimum near 218 nm and a positive effect with a maximum around 195 nm. The amplitude of the far-UV spectra of different immunoglobulins such as light chain variable (VL) and constant (CL) domains also show a minimum around 215-218 nm. Similar results were therefore expected with the TrkAIg proteins.

CD spectra were recorded at room temperature on a Jobin Yvon CD6 instrument using a cuvette of 0.5mm path length at a protein concentration of 40μM. Ten scans were accumulated with a scan speed of 0.5nm/s. Spectra were averaged and the small signal arising from the buffer was subtracted. The CD of the active TrkAIg2shows a minimum at 218nm and a maximum near 200nm (Figure 8). This is typical of anti-parallel β-sheet, which display a negative Cotton effect with a minimum near 218nm and a positive Cotton effect with a maximum at around 195nm (Yang, J.T., Wu, C.S.C. and Martinez, H.M. (1986). Methods Enzymol. **130:** 208-269). Similar results have been reported for other immunoglobulin domains (Ikeda, K., Hamaguchi, K. and Migita, S. (1968) J. Biochem. **63:** 654-660) and for TrkAIg1,2 (Holden, P.H., Asopa, V., Robertson, A.G.S., Clarke, A.R., Tyler, S., Bennett, G.S., Brain, S.D., Wilcock, G.K., Allen, S.J., Smith, S. and Dawbarn, D. (1997) Nat. Biotechnol 15: 668-672). These results are consistent with the model of TrkAIg2 shown in Figure 1B.

Thus the CD data indicates that TrkAIg2 eluting first from the Poros 20HQ column is folded into a compact structure and is likely to have a similar structure to the other immunoglobulin domains.

**The binding of NGF to Immunoglobulin-like Domains of TrkA**

**1 Competitive Binding**

The binding affinity of $^{125}$I-NGF to the Ig-like domains of TrkA was determined by a competitive binding assay using the melanoma cell line A875 American Tissue Culture Collection (ATCC) which expresses the NGF receptor $p75^{NGFR}$.

Purified recombinant human NGF was radioiodinated with $I^{125}$ using a lactoperoxidase method and equilibrium binding with [$^{125}$I]-NGF was carried out (Treanor *et al.,* 1991; *Neuroscience Letters* **121** p73-76). Briefly A875 cells ($10^6$ per ml) were incubated with [$^{125}$I]-NGF (0.14 nM) and serial dilutions of unlabeled human NGF (concentration range: $10^{-6}$ M to 1 x $10^{-11}$M), TrkAIg1,2 (concentration range: 4 x $10^{-6}$ M to 1 x $10^{-11}$ M) or TrkAIg2 (concentration range 5 x $10^{-6}$ M to 1 x $10^{-11}$m). Tubes were shaken vigorously at room temperature for 1 hr. 100 μl aliquots were then layered over 200 μl sucrose (0.15 M in binding buffer) in Beckman tubes. After centrifugation (15 seconds at 20,000 g) bound and free [$^{125}$I]-NGF were separated by freezing the tubes in liquid nitrogen and determining the bound [$^{125}$I]-NGF of the cell pellet. Binding reactions were carried out in triplicate. Counts were corrected for background and specific binding was between 85-87%a of total binding. The competitive binding assay (figure 9) allowed estimation of the binding affinity of [$^{125}$I]-NGF to the recombinant TrkAIg2 protein. A range of concentrations of Ig-like domains are incubated with $^{125}$I-NGF and A875 cells (Vale R. D. & Shooter E. M (1985) Methods in Enzymology **109:** 21-39). This results in a competition between the TrkAIg domains and the $p75^{NGFR}$ for available $^{125}$I-NGF. Two competing equilibria are:

$$\overset{Kd1}{N + R \rightleftharpoons N{:}R} \text{ and } \overset{Kd2}{N + T \rightleftharpoons N{:}T}$$

where N represents NGF; R the $p75^{NGFR}$ cell receptor and T the TrkAIg2 domain.

The data represent the NGF bound to the cell at varying TrkAIg2 concentrations, as a fraction of that bound in the absence of TrkAIg2. Owing to the high affinity of NGF for the $p75^{NGFR}$ cellular receptor, the analytical solution to the curve is complex thus data were fitted using numerical simulation (FACSIMILE, U.K.A.E.A).

The fitted value for the dissociation constant for the TrkAIg1,2/NGF interaction ($K_d2$) was 3.3 nM (Holden *et al.*, 1997; Nature Biotechnology **15** p668-672). This agrees well with a $K_d$ of between 0.1 and 1.0 nM. for NGF binding to ectopically expressed TrkA in mammalian cells. The $IC_{50}$ (concentration of cold NGF required to inhibit $^{125}I$-NGF by 50%) for unlabelled (cold) NGF was 0.2nM (Holden, P. H *et al.* (1997) Nature Biotechnology **15:** 668-672) (Figure 4B).

Results show that TrkAIg2 binds NGF with a similar affinity to TrkAIg1,2 (Fig. 9). The $IC_{50}$ for TrkAIg2 is only three-fold higher than that of TrkAIg1,2, indicating a very similar affinity for NGF. This surprising result indicates that the major contribution to binding within TrkAIg1,2 is found in the second Ig domain, TrkAIg2.

**2 Surface Plasmon Resonance Studies:**

Kinetic data of the binding of NGF to TrkAIg2 was obtained using a BiaCore-X. Biacore technology allows real-time measurements of rate constants using very low amounts of protein. Briefly, varying concentrations of sample (analyte) are flowed across a sensor chip to which the protein of interest (the ligand) has been bound. As the analyte binds to the ligand there is a change in the electron density on the surface of the sensor chip which affects the intensity and wavelength of light absorbed by the surface.

Since the data from competitive binding assays indicated that TrkAIg2 was the major contributor to NGF binding, this domain was further investigated.

TrkAIg2 was covalently attached to the surface of the sensor chip by coupling with amine groups on TrkAIg2 to carboxyl groups on the surface using BiaCore Amine Coupling kit and varying concentrations of NGF passed over at a constant flow rate of 20 μl/min for two minutes. Data were collected for a range of NGF concentrations of 1 μM to 1 nM. It was found that at the high concentrations and at the very low concentrations, the data became difficult to interpret possibly due to aggregation of the NGF at the high concentrations and to non-specific interactions with the surface at very low concentrations. However, data collected for the range 40 nM to 500 nM could be successfully evaluated. Using the fitting software, BiaEval 3.0, a Kd of 11.8 nM was obtained. The $K_d$ value of 11.8 nM obtained is consistent with the fact that the $IC_{50}$ for TrkAIg2 is three fold higher than that of TrkAIg1,2 given that the $K_d$ for TrkAIg1,2 binding to NGF is 3.3 nM as determined by competitive binding assay.

In addition, 20 μM BDNF was also passed over the TrkAIg2 with negligible observed binding. It is clear that as well as being the main contributor to the NGF binding capability of TrkA, TrkAIg2 is also specific for NGF.

**3 Binding of TrkAIg-like domains using the ELISA Technique**

**Method 1**

Anti-βNGF (Sigma polyclonal rabbit anti mouse NGF, 1:1000) diluted in Coat I Buffer (50 mM sodium carbonate pH 9.6, NaN3 0.1%) is plated (50 μl per well) onto 96 well plates and left overnight at 4°C. Wells were emptied and 100 μl per well Coat II Buffer (Coat I plus 1% BSA) was added. After 2 hours at 4°C, the plate was washed 3 times using Wash Buffer (50 mM Tris HCl pH 7.2, 200 mM NaCl, 0.1% Triton X-100, 0.1% $NaN_3$, 0.25% gelatin) and samples and standard curve of NGF (0-1000pg/ml) diluted in Sample Buffer (Wash buffer plus 1% BSA) were added (50 μl per well). Samples had been pre-incubated with varying concentrations of TrkAIg-like domains for ten minutes with shaking at room temperature before adding to the plate. The plate was left one hour at room temperature before washing 3 times with Wash Buffer, anti βNGF galactosidase conjugate (Boerhinger: 2.5-20mU and 5-10ng antibody per assay) diluted (1:40) in wash buffer (50 μl per well was added). The plate was incubated for 2 hours at room temperature and then washed 3 times with Wash Buffer before adding 50 μl of substrate (200 mM of 4-methyl umbelliferyl galactoside (4-MUG)) in Substrate Buffer (100 mM sodium phosphate pH 7.3, 1 mM MgCl2). The production of a fluorescent product (4-methylubelliferone) from 4-MUG was then measured using a fluorimeter at excitation wavelength 364 nm, emission at 448 nm.

**Method 2**

The assay is similar to that of method 1 except that the TrkAIg1,2 domain was plated directly onto the 96 well plate in Coat I Buffer and left overnight at 4°C. The wells were then emptied and Coat II Buffer added for 2 hours at 4°C. A standard curve of βNGF (0-200 nM) was preincubated for 10 minutes at room temperature with 2 μM TrkAIg1 or 2 μM TrkAIg2 and added to the plate. This was incubated at room temperature for one hour before washing and the addition of anti βNGF galactosidase conjugate. The plate was then incubated for 2 hours at room temperature and washed with Wash Buffer before adding substrate (200 mM of 4-MUG). The production of a fluorescent product was then measured using a fluorimeter at an excitation wavelength of 364 nm, emission at 448 nm..

The TrkAIg1 had no effect on NGF binding to the anti-βNGF antibodies on the plate indicating that they were not sequestering NGF in the pre-incubation. By contrast the TrkAIg2 bound to 22% of the NGF at 0.5 nM and 38% at 1 nM NGF (Figure 11)

TrkAIg2 was able to sequester NGF and thus less NGF was available for binding to the TrkAIg 1,2. The binding was lowered by 40% at 200 nM NGF. TrkAIg1 was not able to sequester NGF and therefore the binding to TrkAIg1,2 was unaffected (Figure 12).

These results show that TrkAIg2 will bind to NGF resulting in a lowering of NGF concentration available for binding to a 96 well plate. TrkAIg1 is not able to do this. The preceding protocols describe a choice of methods whereby high throughput screening of non-peptide or peptide databases may be carried out on a 96 well plate format. Competition by unknown ligands with NGF for binding to plated TrkAIg-like domains may be measured by diminution of fluorescence.

**In Vitro Effects of TrkAIg-like Domains on NGF-Induced Neurite Outgrowth By PC12 Cells**

PC12 (derived from a transplantable rat adrenal phaeochromocytoma, ECACC No. 88022401) cells grown in the presence of 4 ng NGF (Fig. 13A) differentiate and produce neurites after 72 hrs. This does not occur in the absence of NGF (Fig. 13B). TrkAIg2 added to PC12 cells in the presence of 4 ng NGF at 2.5 μM (Fig. 13C), 1.25 μM (Fig. 13D) and 0.625 μM (Fig. 13E) inhibits neurite outgrowth. Only when the TrkAIg2 concentration is reduced to 0.312 μM (Fig. 13F) does neurite outgrowth start to appear.

Results show that the TrkAIg2 domain is able to inhibit neurite outgrowth of PC12 cells by sequestration of NGF (Fig. 13) whereas TrkAIg1 is not able to do this.

**In Vivo Effects of TrkAIg-like domains: Inhibition of Plasma Extravasation**

**Inhibition of NGF activity *in vivo***

All *in vivo* experiments were carried out according to the Animals (Scientific Procedures) Act 1986 under terminal anaesthesia. Plasma protein extravasation in rat skin induced by intradermal (i.d.) NGF was measured by the extravascular accumulation of intravenous (i.v.) $^{125}$I-human serum albumin (Brain, S A and Williams T. J. (1985) British Journal of Pharmacology 86: 855-860) Male Wistar rats (200-350 g) were anaesthetised with 60 mg/kg intra peritoneal (i.p.) with maintenance doses (15 mg/ml) as necessary. The dorsal skin was shaved and marked out for injection of test substances according to a balanced, randomized plan with two sites per test agent. The rats received $^{125}$I-human serum albumin (100 kBq) and Evans Blue dye (0.2-0.5 ml of 2.5 % w/v in saline) i.v. via the tail vein at the start of the accumulation period. NGF and other test agents (in Tyrodes buffered salt solution) were then injected i.d. and accumulation allowed over a 30 min period. A blood sample was taken by cardiac puncture (for plasma) and the rats killed by cervical dislocation. The dorsal skin was then removed and injection sites punched out (16 mm diameter). Plasma and skin sites were counted in a gamma counter. The plasma protein extravasation at each site was expressed as volume of plasma extravasated.

For co-injection experiments, all skin sites received 100 μl (i.d.) of either NGF (8 pmol) or Tyrode (with or without TrkAIg1,2, TrkAIg1 or TrkIg2). For pretreatment experiments, skin sites received 100 μl (i.d.) of either TrkAIg1,2 TrkAIg1 or TrkIg2 (24 or 80 pmol) or vehicle (Tyrode solution) at -5 or -40 min. These sites then received 50 μl (i.d.) NGF (8 pmol) or Tyrode at start of accumulation period (0 min).

**The effect of TrkAIg1,2 on NGF-induced plasma extravasation.**

The effect of co-injection of TrkAIg1,2 on NGF-induced plasma extravasation is shown in Fig. 14. Results are expressed as plasma extravasated (μl/site) in response to intradermal test agent, mean ± s.e.mean, n = 6. The response induced by 7S NGF(7S NGF is a complex of 2.55 (β-NGF) and γ NGF), both alone and with co-injection of TrkAIg1,2, is shown (8 pmol, filled squares). For comparison, the response induced by Tyrode's solution (vehicle, open circles), alone and with co-injection of TrkAIg1,2 is also shown. Plasma extravasation in sites receiving agent plus co-injected TrkAIg1,2 differing significantly from the sites receiving agent alone are shown as ** $p < 0.01$, as assessed by ANOVA with Bonferroni's post-test.

The TrkAIg1,2 can antagonize the actions of NGF when used at a dose of 24 pmol, i.e. threefold higher than the dose of NGF used. In contrast, injection of TrkAIg1,2 in vehicle produced no significant plasma extravasation. Thus, TrkAIg1,2 can antagonize the action of NGF particularly when premixed and co-injected. This indicates that TrkAIg12 is able to bind to, and thus sequester, NGF thus inhibiting its action of extravasation. To investigate the ability of TrkAIg1,2 to antagonize NGF *in vivo,* skin sites were pre-treated by intradermal injection of TrkAIg1,2, and NGF was given (i.d.) 5 min later. The results, shown in Fig. 15, show that 24 pmol TrkAIg1,2 can significantly inhibit the plasma extravasation induced by 8 pmol 7S NGF. Results are expressed as plasma extravasated (μl/site) in response to intradermal test agent, mean ± s.e.mean, n = 4. The response induced by 7S NGF (8 pmol) is shown in the filled squares, both alone and in sites pre-treated with increasing doses of TrkAIg1,2, shown. For comparison, the response induced 7S NGF (8 pmol) co-injected with TrkAIg1,2 (24 pmol) is shown in the filled bar. Plasma extravasation induced by intradermal injection of GR 73632 (30 pmol) is shown in the filled triangles and Tyrode's solution (vehicle) in the open circles, with the pre-treatment dose of TrkAIg1,2 shown. Plasma extravasation in sites receiving agent plus co-injected TrkAIg1,2 differing significantly from the sites receiving agent alone are shown as ** $p < 0.01$, as assessed by ANOVA with Bonferroni's post-test.

The plasma extravasation seen with NGF in sites pre-treated with 24 pmol TrkAIg1,2 was similar to the plasma extravasation produced by NGF co-injected with 24 pmol TrkAIg1,2. As with the co-injection experiments, pre-treatment with TrkAIg1,2 produced no significant plasma extravasation when injected alone. In an attempt to determine if the action of TrkAIg1,2 was specific to NGF-induced responses or a general anti-inflammatory effect, the NK1 agonist GR73632 (30 pmol) was injected into TrkAIg1,2 pre-treated sites. The 5 min. pre-treatment failed to inhibit the plasma extravasation induced by GR73632, as also shown in Fig 15.

In order to evaluate the stability of the NGF sequestration, skin sites were pre-treated for a longer period (40 min) with TrkAIg1,2 and NGF given (i.d.) at the start of the accumulation period, as shown in Fig. 16. Results are expressed as plasma extravasated (μl/site) in response to intradermal test agent, mean ± s.e.mean, n = 4. The response induced by 7S NGF (8 pmol) is shown in the filled squares, both alone and in sites pre-treated with increasing doses of TrkAIg1,2, is shown. For comparison, the response induced 7S NGF (8 pmol) co-injected with TrkAIg1,2 (24 pmol) is shown by the filled bar. Plasma extravasation induced by intradermal injection of GR73632 (30 pmol) is shown in the filled triangles and Tyrode's solution (vehicle) in the open circles, with the pre-treatment dose of TrkAIg1,2 shown on the y-axis. Plasma extravasation in sites receiving agent plus co-injected TrkAIg1,2 differing significantly from the sites receiving agent alone are shown as * $p < 0.05$, as assessed by ANOVA with Bonferroni's post-test.

In these experiments, NGF-induced plasma extravasation was significantly inhibited by 80 pmol, but not 24 pmol, TrkAIg1,2. The plasma extravasation induced by co-injection of 8 pmol NGF with 80 pmol TrkAIg1,2 is shown for comparison. In keeping with the results of the previous experiments, the doses of TrkAIg1,2 used failed to produce significant plasma extravasation when injected alone and also failed to inhibit the plasma extravasation induced by GR73632 (as before).

**The effect of TrkAIg1 on NGF-induced plasma extravasation.**

Following the previous series of experiments, using both immunoglobulin-like domains (TrkAIg1,2), we attempted to further characterize the binding of NGF to the immunoglobulin-like domains of TrkA. To do this, we used a sample of recombinant TrkAIg1, the first immunoglobulin-like domain. As can be seen in Fig. 17, co-injection experiments with TrkAIg1 showed no significant inhibition of NGF-induced plasma extravasation at doses up to 80 pmol/site.

Results are expressed as plasma extravasated (μl/site) in response to intradermal test agent, mean ± s.e.mean, n = 6. The response induced by 7S NGF (8 pmol) is shown in the filled squares, both alone and with co-injection of TrkAIg1, shown. For comparison, the response induced by Tyrode's solution (vehicle) is shown in the open circles, with the dose of TrkAIg1 co-injected shown. Plasma extravasation in sites receiving agent plus co-injected TrkAIg1 differing significantly from the sites receiving agent alone are shown as ns, not significant, as assessed by ANOVA with Bonferroni's post-test.

**The effect of TrkAIg2 on NGF-induced plasma extravasation.**

The ability of TrkAIg2 to bind and sequester NGF was evaluated.

As can be seen in Fig. 18, co-injection of TrkAIg2 with NGF was able to produce significant inhibition of NGF-induced plasma extravasation, when given in a ten-fold excess. At all of the doses used, TrkAIg2 produced no inhibition of plasma extravasation induced by GR73632, and also produced no significant plasma extravasation when injected alone. Results are expressed as plasma extravasated (μl/site) in response to intradermal test agent, mean ± s.e.mean, n = 4 - 8. The response induced by 7S NGF (8 pmol) is shown in the filled squares, both alone and with co-injection of TrkAIg2, shown. For comparison, the response induced by GR73632 (30 pmol) is shown in the filled triangles and that induced by Tyrode's solution (vehicle) is shown in the open circles, with the dose of TrkAIg2 co-injected shown. Plasma extravasation in sites receiving agent plus co-injected TrkAIg2 differing significantly from the sites receiving agent alone are shown as *** $p < 0.001$, as assessed by ANOVA with Bonferroni's post-test.

Pre-treatment of skin sites with 80 pmol TrkAIg2 with NGF was also able to inhibit the plasma extravasation induced by 8 pmol NGF, given 5 min later Fig. 19. Results are expressed as plasma extravasated (μl/site) in response to intradermal test agent, mean ± s.e.mean, n = 4. The response induced by 7S NGF (8 pmol) is shown in the filled squares, both alone and in sites pre-treated with increasing doses of TrkAIg2, shown. Plasma extravasation induced by intradermal injection of GR73632 (30 pmol) is shown in the filled triangles and Tyrode's solution (vehicle) in the open circles, with the pre-treatment dose of TrkAIg2 shown. Plasma extravasation in sites receiving agent plus co-injected TrkAIg2 differing significantly from the sites receiving agent alone are shown as [***]$p < 0.001$, as assessed by ANOVA with Bonferroni's post-test. Again, this pre-treatment had no effect on GR73632-induced plasma extravasation, and produced no significant plasma extravasation when injected alone (Fig. 19).

Similar results were seen when TrkAIg2 was used as a 40 min pre-treatment, as shown in Fig. 20. Results are expressed as plasma extravasated (μl/site) in response to intradermal test agent, mean ± s.e.mean, n = 3. The response induced by 7S NGF (8 pmol) is shown in the filled squares, both alone and in sites pre-treated with increasing doses of TrkAIg2, shown. Plasma extravasation induced by intradermal injection of GR73632 (30 pmol) is shown in the filled triangles and Tyrode's solution (vehicle) in the open circles, with the pre-treatment dose of TrkAIg2 shown. Plasma extravasation in sites receiving agent plus co-injected TrkAIg2 differing significantly from the sites receiving agent alone are shown as *** $p < 0.001$, as assessed by ANOVA with Student-Newman-Keuls post-test. The plasma extravasation induced by NGF was significantly inhibited by TrkAIg2 at 80 pmol. For comparison, the plasma extravasation induced by 8 pmol 7S NGF co-injected with 80 pmol TrkAIg2 is shown in the filled column. Pre-treatment with TrkAIg2 induced no plasma extravasation alone and did not affect the plasma extravasation induced by GR 73632.

The results clearly demonstrate that the TrkIg2 domain is able to bind to NGF *in vivo* and block its biological activity.

**Crystallisation of TrkAIg2**

Crystals of recombinant TrkA-Ig2 have been obtained under a variety of conditions between 14-20% MPD, pH5.0 (100mM Na-citrate), 300 to 500mM NaCl, pH 5.0 (100mM Na-citrate), most favourably at 500mM NaCl, pH 5.0. The crystals grow reproducibly to approximate dimensions of 0.2 x 0.2 x 0.2 mm. Crystals are then cryo-preserved. Using the home source (rotating anode, mirrors, imaging plate), and the synchrotron source at Hamburg, these crystals diffract to about 2.8 Å. Assuming 50% solvent, it is estimated that there are 4 (or possibly 3) molecules in the asymmetric unit. Crystals of a selenoMet form of the protein have been prepared using a selenoMet auxotroph (there are 4 methionines in the construct) which has been used for MAD phasing and as a heavy atom derivative. Recombinant forms of both the native and selenoMet TrkA-Ig2 were prepared, purified and refolded using the established procedures as defined elsewhere in the description.

**Therapeutic Aspects of TrkAIg2**

Since certain pain states are caused by overexpression of NGF, it is anticipated and evidence indicates, that application of NGF antagonists such as antibodies or recombinant TrkAIg2 binding domain may alleviate resulting pain states (McMahon, S. B.*Series B-Biological Sciences,* (1996), **351,** No.1338, 431- 440; Woolf, C. J. *et al. British Journal Of Pharmacology,* (1997), **121, No.3**, 417- 424; Lowe, E. M. *et al. British Journal Of Urology,* (1997), **79, No.4**, 572-577; Dmitrieva, N. *et al. Neuroscience,* (1997), **78, No.2**, 449-459; Aloe, L. *et al. International Journal Of Tissue Reactions-Experimental And Clinical Aspects,* (1993), **15**, **No.4**, 139-143; Aloe, L. *et al. Rheumatology International,* (1995), **14, No.6**, 249-252).

Therefore, in summary, the inventors have demonstrated the inability of the region referred to as TrkAIg1 to bind NGF. The smallness of the TrkAIg2 molecule and the abundance with which this protein can be produced for example in *E. coli,* and purified and refolded into its correct formation confers certain advantages over the complete extracellular domain which, by necessity, must be made in mammalian or insect cells.

There are known to be various pain states, often chronic inflammatory conditions which are associated with an increase in NGF protein levels. These include idiopathic sensory urgency and interstitial cystitis, arthritis and shingles. It is also suggested that such chronic conditions may result in sensitization of peripheral neurons and perhaps even long-term sensory neuronal abnormalities. By sequestration of this increased NGF, by the use of TrkAIg2, it will be possible to alleviate pain in such conditions and in other conditions in which NGF is elevated.

Throughout the specification, the following abbreviations have been used:

Abbreviations for amino acids

| *Amino acid* | *Three-letter abbreviation* | *One-letter symbol* |
|---|---|---|
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamic acid | Glu | E |
| Glutamine or glutamic acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Abbreviations for nucleotides:

A Adenine
G Guanine
C Cytosine
T Thymine
U Uracil

Abbreviations for mutations:

$$X_1NNNX_2$$

$X_1$ and $X_2$ = an amino acid one letter symbol as defined above.
NNN = numerical digits indicating the position of the mutation within the amino acid sequence.

**Further embodiments of the invention**

1. A polypeptide consisting of or comprising the amino acid sequence of residues 22 to 119 of Fig. 4B or a portion of the amino acid sequence of Fig. 4B, the amino acid sequence being capable of binding a neurotrophin.
2. A polypeptide according to claim 1 comprising residues 22 to 144 of Fig. 4 B.
3. A polypeptide according to claim 1 or 2 wherein the polypeptide is TrkAIg1,2, or a portion thereof.
4. A polypeptide according to any one of claims 1 to 3 which binds with high affinity to a neurotrophin.
5. A polypeptide according to claim 4 which binds to a neurotrophin with a disassociation constant of less than 10nM.
6. A polypeptide according to any preceding claim wherein the polypeptide is isolated from animal cells.
7. A polypeptide according to claim 6 wherein the animal cells are mammalian cells.

8. A polypeptide according to claim 7 wherein the mammalian cells are human cells.
9. A polypeptide according to claim 6 wherein the animal cells are insect cells reptilian cells, fish cells, avian cells or amphibian cells.
10. A polypeptide according to any preceding claim wherein the neurotrophin is NGF, NT-3 or a neurotrophin which binds p75NGFR.
11. A polypeptide according to any preceding claim wherein the neurotrophin exists as a monomer, dimer, trimer, or a neurotrophin heterodimer.
12. A polypeptide according to any preceding claim wherein the neurotrophin is from a mammal, insect, reptile, fish, bird or amphibian.
13. A polypeptide according to claim 12 wherein the mammalian neurotrophin is a human neurotrophin.
14. A DNA sequence which encodes a polypeptide according to any of claims 1 to 13 or variants of such a DNA sequence due to the degeneracy of the genetic code, or insertion or deletion mutants thereof that encode a polypeptide according to any of claims 1 to 13 and DNA sequences which hybridise at 50°C, 6xSSC salt concentration to such DNA sequences.
15. A DNA sequence which encodes a polypeptide according to any of claims 1 to 13 or variants of such a DNA sequence due to the degeneracy of the genetic code, or insertion or deletion mutants thereof that encode a polypeptide according to any of claims 1 to 13 and DNA sequences which hybridise at 65°C, 2xSSC salt concentration to such DNA sequences.
16. A plasmid or other vector comprising a DNA sequence according to claim 14 or claim 15.
17. A plasmid according to claim 16 wherein the plasmid is an expression vector.
18. A plasmid according to claim 16 or claim 17 wherein the plasmid is pET-15b.
19. A complex comprising at least one polypeptide according to any of claims 1 to 13 and at least one neurotrophin or neurotrophin subunit, manomer or biologically active portion thereof.
20. A method of producing a polypeptide according to any one of claims 1 to 13 comprising introducing a DNA sequence according to claim 14 or a plasmid according to any of claims 15 to 17 into a suitable host whereby the DNA sequence is expressed.
21. A method according to claim 20 wherein the host is an animal cell.
22. A method according to claim 21 wherein the host is a bacterial cell.
23. A method according to claim 22 wherein the host is a mammalian cell.
24. A method according to claim 23 wherein the host is a human cell.
25. A method of screening for molecules which bind to the TrkA receptor using a polypeptide according to any of claims 1 to 13.
26. A method according to claim 25 comprising comparing the binding of a putative ligand to TrkAIg1, or a portion thereof, with the binding of the same putative ligand to TrkAIg2 or a portion thereof.
27. A method according to claim 25 or claim 26 comprising selecting molecules which bind to at least one solvent-exposed loop of TrkAIg2.
28. A method according to claim 27 wherein the solvent-exposed loop is loop E to F as shown in Fig.1(B).
29. A method according to claim 27 or 28 wherein the solvent-exposed loop is loop C" to D as shown in Fig. 1(B).
30. A method according to claim 28 or claim 29 wherein molecules with an affinity of at least 10nM are selected.
31. A method according to any claims 25 to 30 comprising selecting molecules which enhance binding of a polypeptide according to any one of claims 1 to 13 or TrkA or a portion thereof in its natural state to a neurotrophin.
32. A method of combinatorial chemistry comprising:

1. a compound generating step
2. a compound screening step which involves the binding of the compound generated during step 1 with a polypeptide or a portion of a polypeptide according to any of claims 1 to 13.

33. An antibody raised against a polypeptide according to any of claims 1 to 13.
34. An antibody according to claim 33 wherein the polypeptide is TrkAIg2.
35. A host cell containing a DNA sequence according to claim 14 or a plasmid or other vector according to any of claims 16 to 18.
36. A host cell according to claim 35 wherein the host cell is a mammalian, bacterial, insect, or yeast cell.
37. A host cell according to claim 32 wherein the mammalian cell is a human cell.
38. A diagnostic probe wherein the probe comprises any portion of a polypeptide according to any of claims 1 to 13.
39. A diagnostic probe according to claim 38 wherein the probe is labelled.
40. A diagnostic probe according to claim 39 wherein the label comprises a fluorescent tag or a radiolabel.
41. Diagnostic tests, assays or monitoring methods using a polypeptide or any fragment of a polypeptide according to any of claims 1 to 13, or an antibody according to claim 33 or 34.

42. Diagnostic tests, assays or monitoring methods using a probe comprising at least a portion of a DNA sequence according to claim 14, or a probe according to any of claims 38 to 40.
43. Diagnostic tests, assays or monitoring methods according to claim 41 or claim 42 wherein the tests, assays, or monitoring methods comprise microbiological, animal cell, or biodiagnostic tests, assays or monitoring methods.
44. Diagnostic tests, assays or monitoring methods according to any of claims 41 to 43 which detect elevated neurotrophin levels associated with peripheral inflammation, chronic inflammation, postherpetic neuralgia, interstitial cystitis, arthritis or shingles.
45. A method of producing a polypeptide according to any of claims 1 to 13 by chemical or biological means.
46. An organism engineered to contain, express or overexpress a polypeptide according to any of claims 1 to 13 or a DNA sequence according to claim 14 or claim 15.
47. An organism according to claim 46 wherein the organism is an animal, bacteria, yeast, or insect.
48. An organism according to claim 47 wherein the animal is a mammal, bacteria, yeast or insect.
49. A composition for the control of pain associated with an increase in neurotrophin levels comprising a polypeptide according to any of claims 1 to 13.
50. A method of treating a subject with pain associated with increased neurotrophin levels, the method comprising supplying to the subject a pharmaceutical composition comprising a polypeptide according to any of claims 1 to 13 or a neurotrophin analogue isolated or identified by a screening procedure involving a polypeptide according to any of claims 1 to 13.
51. A method according to claim 50 wherein the pain is a symptom of conditions selected from idiopathic sensory urgency (ISU), interstitial cystitis, arthritis, shingles, peripheral inflammation, chronic inflammation, or postherpetic neuralgia.
52. A method of treating a subject with Alzheimers disease, the method comprising supplying to the subject a pharmaceutical composition comprising a polypeptide according to any of claims 1 to 13.
53. A method of treating a subject with Alzheimers disease, the method comprising supplying to the subject a pharmaceutical composition comprising an neurotrophin analogue isolated or identified by a screening procedure involving a polypeptide according to any of claims 1 to 13.
54. A method of reducing free NGF levels in a subject, the method comprising supplying to a subject, a polypeptide according to any of claims 1 to 13.
55. A method of reducing plasma extravasation comprising supplying to a subject, a polypeptide according to any of claims 1 to 13.
56. A method according to any of claims 50 to 555 in which the neurotrophin is NGF.
57. A pharmaceutical composition comprising a polypeptide according to any of claims 1 to 13 together with a pharmaceutically acceptable carrier or diluent.
58. A pharmaceutical composition according to claim 57 including at least one neurotrophin.
59. A machine readable data storage medium, comprising a data storage material encoded with machine readable data which, when using a machine programmed with instructions for using the data, is capable of displaying a graphical three-dimensional representation of a polypeptide according to any of claims 1 to 13.
60. A homology model having the coordinates shown in Fig. 21.
61. A computer programmed with or arranged to provide a homology model for at least a portion of a polypeptide according to any one of claims 1 to 13, or a complex of such a polypetide with another molecule.
62. A machine readable data storage medium on which has been stored in machine readable form a homology model of a polypeptide according to any one of claims 1 to 13 or a complex of such a polypetide with another molecule.
63. A computer according to claim 61 or a machine readable data storage medium according to claim 62 in which the model is obtained from coordinates shown in Fig. 21.
64. Compounds obtained by a method according to any of claims 25 to 32 or using a computer according to claim 61 or 63 or using a machine readable data storage medium according to claim 62 or 63.
65. Crystalline Trk AIg2.
66. A crystal comprising at least a portion of a polypeptide according to any of claims 1 to 11.
67. A crystal according to claim 63 wherein a polypeptide is TrkAIg2.

## Claims

1. The use of a polypeptide consisting of the amino acid sequence of residues 22 to 144 of Fig. 4B in the preparation of a medicament for the treatment and/or prevention of pain.

2. The use of a polypeptide consisting of a portion of the amino acid sequence of residues 22 to 144 of Fig. 4B in the preparation of a medicament for the treatment and/or prevention of pain.

3. The use of a polypeptide consisting of the amino acid sequence of residues 22 to 150 of Fig. 4C in the preparation of a medicament for the treatment and/or prevention of pain.

4. The use according to claim 1, 2 or 3 wherein the pain is associated with increased levels of at least one neurotrophin.

5. The use according to claim 4 wherein the at least one neurotrophin is selected from NGF and NT3.

6. The use according to any preceding claim wherein the pain is associated with peripheral inflammation.

7. The use according to any preceding claim wherein the pain is associated with interstitial cystitis.

8. The use according to any of claims 1-6 wherein the pain is associated with rheumatoid arthritis.

9. The use according to any of claims 1-6 wherein the pain is neuralgia.

10. The use according to claim 9 wherein the neuralgia is postherpetic neuralgia associated with shingles.

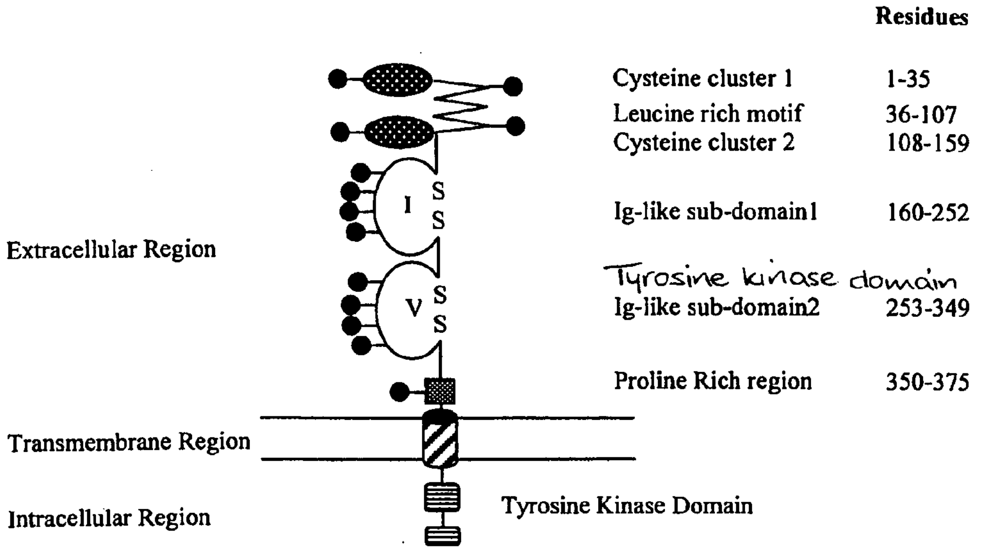
Residues
Cysteine cluster 1 1-35
Leucine rich motif 36-107
Cysteine cluster 2 108-159
Ig-like sub-domain1 160-252
Tyrosine kinase domain
Ig-like sub-domain2 253-349
Proline Rich region 350-375
Extracellular Region
Transmembrane Region
Intracellular Region
Tyrosine Kinase Domain
I
V
S
S
S
S

Fig. 1(B)

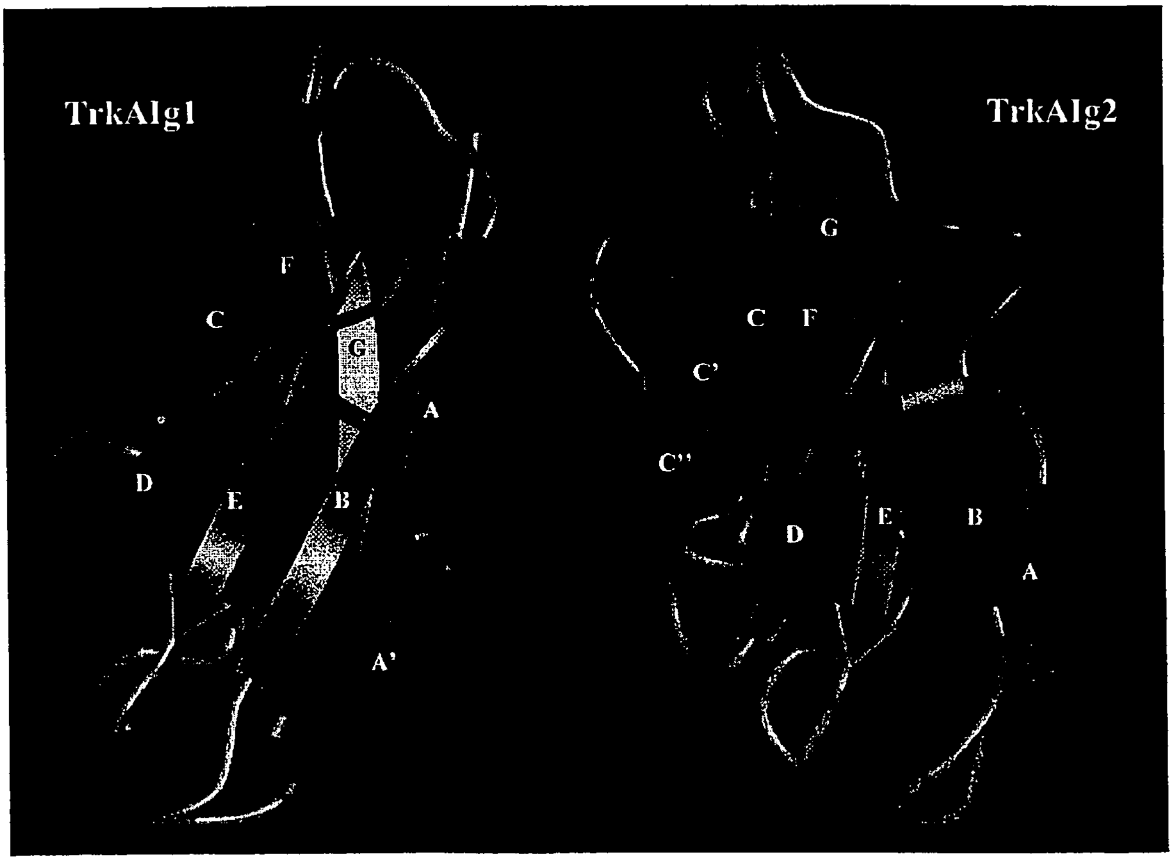
TrkAIg1
TrkAIg2
F
C
G
A
D
E
B
A'
G
C F
C'
C''
D
E
B
A

Fig. 2.

2/27

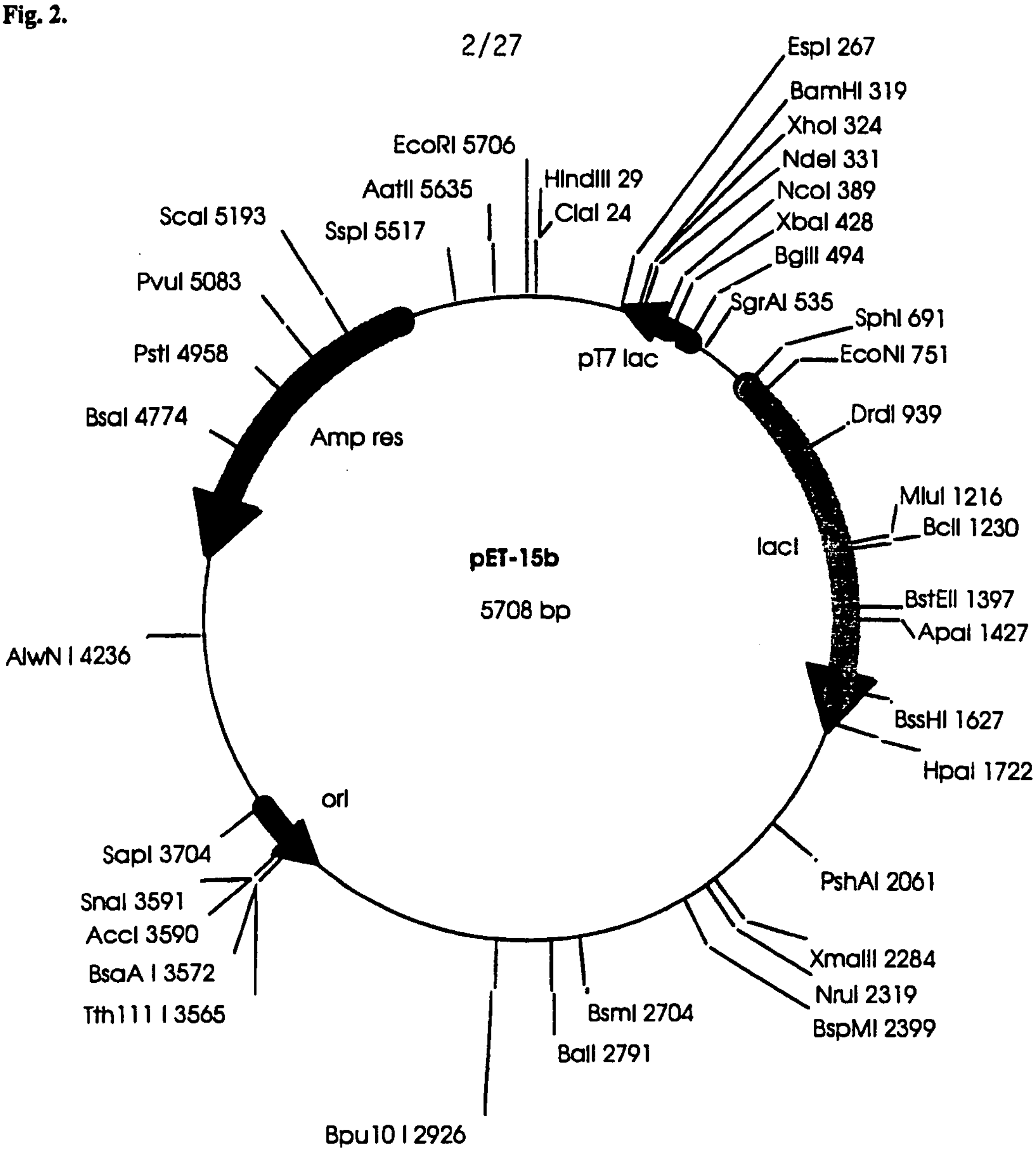


A)

B)

**Oligo10692** Left Primer for TrkAIg1,2
CCGATCTCGAGGGTGTGCCCACGCTG
*Xho*I

**Oligo10693** Right Primer for TrkAIg1,2
CCGATCTCGAG TTA TCA TTCGTCCTTCTTCTCCACCGGGTC
*Xho*I Stop Stop

**Fig. 3.**

```
    Met Gly Ser Ser His His His His His His Ser Ser    12
  1 ATG GGC AGC AGC CAT CAT CAT CAT CAT CAC AGC AGC
    Gly Leu Val Pro Arg Gly Ser His Met Leu Glu Gly    24
 38 GGC CTG GTG CCG CGC GGC AGC CAT ATG CTC GAG GGT
    Val Pro Thr Leu Lys Val Gln Val Pro Asn Ala Ser    36
 74 GTG CCC ACG CTG AAG GTC CAG GTG CCC AAT GCC TCG
    Val Asp Val Gly Asp Asp Val Leu Leu Arg Cys Gln    48
110 GTG GAT GTG GGG GAC GAC GTG CTG CTG CGG TGC CAG
    Val Glu Gly Arg Gly Leu Glu Gln Ala Gly Trp Ile    60
146 GTG GAG GGG CGG GGC CTG GAG CAG GCC GGC TGG ATC
    Leu Thr Glu Leu Glu Gln Ser Ala Thr Val Met Lys    72
182 CTC ACA GAG CTG GAG CAG TCA GCC ACG GTG ATG AAA
    Ser Gly Gly Leu Pro Ser Leu Gly Leu Thr Leu Ala    84
218 TCT GGG GGT CTG CCA TCC CTG GGG CTG ACC CTG GCC
    Asn Val Thr Ser Asp Leu Asn Arg Lys Asn Leu Thr    96
254 AAT GTC ACC AGT GAC CTC AAC AGG AAG AAC TTG ACG
    Cys Trp Ala Glu Asn Asp Val Gly Arg Ala Glu Val   108
290 TGC TGG GCA GAG AAC GAT GTG GGC CGG GCA GAG GTC
    Ser Val Gln Val Asn Val Ser Phe Pro Ala Ser Val   120
326 TCT GTT CAG GTC AAC GTC TCC TTC CCG GCC AGT GTG
    Gln Leu His Thr Ala Val Glu Met His His Trp Cys   132
362 CAG CTG CAC ACG GCG GTG GAG ATG CAC CAC TGG TGC
    Ile Pro Phe Ser Val Asp Gly Gln Pro Ala Pro Ser   144
398 ATC CCC TTC TCT GTG GAT GGG CAG CCG GCA CCG TCT
    Leu Arg Trp Leu Phe Asn Gly Ser Val Leu Asn Glu   156
434 CTG CGC TGG CTC TTC AAT GGC TCC GTG CTC AAT GAG
    Thr Ser Phe Ile Phe Thr Glu Phe Leu Glu Pro Ala   168
470 ACC AGC TTC ATC TTC ACT GAG TTC CTG GAG CCG GCA
    Ala Asn Glu Thr Val Arg His Gly Cys Leu Arg Leu   180
506 GCC AAT GAG ACC GTG CGG CAC GGG TGT CTG CGC CTC
    Asn Gln Pro Thr His Val Asn Asn Gly Asn Tyr Thr   192
542 AAC CAG CCC ACC CAC GTC AAC AAC GGC AAC TAC ACG
    Leu Leu Ala Ala Asn Pro Phe Gly Gln Ala Ser Ala   204
578 CTG CTG GCT GCC AAC CCC TTC GGC CAG GCC TCC GCC
    Ser Ile Met Ala Ala Phe Met Asp Asn Pro Phe Glu   216
614 TCC ATC ATG GCT GCC TTC ATG GAC AAC CCT TTC GAG
    Phe Asn Pro Glu Asp Pro Ile Pro Asp Thr Asn Ser   228
650 TTC AAC CCC GAG GAC CCC ATC CCT GAC ACT AAC AGC
    Thr Ser Gly Asp Pro Val Glu Lys Lys Asp Glu Stop  239
686 ACA TCT GGA GAC CCG GTG GAG AAG AAG GAC GAA TGA
722 TAACTCGAGATCGG
```

**Fig.4.**

A)

```
    Met Gly Ser Ser His His His His His His Ser Ser   12
  1 ATG GGC AGC AGC CAT CAT CAT CAT CAT CAC AGC AGC
    Gly Leu Val Pro Arg Gly Ser His Met Leu Glu Gly   24
 38 GGC CTG GTG CCG CGC GGC AGC CAT ATG CTC GAG GGT
    Val Pro Thr Leu Lys Val Gln Val Pro Asn Ala Ser   36
 74 GTG CCC ACG CTG AAG GTC CAG GTG CCC AAT GCC TCG
    Val Asp Val Gly Asp Asp Val Leu Leu Arg Cys Gln   48
110 GTG GAT GTG GGG GAC GAC GTG CTG CTG CGG TGC CAG
    Val Glu Gly Arg Gly Leu Glu Gln Ala Gly Trp Ile   60
146 GTG GAG GGG CGG GGC CTG GAG CAG GCC GGC TGG ATC
    Leu Thr Glu Leu Glu Gln Ser Ala Thr Val Met Lys   72
182 CTC ACA GAG CTG GAG CAG TCA GCC ACG GTG ATG AAA
    Ser Gly Gly Leu Pro Ser Leu Gly Leu Thr Leu Ala   84
218 TCT GGG GGT CTG CCA TCC CTG GGG CTG ACC CTG GCC
    Asn Val Thr Ser Asp Leu Asn Arg Lys Asn Leu Thr   96
254 AAT GTC ACC AGT GAC CTC AAC AGG AAG AAC TTG ACG
    Cys Trp Ala Glu Asn Asp Val Gly Arg Ala Glu Val  108
290 TGC TGG GCA GAG AAC GAT GTG GGC CGG GCA GAG GTC
    Ser Val Gln Val Asn Val Ser Phe Stop             116
326 TCT GTT CAG GTC AAC GTC TCC TTC TGA TAACTCGAGCG
```

B)

```
    Met Gly Ser Ser His His His His His His Ser Ser   12
  1 ATG GGC AGC AGC CAT CAT CAT CAT CAT CAC AGC AGC
    Gly Leu Val Pro Arg Gly Ser His Met Pro Ala Ser   24
 38 GGC CTG GTG CCG CGC GGC AGC CAT ATG CCG GCC AGT
    Val Gln Leu His Thr Ala Val Glu Met His His Trp   36
 74 GTG CAG CTG CAC ACG GCG GTG GAG ATG CAC CAC TGG
    Cys Ile Pro Phe Ser Val Asp Gly Gln Pro Ala Pro   48
110 TGC ATC CCC TTC TCT GTG GAT GGG CAG CCG GCA CCG
    Ser Leu Arg Trp Leu Phe Asn Gly Ser Val Leu Asn   60
146 TCT CTG CGC TGG CTC TTC AAT GGC TCC GTG CTC AAT
    Glu Thr Ser Phe Ile Phe Thr Glu Phe Leu Glu Pro   72
182 GAG ACC AGC TTC ATC TTC ACT GAG TTC CTG GAG CCG
    Ala Ala Asn Glu Thr Val Arg His Gly Cys Leu Arg   84
218 GCA GCC AAT GAG ACC GTG CGG CAC GGG TGT CTG CGC
    Leu Asn Gln Pro Thr His Val Asn Asn Gly Asn Tyr   96
254 CTC AAC CAG CCC ACC CAC GTC AAC AAC GGC AAC TAC
    Thr Leu Leu Ala Ala Asn Pro Phe Gly Gln Ala Ser  108
290 ACG CTG CTG GCT GCC AAC CCC TTC GGC CAG GCC TCC
    Ala Ser Ile Met Ala Ala Phe Met Asp Asn Pro Phe  120
326 GCC TCC ATC ATG GCT GCC TTC ATG GAC AAC CCT TTC
    Glu Phe Asn Pro Glu Asp Pro Ile Pro Asp Thr Asn  132
362 GAG TTC AAC CCC GAG GAC CCC ATC CCT GAC ACT AAC
    Ser Thr Ser Gly Asp Pro Val Glu Lys Lys Asp Glu  144
398 AGC ACA TCT GGA GAC CCG GTG GAG AAG AAG GAC GAA
    Stop                                             144
434 TGA TAACTCGAGATCGG
```

**C)**

| | | |
|---|---|---|
| | Met Gly Ser Ser His His His His His His Ser Ser | 12 |
| 1 | ATG GGC AGC AGC CAT CAT CAT CAT CAT CAC AGC AGC | |
| | Gly Leu Val Pro Arg Gly Ser His Met **Pro Ala Ser** | **24** |
| **38** | **GGC CTG GTG CCG CGC GGC AGC CAT ATG CCG GCC AGT** | |
| | **Val Gln Leu His Thr Ala Val Glu Met His His Trp** | 36 |
| 74 | **GTG CAG CTG CAC ACG GCG GTG GAG ATG CAC CAC TGG** | |
| | **Ser Ile Pro Phe Ser Val Asp Gly Gln Pro Ala Pro** | **48** |
| **110** | **TCG ATC CCC TTC TCT GTG GAT GGG CAG CCG GCA CCG** | |
| | **Ser Leu Arg Trp Leu Phe Asn Gly Ser Val Leu Asn** | 60 |
| **146** | **TCT CTG CGC TGG CTC TTC AAT GGC TCC GTG CTC AAT** | |
| | **Glu Thr Ser Phe Ile Phe Thr Glu Phe Leu Glu Pro** | 72 |
| 182 | **GAG ACC AGC TTC ATC TTC ACT GAG TTC CTG GAG CCG** | |
| | **Ala Ala Asn Glu Thr Val Arg His Gly Cys Leu Arg** | **84** |
| **218** | **GCA GCC AAT GAG ACC GTG CGG CAC GGG TGT CTG CGC** | |
| | **Leu Asn Gln Pro Thr His Val Asn Asn Gly Asn Tyr** | 96 |
| **254** | **CTC AAC CAG CCC ACC CAC GTC AAC AAC GGC AAC TAC** | |
| | **Thr Leu Leu Ala Ala Asn Pro Phe Gly Gln Ala Ser** | 108 |
| **290** | **ACG CTG CTG GCT GCC AAC CCC TTC GGC CAG GCC TCC** | |
| | **Ala Ser Ile Met Ala Ala Phe Met Asp Asn Pro Phe** | 120 |
| 326 | **GCC TCC ATC ATG GCT GCC TTC ATG GAC AAC CCT TTC** | |
| | **Glu Phe Asn Pro Glu Asp Pro Ile Pro Val Ser Phe** | 132 |
| 362 | **GAG TTC AAC CCC GAG GAC CCC ATC CCT GTC TCC TTC** | |
| | **Ser Pro Val Asp Thr Asn Ser Thr Ser Gly Asp Pro** | 144 |
| 398 | **TCG CCA GTG GAC ACT AAC AGC ACA TCT GGA GAC CCG** | |
| | **Val Glu Lys Lys Asp Glu** Stop | 150 |
| 434 | **GTG GAG AAG AAG GAC GAA** TGA TAACTCGAGATCGG | |

**Fig. 5.**

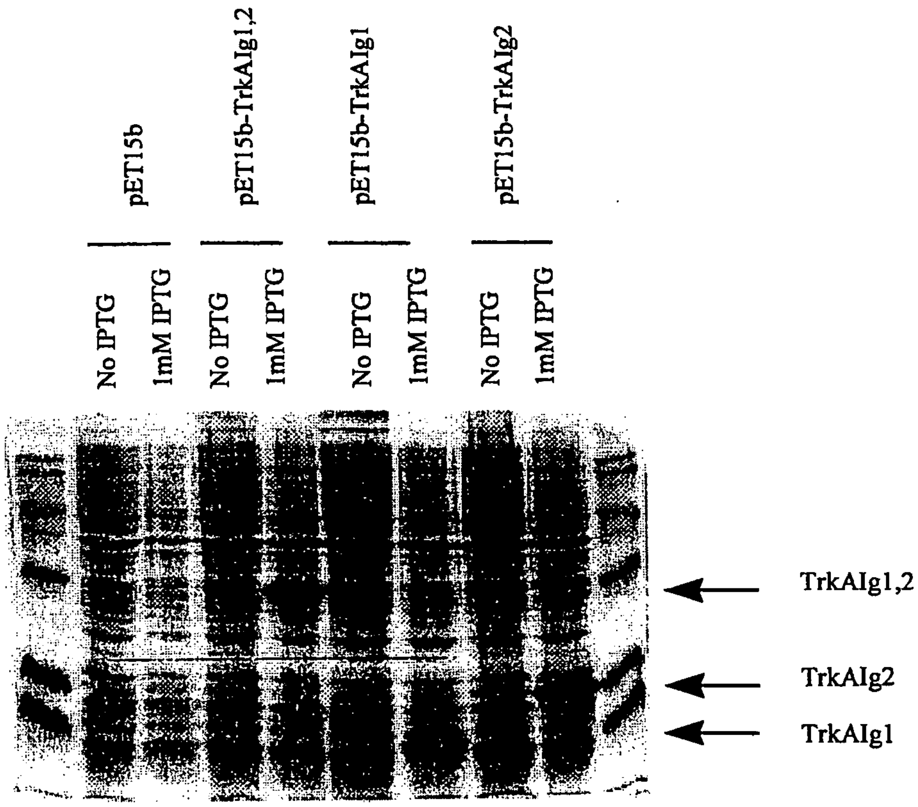

Fig. 6A.

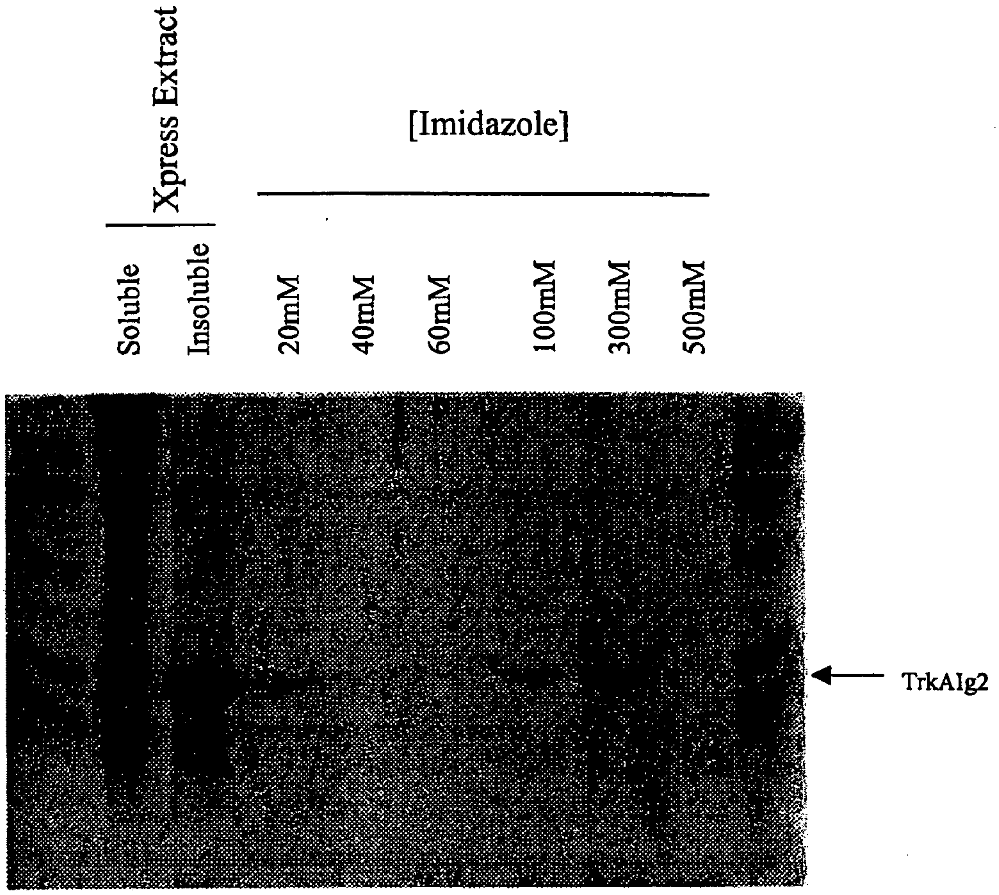
Xpress Extract
[Imidazole]
Soluble
Insoluble
20mM
40mM
60mM
100mM
300mM
500mM
TrkAIg2

Fig. 6B.

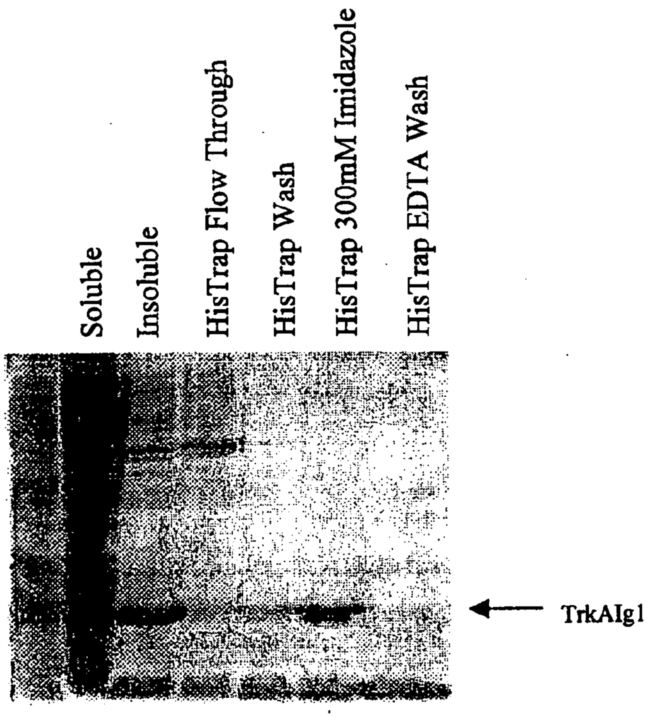
Soluble
Insoluble
HisTrap Flow Through
HisTrap Wash
HisTrap 300mM Imidazole
HisTrap EDTA Wash
TrkAIg1

Fig. 7(A)

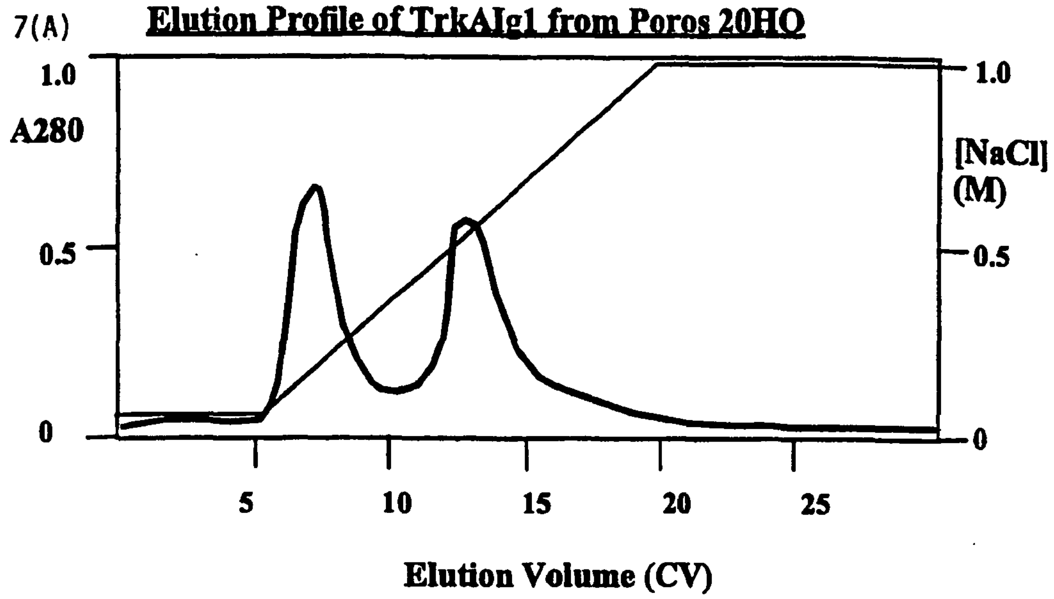
Elution Profile of TrkAIg1 from Poros 20HQ
1.0
A280
0.5
0
1.0
[NaCl] (M)
0.5
0
5
10
15
20
25
Elution Volume (CV)

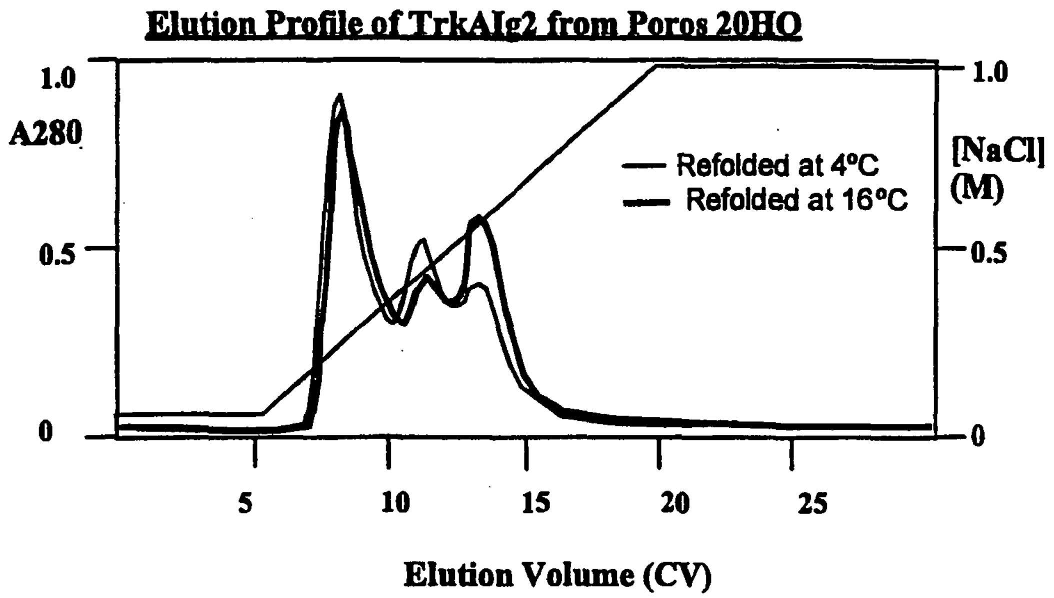
Elution Profile of TrkAIg2 from Poros 20HQ
1.0
A280
0.5
0
Refolded at 4°C
Refolded at 16°C
1.0
[NaCl] (M)
0.5
0
5
10
15
20
25
Elution Volume (CV)

Fig. 7(B)

Fig. 8

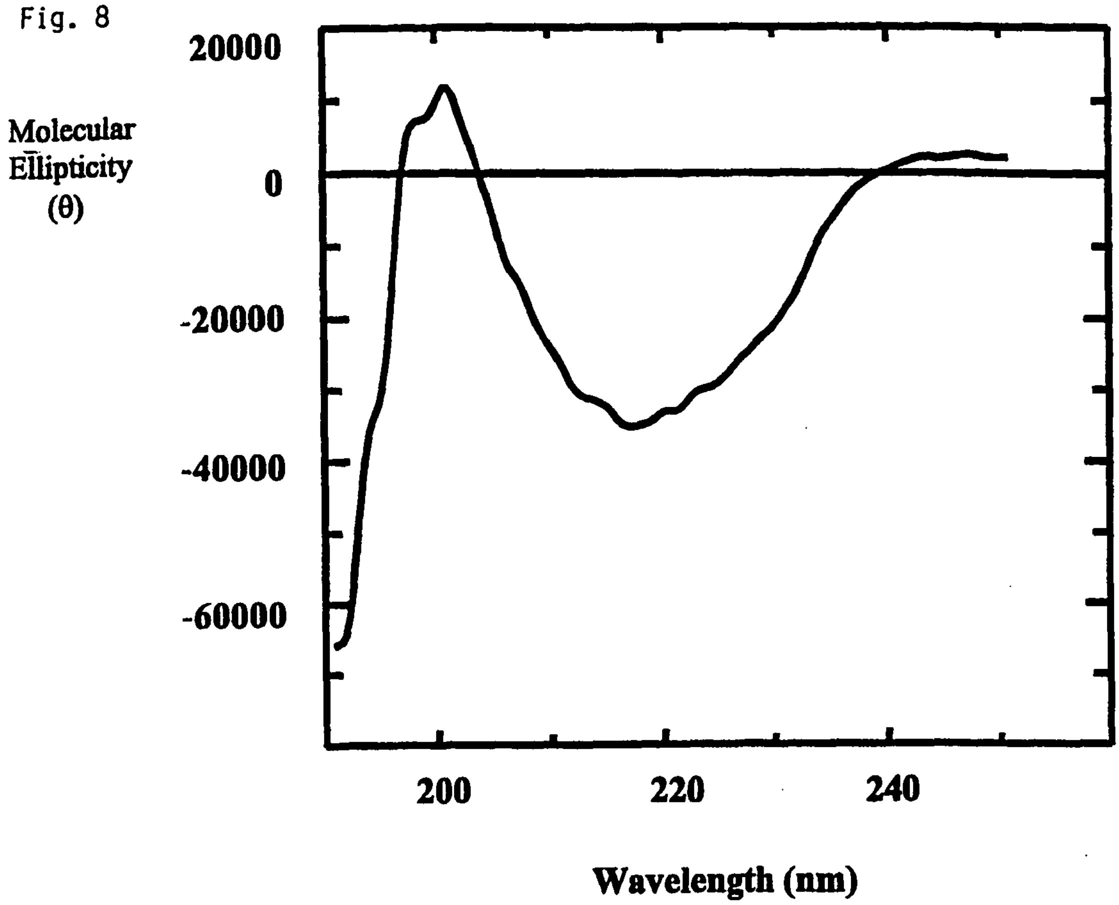

20000
Molecular
Ellipticity
(θ)
0
-20000
-40000
-60000
200
220
240
Wavelength (nm)


Fig. 9.

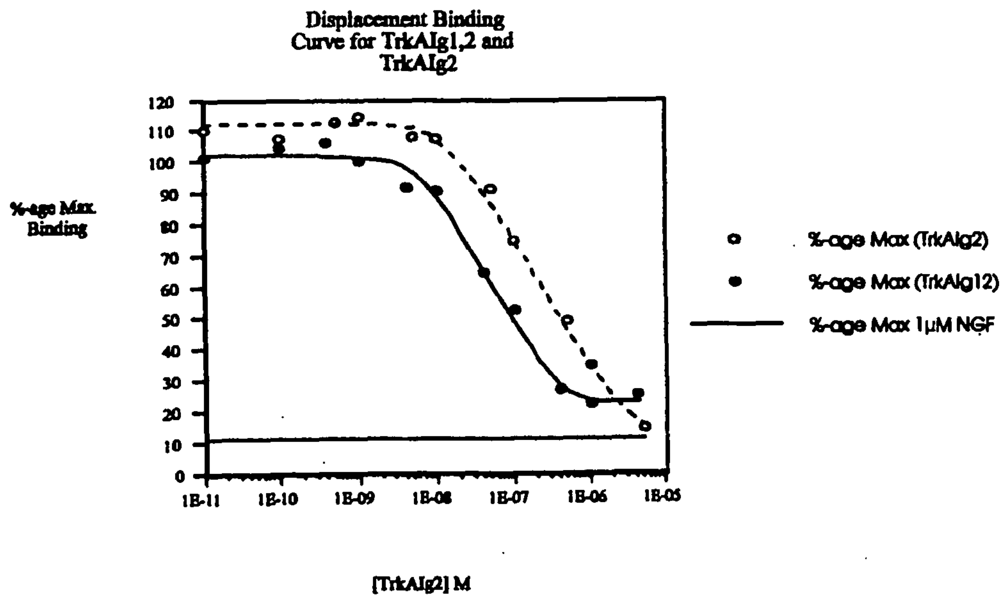

Displacement Binding
Curve for TrkAIg1,2 and
TrkAIg2
%-age Max
Binding
120
110
100
90
80
70
60
50
40
30
20
10
0
1E-11
1E-10
1E-09
1E-08
1E-07
1E-06
1E-05
%-age Max (TrkAIg2)
%-age Max (TrkAIg12)
%-age Max 1μM NGF
[TrkAIg2] M

Fig. 10.

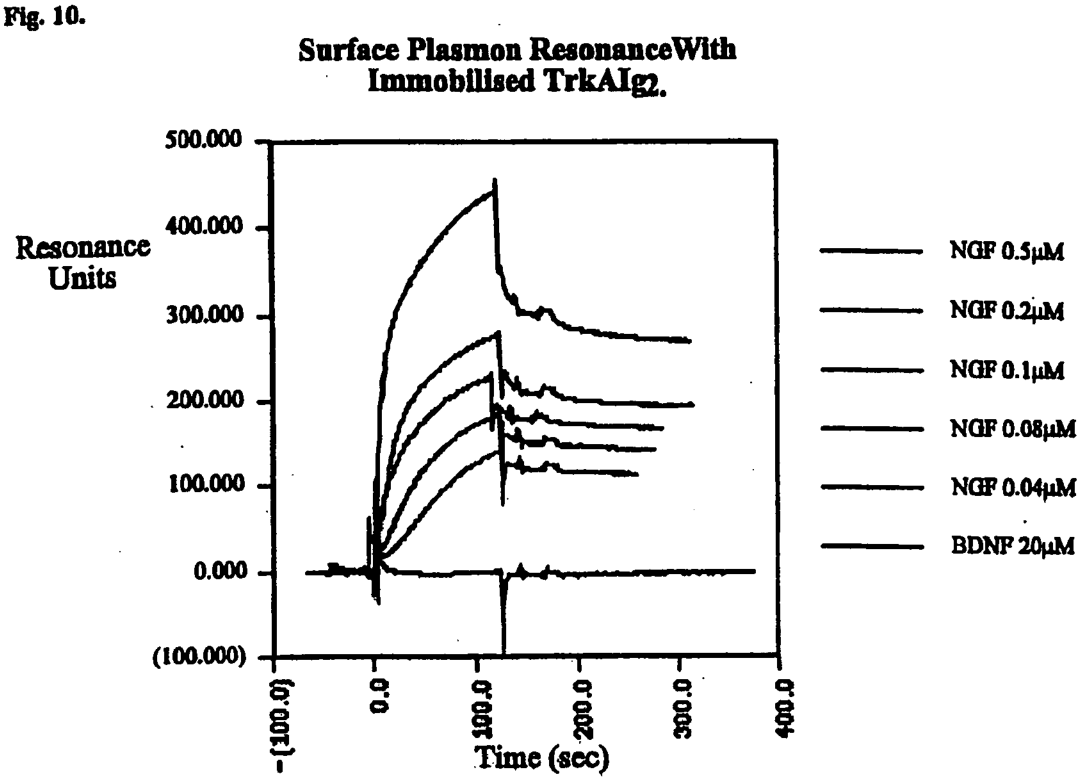
Surface Plasmon ResonanceWith
Immobilised TrkAIg2.
Resonance Units
500.000
400.000
300.000
200.000
100.000
0.000
(100.000)
(100.0)
0.0
100.0
200.0
300.0
400.0
Time (sec)
NGF 0.5μM
NGF 0.2μM
NGF 0.1μM
NGF 0.08μM
NGF 0.04μM
BDNF 20μM

Fig. 11.

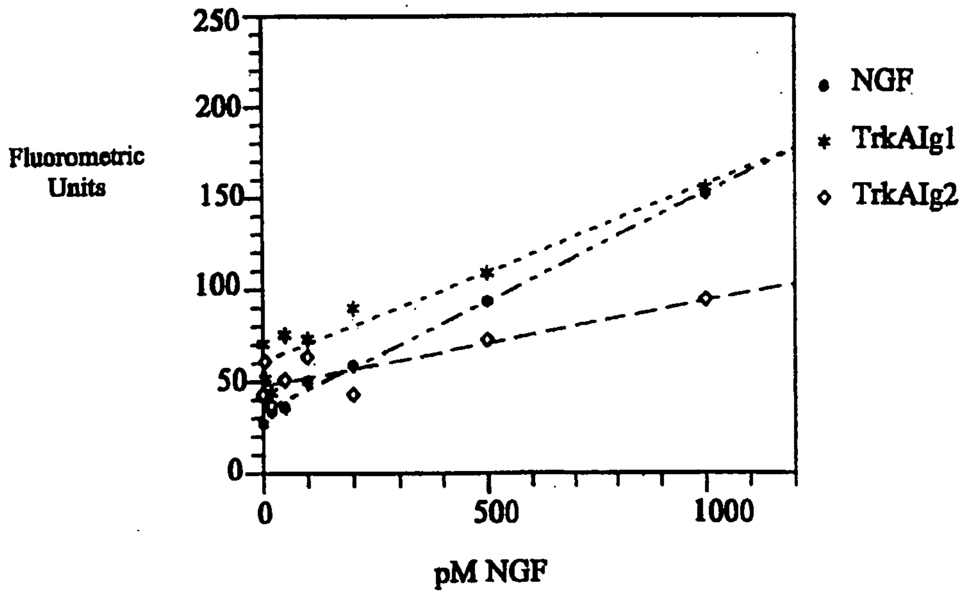
Fluorometric Units
250
200
150
100
50
0
0
500
1000
pM NGF
NGF
TrkAIg1
TrkAIg2

Fig. 12.



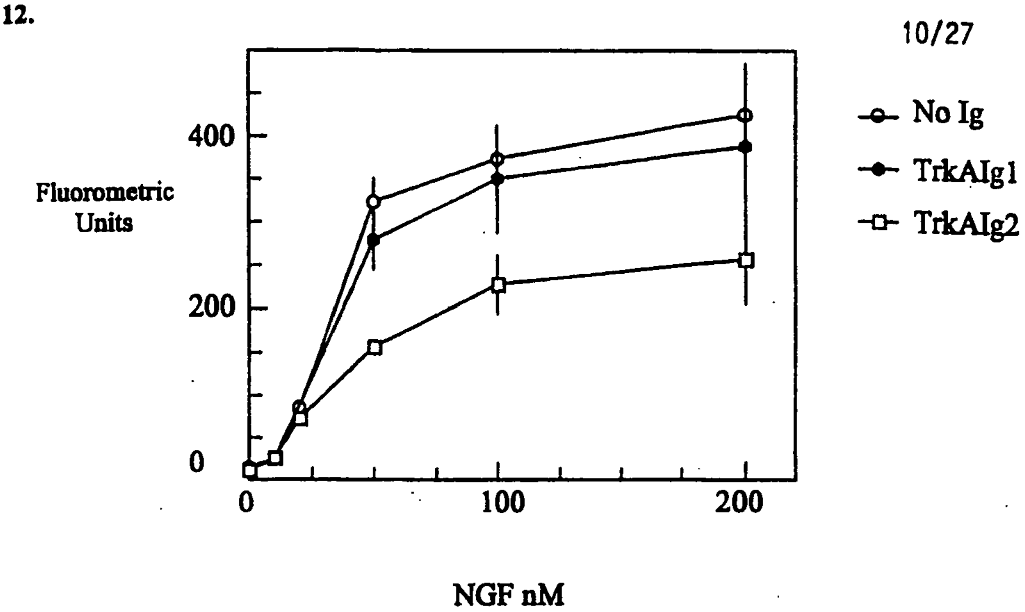

400
Fluorometric Units
200
0
0
100
200
NGF nM
No Ig
TrkAIg1
TrkAIg2

Fig. 13.

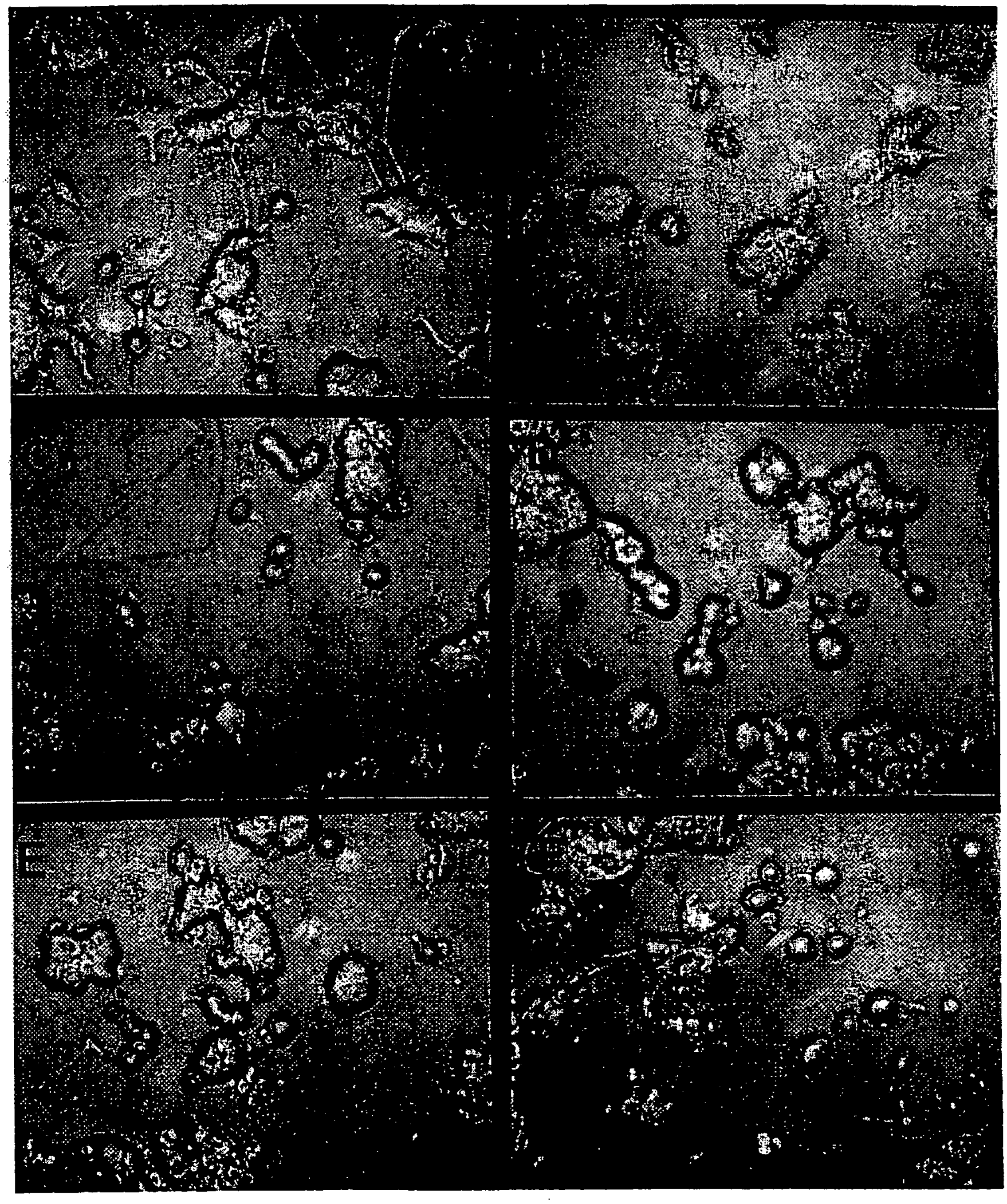

Fig. 14.

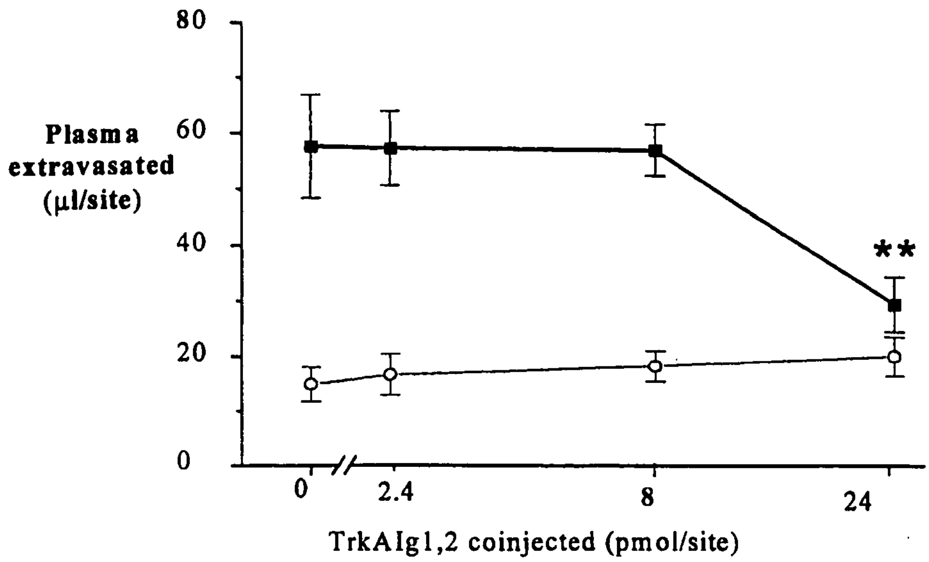
80
60
40
20
0
Plasma
extravasated
(µl/site)
**
0
2.4
8
24
TrkAIg1,2 coinjected (pmol/site)

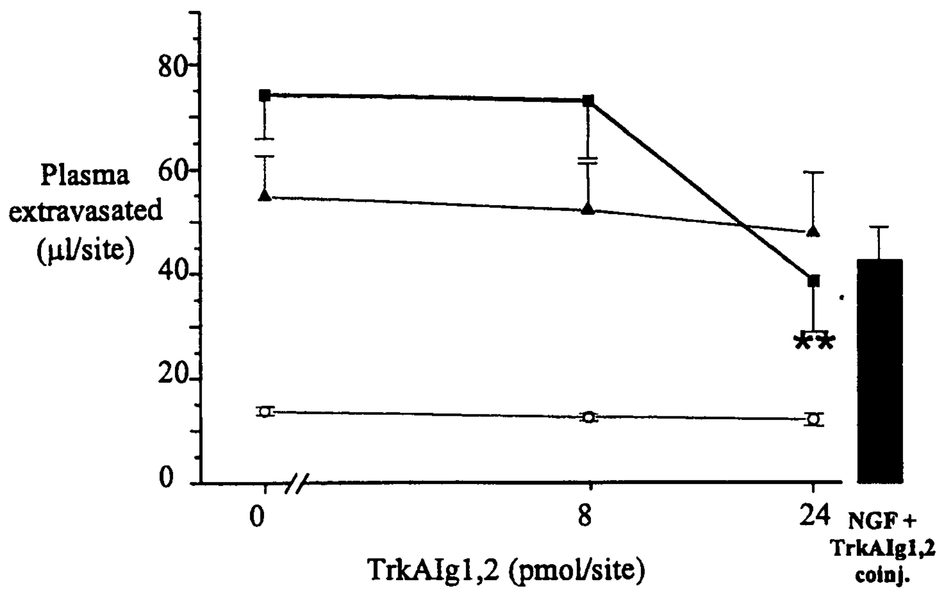
80
60
40
20
0
Plasma
extravasated
(µl/site)
**
0
8
24
NGF +
TrkAIg1,2
coinj.
TrkAIg1,2 (pmol/site)

Fig. 15.

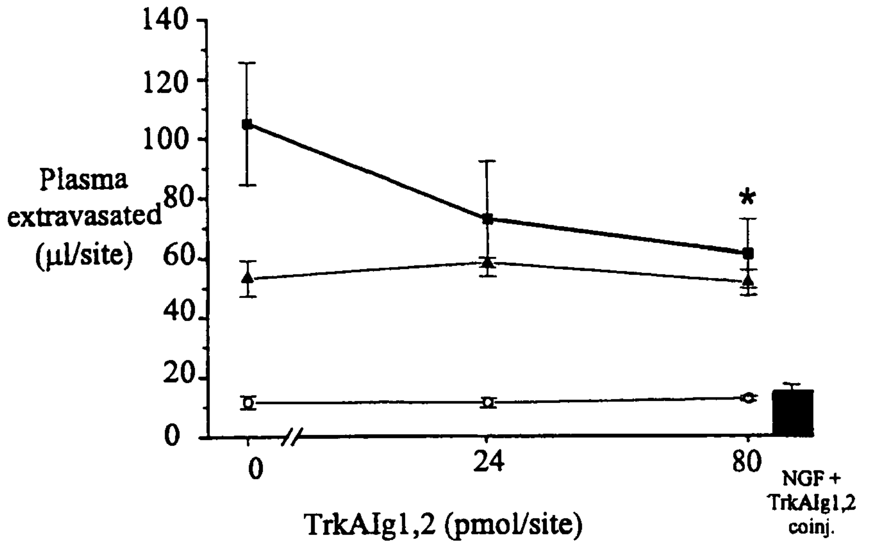
140
120
100
80
60
40
20
0
Plasma extravasated (µl/site)
*
0
24
80
TrkAIg1,2 (pmol/site)
NGF + TrkAIg1,2 coinj.

Fig. 16.

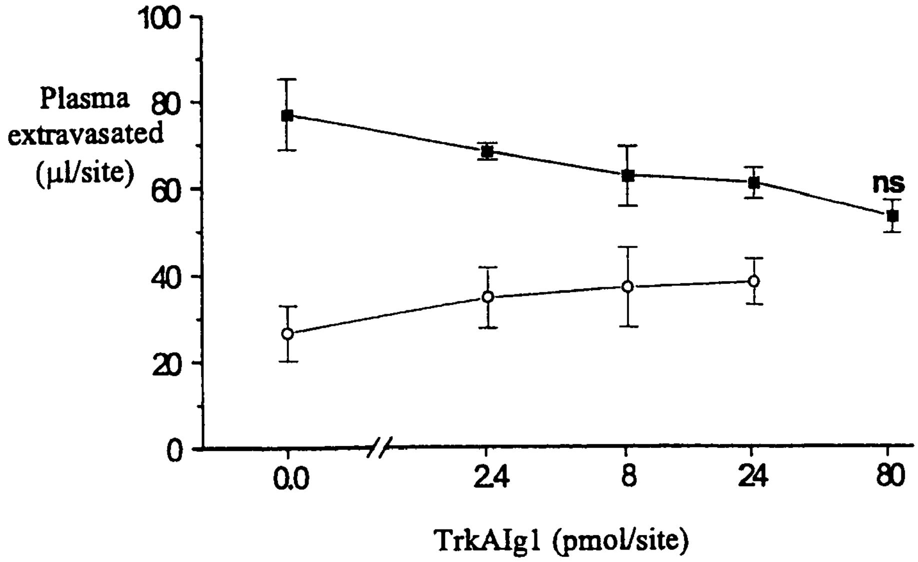
100
80
60
40
20
0
Plasma extravasated (µl/site)
ns
0.0
2.4
8
24
80
TrkAIg1 (pmol/site)

Fig. 17.

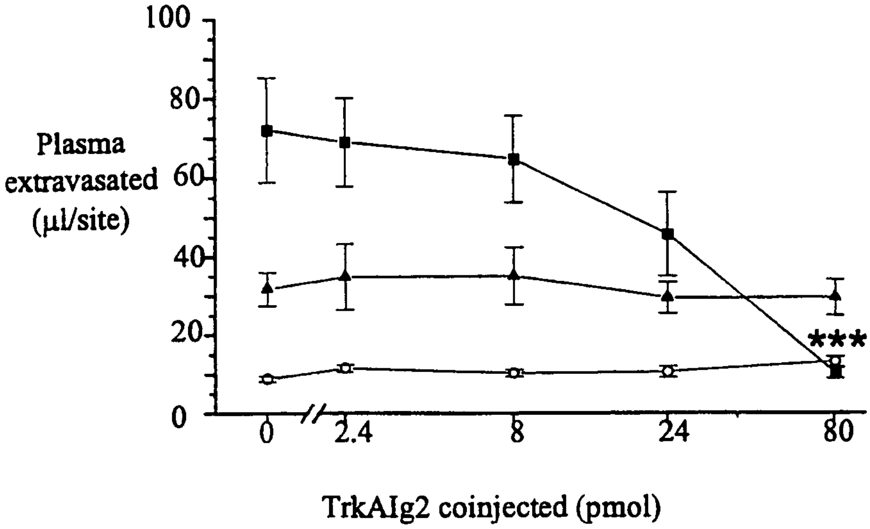
100
80
Plasma
extravasated
(µl/site)
60
40
20
0
***
0
2.4
8
24
80
TrkAIg2 coinjected (pmol)

Fig. 18.

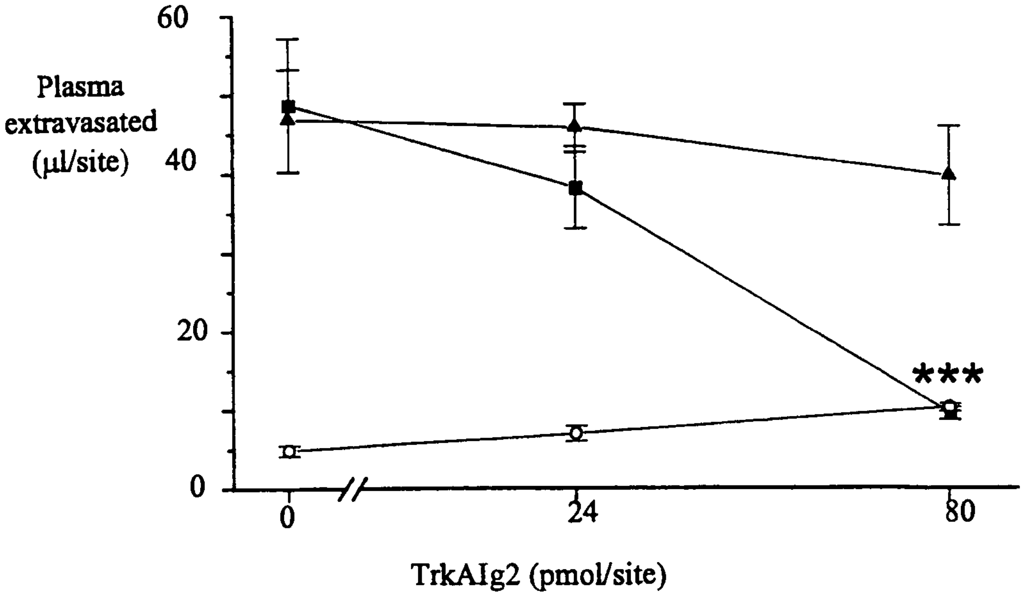
60
Plasma
extravasated
(µl/site)
40
20
0
***
0
24
80
TrkAIg2 (pmol/site)

Fig. 19

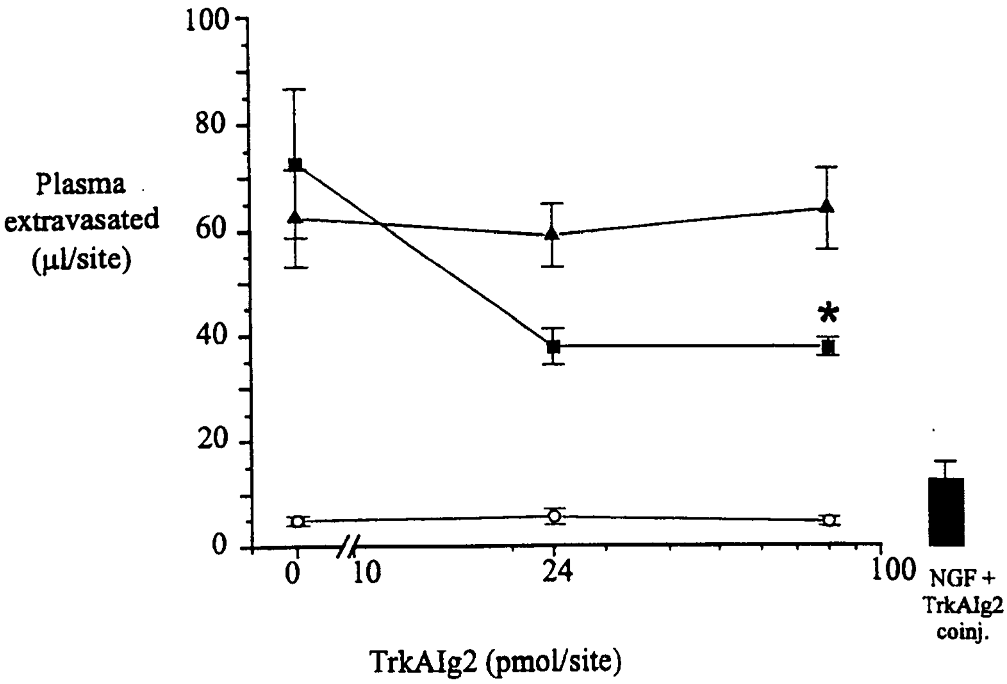
100
80
60
40
20
0
Plasma extravasated (µl/site)
*
0
10
24
100
NGF + TrkAIg2 coinj.
TrkAIg2 (pmol/site)

Fig. 20.

**Fig. 21**

```
REMARK   TrkA domain 2
REMARK
SHEET    1 SEX    SER S   3   LEU S   6
SHEET    2 SEX    HIS S  13   CYS S  16
SHEET    3 SEX    SER S  28   LEU S  32
SHEET    4 SEX    LEU S  38   SER S  42
SHEET    5 SEX    GLU S  47   GLU S  50
SHEET    6 SEX    ALA S  53   VAL S  57
SHEET    7 SEX    CYS S  61   ASN S  65
SHEET    8 SEX    THR S  76   ASN S  81
SHEET    9 SEX    GLY S  84   ALA S  88
TURN     1        PRO S   7   ALA S  11
TURN     2        SER S  17   VAL S  20
TURN     3        LEU S  35   LEU S  37
TURN     4        LEU S  42   HIS S  45
TURN     5        HIS S  51   HIS S  52
TURN     6        THR S  58   THR S  59
TURN     7        MET S  82   MET S  83
SSBOND   1 CYS S   16     CYS S    61
ATOM      1  N   PRO S   1       25.261 138.102  16.261  1.00  0.00
ATOM      2  CA  PRO S   1       25.908 137.182  17.231  1.00  0.00
ATOM      3  C   PRO S   1       27.226 137.778  17.735  1.00  0.00
ATOM      4  O   PRO S   1       27.661 138.818  17.283  1.00  0.00
ATOM      5  CB  PRO S   1       26.158 135.916  16.416  1.00  0.00
ATOM      6  CG  PRO S   1       26.226 136.374  14.994  1.00  0.00
ATOM      7  CD  PRO S   1       25.365 137.605  14.880  1.00  0.00
ATOM      8  N   ALA S   2       27.863 137.129  18.673  1.00  0.00
ATOM      9  CA  ALA S   2       29.147 137.662  19.204  1.00  0.00
ATOM     10  C   ALA S   2       30.334 136.946  18.552  1.00  0.00
ATOM     11  O   ALA S   2       30.246 136.466  17.439  1.00  0.00
ATOM     12  CB  ALA S   2       29.116 137.510  20.735  1.00  0.00
ATOM     13  N   SER S   3       31.442 136.873  19.236  1.00  0.00
ATOM     14  CA  SER S   3       32.633 136.190  18.656  1.00  0.00
ATOM     15  C   SER S   3       33.500 135.589  19.764  1.00  0.00
ATOM     16  O   SER S   3       33.256 135.791  20.937  1.00  0.00
ATOM     17  CB  SER S   3       33.393 137.278  17.924  1.00  0.00
ATOM     18  OG  SER S   3       33.868 138.236  18.860  1.00  0.00
ATOM     19  N   VAL S   4       34.518 134.856  19.396  1.00  0.00
ATOM     20  CA  VAL S   4       35.419 134.237  20.420  1.00  0.00
ATOM     21  C   VAL S   4       36.421 133.276  19.783  1.00  0.00
ATOM     22  O   VAL S   4       36.512 133.123  18.577  1.00  0.00
ATOM     23  CB  VAL S   4       34.556 133.490  21.513  1.00  0.00
ATOM     24  CG1 VAL S   4       33.872 132.169  21.068  1.00  0.00
ATOM     25  CG2 VAL S   4       35.345 133.129  22.795  1.00  0.00
ATOM     26  N   GLN S   5       37.157 132.611  20.620  1.00  0.00
ATOM     27  CA  GLN S   5       38.157 131.621  20.143  1.00  0.00
ATOM     28  C   GLN S   5       38.093 130.394  21.051  1.00  0.00
ATOM     29  O   GLN S   5       38.538 130.421  22.181  1.00  0.00
ATOM     30  CB  GLN S   5       39.552 132.283  20.151  1.00  0.00
ATOM     31  CG  GLN S   5       39.779 133.429  19.110  1.00  0.00
ATOM     32  CD  GLN S   5       39.726 133.100  17.612  1.00  0.00
ATOM     33  OE1 GLN S   5       40.247 132.089  17.171  1.00  0.00
ATOM     34  NE2 GLN S   5       39.138 133.925  16.783  1.00  0.00
ATOM     35  N   LEU S   6       37.535 129.322  20.568  1.00  0.00
ATOM     36  CA  LEU S   6       37.435 128.093  21.423  1.00  0.00
ATOM     37  C   LEU S   6       38.594 127.146  21.128  1.00  0.00
ATOM     38  O   LEU S   6       39.504 127.475  20.397  1.00  0.00
ATOM     39  CB  LEU S   6       36.064 127.391  21.210  1.00  0.00
ATOM     40  CG  LEU S   6       34.840 127.921  22.004  1.00  0.00
```

| | | | | | | | | | | |
|---|---|---|---|---|---|---|---|---|---|---|
| ATOM | 41 | CD1 | LEU | S | 6 | 34.865 | 127.375 | 23.439 | 1.00 | 0.00 |
| ATOM | 42 | CD2 | LEU | S | 6 | 34.772 | 129.458 | 22.035 | 1.00 | 0.00 |
| ATOM | 43 | N | HIS | S | 7 | 38.564 | 125.968 | 21.690 | 1.00 | 0.00 |
| ATOM | 44 | CA | HIS | S | 7 | 39.722 | 125.213 | 21.289 | 1.00 | 0.00 |
| ATOM | 45 | C | HIS | S | 7 | 39.524 | 123.698 | 21.380 | 1.00 | 0.00 |
| ATOM | 46 | O | HIS | S | 7 | 38.384 | 123.188 | 21.436 | 1.00 | 0.00 |
| ATOM | 47 | CB | HIS | S | 7 | 40.908 | 125.664 | 22.188 | 1.00 | 0.00 |
| ATOM | 48 | CG | HIS | S | 7 | 42.119 | 124.819 | 21.872 | 1.00 | 0.00 |
| ATOM | 49 | ND1 | HIS | S | 7 | 42.551 | 124.574 | 20.613 | 1.00 | 0.00 |
| ATOM | 50 | CD2 | HIS | S | 7 | 42.953 | 124.134 | 22.769 | 1.00 | 0.00 |
| ATOM | 51 | CE1 | HIS | S | 7 | 43.603 | 123.745 | 20.744 | 1.00 | 0.00 |
| ATOM | 52 | NE2 | HIS | S | 7 | 43.868 | 123.468 | 22.021 | 1.00 | 0.00 |
| ATOM | 53 | N | THR | S | 8 | 40.680 | 123.000 | 21.431 | 1.00 | 0.00 |
| ATOM | 54 | CA | THR | S | 8 | 40.815 | 121.569 | 21.109 | 1.00 | 0.00 |
| ATOM | 55 | C | THR | S | 8 | 39.587 | 120.616 | 21.219 | 1.00 | 0.00 |
| ATOM | 56 | O | THR | S | 8 | 38..960 | 120.395 | 22.332 | 1.00 | 0.00 |
| ATOM | 57 | CB | THR | S | 8 | 41.972 | 121.037 | 21.933 | 1.00 | 0.00 |
| ATOM | 58 | OG1 | THR | S | 8 | 42.669 | 120.062 | 21.171 | 1.00 | 0.00 |
| ATOM | 59 | CG2 | THR | S | 8 | 41.468 | 120.390 | 23.235 | 1.00 | 0.00 |
| ATOM | 60 | N | ALA | S | 9 | 39.249 | 120.074 | 19.968 | 1.00 | 0.00 |
| ATOM | 61 | CA | ALA | S | 9 | 38.454 | 118.829 | 19.787 | 1.00 | 0.00 |
| ATOM | 62 | C | ALA | S | 9 | 37.251 | 118.752 | 20.710 | 1.00 | 0.00 |
| ATOM | 63 | O | ALA | S | 9 | 36.995 | 117.716 | 21.365 | 1.00 | 0.00 |
| ATOM | 64 | CB | ALA | S | 9 | 39.376 | 117.660 | 20.007 | 1.00 | 0.00 |
| ATOM | 65 | N | VAL | S | 10 | 36.492 | 119.834 | 20.758 | 1.00 | 0.00 |
| ATOM | 66 | CA | VAL | S | 10 | 36.254 | 120.358 | 22.047 | 1.00 | 0.00 |
| ATOM | 67 | C | VAL | S | 10 | 34.802 | 120.909 | 22.345 | 1.00 | 0.00 |
| ATOM | 68 | O | VAL | S | 10 | 33.805 | 120.141 | 22.521 | 1.00 | 0.00 |
| ATOM | 69 | CB | VAL | S | 10 | 37.284 | 121.436 | 22.177 | 1.00 | 0.00 |
| ATOM | 70 | CG1 | VAL | S | 10 | 37.961 | 121.303 | 23.514 | 1.00 | 0.00 |
| ATOM | 71 | CG2 | VAL | S | 10 | 38.331 | 121.282 | 21.066 | 1.00 | 0.00 |
| ATOM | 72 | N | GLU | S | 11 | 34.720 | 122.270 | 22.326 | 1.00 | 0.00 |
| ATOM | 73 | CA | GLU | S | 11 | 34.480 | 123.086 | 23.593 | 1.00 | 0.00 |
| ATOM | 74 | C | GLU | S | 11 | 33.044 | 123.702 | 23.901 | 1.00 | 0.00 |
| ATOM | 75 | O | GLU | S | 11 | 32.262 | 123.138 | 24.739 | 1.00 | 0.00 |
| ATOM | 76 | CB | GLU | S | 11 | 35.519 | 124.181 | 23.462 | 1.00 | 0.00 |
| ATOM | 77 | CG | GLU | S | 11 | 35.501 | 125.138 | 24.601 | 1.00 | 0.00 |
| ATOM | 78 | CD | GLU | S | 11 | 36.545 | 126.192 | 24.357 | 1.00 | 0.00 |
| ATOM | 79 | OE1 | GLU | S | 11 | 36.715 | 127.008 | 25.222 | 1.00 | 0.00 |
| ATOM | 80 | OE2 | GLU | S | 11 | 37.184 | 126.186 | 23.288 | 1.00 | 0.00 |
| ATOM | 81 | N | MET | S | 12 | 32.751 | 124.775 | 23.233 | 1.00 | 0.00 |
| ATOM | 82 | CA | MET | S | 12 | 31.427 | 125.429 | 23.416 | 1.00 | 0.00 |
| ATOM | 83 | C | MET | S | 12 | 31.004 | 126.157 | 22.137 | 1.00 | 0.00 |
| ATOM | 84 | O | MET | S | 12 | 30.315 | 125.609 | 21.300 | 1.00 | 0.00 |
| ATOM | 85 | CB | MET | S | 12 | 31.557 | 126.421 | 24.555 | 1.00 | 0.00 |
| ATOM | 86 | CG | MET | S | 12 | 31.870 | 125.730 | 25.889 | 1.00 | 0.00 |
| ATOM | 87 | SD | MET | S | 12 | 32.043 | 126.899 | 27.244 | 1.00 | 0.00 |
| ATOM | 88 | CE | MET | S | 12 | 32.387 | 125.712 | 28.551 | 1.00 | 0.00 |
| ATOM | 89 | N | HIS | S | 13 | 31.408 | 127.382 | 21.980 | 1.00 | 0.00 |
| ATOM | 90 | CA | HIS | S | 13 | 31.032 | 128.146 | 20.757 | 1.00 | 0.00 |
| ATOM | 91 | C | HIS | S | 13 | 29.527 | 128.109 | 20.528 | 1.00 | 0.00 |
| ATOM | 92 | O | HIS | S | 13 | 28.991 | 127.162 | 19.988 | 1.00 | 0.00 |
| ATOM | 93 | CB | HIS | S | 13 | 31.762 | 127.510 | 19.580 | 1.00 | 0.00 |
| ATOM | 94 | CG | HIS | S | 13 | 33.222 | 127.891 | 19.650 | 1.00 | 0.00 |
| ATOM | 95 | ND1 | HIS | S | 13 | 34.051 | 127.508 | 20.653 | 1.00 | 0.00 |
| ATOM | 96 | CD2 | HIS | S | 13 | 33.950 | 128.671 | 18.746 | 1.00 | 0.00 |
| ATOM | 97 | CE1 | HIS | S | 13 | 35.251 | 128.044 | 20.356 | 1.00 | 0.00 |
| ATOM | 98 | NE2 | HIS | S | 13 | 35.220 | 128.745 | 19.220 | 1.00 | 0.00 |
| ATOM | 99 | N | HIS | S | 14 | 28.850 | 129.145 | 20.904 | 1.00 | 0.00 |
| ATOM | 100 | CA | HIS | S | 14 | 27.382 | 129.193 | 20.683 | 1.00 | 0.00 |
| ATOM | 101 | C | HIS | S | 14 | 27.016 | 130.561 | 20.107 | 1.00 | 0.00 |

| | | | | | | | | | | |
|---|---|---|---|---|---|---|---|---|---|---|
| ATOM | 102 | O | HIS | S | 14 | 27.447 | 131.584 | 20.601 | 1.00 | 0.00 |
| ATOM | 103 | CB | HIS | S | 14 | 26.730 | 128.971 | 22.058 | 1.00 | 0.00 |
| ATOM | 104 | CG | HIS | S | 14 | 27.070 | 127.629 | 22.639 | 1.00 | 0.00 |
| ATOM | 105 | ND1 | HIS | S | 14 | 26.417 | 126.431 | 22.359 | 1.00 | 0.00 |
| ATOM | 106 | CD2 | HIS | S | 14 | 28.105 | 127.445 | 23.544 | 1.00 | 0.00 |
| ATOM | 107 | CE1 | HIS | S | 14 | 27.133 | 125.594 | 23.135 | 1.00 | 0.00 |
| ATOM | 108 | NE2 | HIS | S | 14 | 28.149 | 126.115 | 23.874 | 1.00 | 0.00 |
| ATOM | 109 | N | TRP | S | 15 | 26.242 | 130.593 | 19.062 | 1.00 | 0.00 |
| ATOM | 110 | CA | TRP | S | 15 | 25.876 | 131.900 | 18.460 | 1.00 | 0.00 |
| ATOM | 111 | C | TRP | S | 15 | 24.365 | 131.983 | 18.264 | 1.00 | 0.00 |
| ATOM | 112 | O | TRP | S | 15 | 23.701 | 130.988 | 18.052 | 1.00 | 0.00 |
| ATOM | 113 | CB | TRP | S | 15 | 26.577 | 131.999 | 17.118 | 1.00 | 0.00 |
| ATOM | 114 | CG | TRP | S | 15 | 28.063 | 131.962 | 17.347 | 1.00 | 0.00 |
| ATOM | 115 | CD1 | TRP | S | 15 | 28.908 | 133.072 | 17.566 | 1.00 | 0.00 |
| ATOM | 116 | CD2 | TRP | S | 15 | 28.907 | 130.786 | 17.383 | 1.00 | 0.00 |
| ATOM | 117 | NE1 | TRP | S | 15 | 30.206 | 132.710 | 17.743 | 1.00 | 0.00 |
| ATOM | 118 | CE2 | TRP | S | 15 | 30.232 | 131.231 | 17.622 | 1.00 | 0.00 |
| ATOM | 119 | CE3 | TRP | S | 15 | 28.661 | 129.425 | 17.227 | 1.00 | 0.00 |
| ATOM | 120 | CZ2 | TRP | S | 15 | 31.265 | 130.312 | 17.709 | 1.00 | 0.00 |
| ATOM | 121 | CZ3 | TRP | S | 15 | 29.694 | 128.506 | 17.314 | 1.00 | 0.00 |
| ATOM | 122 | CH2 | TRP | S | 15 | 31.006 | 128.955 | 17.552 | 1.00 | 0.00 |
| ATOM | 123 | N | CYS | S | 16 | 23.816 | 133.160 | 18.327 | 1.00 | 0.00 |
| ATOM | 124 | CA | CYS | S | 16 | 22.457 | 133.496 | 18.183 | 1.00 | 0.00 |
| ATOM | 125 | C | CYS | S | 16 | 22.258 | 134.927 | 18.480 | 1.00 | 0.00 |
| ATOM | 126 | O | CYS | S | 16 | 22.586 | 135.408 | 19.605 | 1.00 | 0.00 |
| ATOM | 127 | CB | CYS | S | 16 | 21.703 | 132.638 | 19.173 | 1.00 | 0.00 |
| ATOM | 128 | SG | CYS | S | 16 | 19.922 | 132.873 | 19.077 | 1.00 | 0.00 |
| ATOM | 129 | N | ILE | S | 17 | 21.796 | 135.659 | 17.467 | 1.00 | 0.00 |
| ATOM | 130 | CA | ILE | S | 17 | 21.625 | 137.083 | 17.695 | 1.00 | 0.00 |
| ATOM | 131 | C | ILE | S | 17 | 20.223 | 137.436 | 18.157 | 1.00 | 0.00 |
| ATOM | 132 | O | ILE | S | 17 | 19.263 | 137.501 | 17.348 | 1.00 | 0.00 |
| ATOM | 133 | CB | ILE | S | 17 | 21.943 | 137.815 | 16.391 | 1.00 | 0.00 |
| ATOM | 134 | CG1 | ILE | S | 17 | 22.054 | 139.327 | 16.656 | 1.00 | 0.00 |
| ATOM | 135 | CG2 | ILE | S | 17 | 20.836 | 137.589 | 15.364 | 1.00 | 0.00 |
| ATOM | 136 | CD1 | ILE | S | 17 | 22.761 | 140.068 | 15.520 | 1.00 | 0.00 |
| ATOM | 137 | N | PRO | S | 18 | 20.084 | 137.607 | 19.471 | 1.00 | 0.00 |
| ATOM | 138 | CA | PRO | S | 18 | 18.821 | 138.065 | 19.995 | 1.00 | 0.00 |
| ATOM | 139 | C | PRO | S | 18 | 18.308 | 139.267 | 19.219 | 1.00 | 0.00 |
| ATOM | 140 | O | PRO | S | 18 | 17.124 | 139.406 | 18.932 | 1.00 | 0.00 |
| ATOM | 141 | CB | PRO | S | 18 | 19.028 | 138.436 | 21.470 | 1.00 | 0.00 |
| ATOM | 142 | CG | PRO | S | 18 | 20.525 | 138.302 | 21.793 | 1.00 | 0.00 |
| ATOM | 143 | CD | PRO | S | 18 | 21.148 | 138.094 | 20.336 | 1.00 | 0.00 |
| ATOM | 144 | N | PHE | S | 19 | 19.228 | 140.179 | 18.875 | 1.00 | 0.00 |
| ATOM | 145 | CA | PHE | S | 19 | 18.797 | 141.326 | 18.074 | 1.00 | 0.00 |
| ATOM | 146 | C | PHE | S | 19 | 18.034 | 140.915 | 16.867 | 1.00 | 0.00 |
| ATOM | 147 | O | PHE | S | 19 | 17.580 | 141.768 | 16.033 | 1.00 | 0.00 |
| ATOM | 148 | CB | PHE | S | 19 | 20.025 | 142.135 | 17.663 | 1.00 | 0.00 |
| ATOM | 149 | CG | PHE | S | 19 | 19.895 | 143.469 | 18.331 | 1.00 | 0.00 |
| ATOM | 150 | CD1 | PHE | S | 19 | 20.170 | 143.597 | 19.685 | 1.00 | 0.00 |
| ATOM | 151 | CD2 | PHE | S | 19 | 19.393 | 144.542 | 17.612 | 1.00 | 0.00 |
| ATOM | 152 | CE1 | PHE | S | 19 | 19.930 | 144.800 | 20.327 | 1.00 | 0.00 |
| ATOM | 153 | CE2 | PHE | S | 19 | 19.160 | 145.750 | 18.264 | 1.00 | 0.00 |
| ATOM | 154 | CZ | PHE | S | 19 | 19.424 | 145.880 | 19.624 | 1.00 | 0.00 |
| ATOM | 155 | N | SER | S | 20 | 17.950 | 139.567 | 16.695 | 1.00 | 0.00 |
| ATOM | 156 | CA | SER | S | 20 | 16.868 | 138.992 | 15.860 | 1.00 | 0.00 |
| ATOM | 157 | C | SER | S | 20 | 17.080 | 139.163 | 14.368 | 1.00 | 0.00 |
| ATOM | 158 | O | SER | S | 20 | 17.855 | 140.071 | 13.942 | 1.00 | 0.00 |
| ATOM | 159 | CB | SER | S | 20 | 15.575 | 139.695 | 16.291 | 1.00 | 0.00 |
| ATOM | 160 | OG | SER | S | 20 | 15.837 | 141.102 | 16.423 | 1.00 | 0.00 |
| ATOM | 161 | N | VAL | S | 21 | 15.885 | 138.323 | 13.764 | 1.00 | 0.00 |
| ATOM | 162 | CA | VAL | S | 21 | 15.991 | 138.891 | 12.422 | 1.00 | 0.00 |

| | | | | | | | | | | |
|---|---|---|---|---|---|---|---|---|---|---|
| ATOM | 163 | C | VAL | S | 21 | 14.596 | 139.156 | 11.835 | 1.00 | 0.00 |
| ATOM | 164 | O | VAL | S | 21 | 13.712 | 138.263 | 11.884 | 1.00 | 0.00 |
| ATOM | 165 | CB | VAL | S | 21 | 16.723 | 137.887 | 11.521 | 1.00 | 0.00 |
| ATOM | 166 | CG1 | VAL | S | 21 | 16.178 | 136.482 | 11.752 | 1.00 | 0.00 |
| ATOM | 167 | CG2 | VAL | S | 21 | 16.517 | 138.257 | 10.049 | 1.00 | 0.00 |
| ATOM | 168 | N | ASP | S | 22 | 14.140 | 140.310 | 11.434 | 1.00 | 0.00 |
| ATOM | 169 | CA | ASP | S | 22 | 12.707 | 140.438 | 11.055 | 1.00 | 0.00 |
| ATOM | 170 | C | ASP | S | 22 | 12.549 | 140.092 | 9.579 | 1.00 | 0.00 |
| ATOM | 171 | O | ASP | S | 22 | 13.508 | 140.011 | 8.840 | 1.00 | 0.00 |
| ATOM | 172 | CB | ASP | S | 22 | 12.329 | 141.897 | 11.323 | 1.00 | 0.00 |
| ATOM | 173 | CG | ASP | S | 22 | 12.290 | 142.179 | 12.831 | 1.00 | 0.00 |
| ATOM | 174 | OD1 | ASP | S | 22 | 11.957 | 141.271 | 13.575 | 1.00 | 0.00 |
| ATOM | 175 | OD2 | ASP | S | 22 | 12.595 | 143.297 | 13.212 | 1.00 | 0.00 |
| ATOM | 176 | N | GLY | S | 23 | 11.345 | 139.890 | 9.152 | 1.00 | 0.00 |
| ATOM | 177 | CA | GLY | S | 23 | 11.114 | 139.540 | 7.722 | 1.00 | 0.00 |
| ATOM | 178 | C | GLY | S | 23 | 9.731 | 140.014 | 7.282 | 1.00 | 0.00 |
| ATOM | 179 | O | GLY | S | 23 | 9.222 | 141.007 | 7.764 | 1.00 | 0.00 |
| ATOM | 180 | N | GLN | S | 24 | 9.118 | 139.316 | 6.363 | 1.00 | 0.00 |
| ATOM | 181 | CA | GLN | S | 24 | 7.769 | 139.736 | 5.890 | 1.00 | 0.00 |
| ATOM | 182 | C | GLN | S | 24 | 7.193 | 138.705 | 4.901 | 1.00 | 0.00 |
| ATOM | 183 | O | GLN | S | 24 | 7.087 | 138.988 | 3.725 | 1.00 | 0.00 |
| ATOM | 184 | CB | GLN | S | 24 | 7.885 | 141.130 | 5.236 | 1.00 | 0.00 |
| ATOM | 185 | CG | GLN | S | 24 | 6.539 | 141.817 | 4.827 | 1.00 | 0.00 |
| ATOM | 186 | CD | GLN | S | 24 | 6.577 | 143.203 | 4.172 | 1.00 | 0.00 |
| ATOM | 187 | OE1 | GLN | S | 24 | 7.637 | 143.767 | 3.950 | 1.00 | 0.00 |
| ATOM | 188 | NE2 | GLN | S | 24 | 5.460 | 143.813 | 3.867 | 1.00 | 0.00 |
| ATOM | 189 | N | PRO | S | 25 | 6.805 | 137.546 | 5.398 | 1.00 | 0.00 |
| ATOM | 190 | CA | PRO | S | 25 | 6.929 | 137.190 | 6.830 | 1.00 | 0.00 |
| ATOM | 191 | C | PRO | S | 25 | 8.136 | 136.266 | 7.043 | 1.00 | 0.00 |
| ATOM | 192 | O | PRO | S | 25 | 8.532 | 135.546 | 6.150 | 1.00 | 0.00 |
| ATOM | 193 | CB | PRO | S | 25 | 5.633 | 136.428 | 7.090 | 1.00 | 0.00 |
| ATOM | 194 | CG | PRO | S | 25 | 5.209 | 135.877 | 5.752 | 1.00 | 0.00 |
| ATOM | 195 | CD | PRO | S | 25 | 6.086 | 136.497 | 4.685 | 1.00 | 0.00 |
| ATOM | 196 | N | ALA | S | 26 | 8.702 | 136.264 | 8.222 | 1.00 | 0.00 |
| ATOM | 197 | CA | ALA | S | 26 | 9.862 | 135.375 | 8.510 | 1.00 | 0.00 |
| ATOM | 198 | C | ALA | S | 26 | 10.981 | 135.547 | 7.467 | 1.00 | 0.00 |
| ATOM | 199 | O | ALA | S | 26 | 10.858 | 135.095 | 6.346 | 1.00 | 0.00 |
| ATOM | 200 | CB | ALA | S | 26 | 9.336 | 133.931 | 8.577 | 1.00 | 0.00 |
| ATOM | 201 | N | PRO | S | 27 | 12.050 | 136.187 | 7.874 | 1.00 | 0.00 |
| ATOM | 202 | CA | PRO | S | 27 | 13.198 | 136.405 | 6.956 | 1.00 | 0.00 |
| ATOM | 203 | C | PRO | S | 27 | 13.952 | 135.095 | 6.713 | 1.00 | 0.00 |
| ATOM | 204 | O | PRO | S | 27 | 13.526 | 134.033 | 7.120 | 1.00 | 0.00 |
| ATOM | 205 | CB | PRO | S | 27 | 14.085 | 137.384 | 7.719 | 1.00 | 0.00 |
| ATOM | 206 | CG | PRO | S | 27 | 13.745 | 137.163 | 9.158 | 1.00 | 0.00 |
| ATOM | 207 | CD | PRO | S | 27 | 12.296 | 136.763 | 9.204 | 1.00 | 0.00 |
| ATOM | 208 | N | SER | S | 28 | 15.081 | 135.173 | 6.064 | 1.00 | 0.00 |
| ATOM | 209 | CA | SER | S | 28 | 15.887 | 133.951 | 5.801 | 1.00 | 0.00 |
| ATOM | 210 | C | SER | S | 28 | 17.330 | 134.208 | 6.234 | 1.00 | 0.00 |
| ATOM | 211 | O | SER | S | 28 | 17.789 | 135.332 | 6.234 | 1.00 | 0.00 |
| ATOM | 212 | CB | SER | S | 28 | 15.773 | 133.525 | 4.316 | 1.00 | 0.00 |
| ATOM | 213 | OG | SER | S | 28 | 16.338 | 134.484 | 3.416 | 1.00 | 0.00 |
| ATOM | 214 | N | LEU | S | 29 | 18.050 | 133.191 | 6.611 | 1.00 | 0.00 |
| ATOM | 215 | CA | LEU | S | 29 | 19.466 | 133.421 | 7.049 | 1.00 | 0.00 |
| ATOM | 216 | C | LEU | S | 29 | 20.429 | 132.569 | 6.214 | 1.00 | 0.00 |
| ATOM | 217 | O | LEU | S | 29 | 20.055 | 131.551 | 5.664 | 1.00 | 0.00 |
| ATOM | 218 | CB | LEU | S | 29 | 19.622 | 133.124 | 8.568 | 1.00 | 0.00 |
| ATOM | 219 | CG | LEU | S | 29 | 19.235 | 134.240 | 9.575 | 1.00 | 0.00 |
| ATOM | 220 | CD1 | LEU | S | 29 | 20.371 | 135.266 | 9.687 | 1.00 | 0.00 |
| ATOM | 221 | CD2 | LEU | S | 29 | 17.927 | 134.955 | 9.196 | 1.00 | 0.00 |
| ATOM | 222 | N | ARG | S | 30 | 21.666 | 132.977 | 6.111 | 1.00 | 0.00 |
| ATOM | 223 | CA | ARG | S | 30 | 22.641 | 132.189 | 5.308 | 1.00 | 0.00 |

| | | | | | | | | | | |
|---|---|---|---|---|---|---|---|---|---|---|
| ATOM | 224 | C | ARG | S | 30 | 24.018 | 132.227 | 5.965 | 1.00 | 0.00 |
| ATOM | 225 | O | ARG | S | 30 | 24.899 | 132.935 | 5.524 | 1.00 | 0.00 |
| ATOM | 226 | CB | ARG | S | 30 | 22.676 | 132.754 | 3.862 | 1.00 | 0.00 |
| ATOM | 227 | CG | ARG | S | 30 | 23.417 | 131.864 | 2.829 | 1.00 | 0.00 |
| ATOM | 228 | CD | ARG | S | 30 | 23.445 | 132.487 | 1.429 | 1.00 | 0.00 |
| ATOM | 229 | NE | ARG | S | 30 | 24.147 | 131.551 | 0.514 | 1.00 | 0.00 |
| ATOM | 230 | CZ | ARG | S | 30 | 24.360 | 131.752 | -0.779 | 1.00 | 0.00 |
| ATOM | 231 | NH1 | ARG | S | 30 | 23.980 | 132.812 | -1.429 | 1.00 | 0.00 |
| ATOM | 232 | NH2 | ARG | S | 30 | 24.983 | 130.833 | -1.428 | 1.00 | 0.00 |
| ATOM | 233 | N | TRP | S | 31 | 24.224 | 131.467 | 7.007 | 1.00 | 0.00 |
| ATOM | 234 | CA | TRP | S | 31 | 25.571 | 131.477 | 7.655 | 1.00 | 0.00 |
| ATOM | 235 | C | TRP | S | 31 | 26.608 | 131.059 | 6.620 | 1.00 | 0.00 |
| ATOM | 236 | O | TRP | S | 31 | 26.372 | 130.178 | 5.818 | 1.00 | 0.00 |
| ATOM | 237 | CB | TRP | S | 31 | 25.490 | 130.474 | 8.809 | 1.00 | 0.00 |
| ATOM | 238 | CG | TRP | S | 31 | 24.550 | 131.007 | 9.829 | 1.00 | 0.00 |
| ATOM | 239 | CD1 | TRP | S | 31 | 23.216 | 130.849 | 9.797 | 1.00 | 0.00 |
| ATOM | 240 | CD2 | TRP | S | 31 | 24.845 | 131.793 | 11.019 | 1.00 | 0.00 |
| ATOM | 241 | NE1 | TRP | S | 31 | 22.661 | 131.495 | 10.886 | 1.00 | 0.00 |
| ATOM | 242 | CE2 | TRP | S | 31 | 23.627 | 132.093 | 11.670 | 1.00 | 0.00 |
| ATOM | 243 | CE3 | TRP | S | 31 | 26.038 | 132.273 | 11.588 | 1.00 | 0.00 |
| ATOM | 244 | CZ2 | TRP | S | 31 | 23.594 | 132.846 | 12.844 | 1.00 | 0.00 |
| ATOM | 245 | CZ3 | TRP | S | 31 | 26.005 | 133.028 | 12.770 | 1.00 | 0.00 |
| ATOM | 246 | CH2 | TRP | S | 31 | 24.787 | 133.315 | 13.397 | 1.00 | 0.00 |
| ATOM | 247 | N | LEU | S | 32 | 27.742 | 131.699 | 6.616 | 1.00 | 0.00 |
| ATOM | 248 | CA | LEU | S | 32 | 28.784 | 131.367 | 5.611 | 1.00 | 0.00 |
| ATOM | 249 | C | LEU | S | 32 | 30.043 | 132.188 | 5.861 | 1.00 | 0.00 |
| ATOM | 250 | O | LEU | S | 32 | 29.992 | 133.349 | 6.218 | 1.00 | 0.00 |
| ATOM | 251 | CB | LEU | S | 32 | 28.152 | 131.687 | 4.232 | 1.00 | 0.00 |
| ATOM | 252 | CG | LEU | S | 32 | 27.768 | 133.174 | 4.091 | 1.00 | 0.00 |
| ATOM | 253 | CD1 | LEU | S | 32 | 28.993 | 133.957 | 3.631 | 1.00 | 0.00 |
| ATOM | 254 | CD2 | LEU | S | 32 | 26.664 | 133.348 | 3.045 | 1.00 | 0.00 |
| ATOM | 255 | N | PHE | S | 33 | 31.169 | 131.582 | 5.667 | 1.00 | 0.00 |
| ATOM | 256 | CA | PHE | S | 33 | 32.454 | 132.282 | 5.871 | 1.00 | 0.00 |
| ATOM | 257 | C | PHE | S | 33 | 33.583 | 131.325 | 5.513 | 1.00 | 0.00 |
| ATOM | 258 | O | PHE | S | 33 | 34.361 | 130.920 | 6.353 | 1.00 | 0.00 |
| ATOM | 259 | CB | PHE | S | 33 | 32.585 | 132.738 | 7.356 | 1.00 | 0.00 |
| ATOM | 260 | CG | PHE | S | 33 | 32.691 | 131.623 | 8.407 | 1.00 | 0.00 |
| ATOM | 261 | CD1 | PHE | S | 33 | 33.950 | 131.117 | 8.756 | 1.00 | 0.00 |
| ATOM | 262 | CD2 | PHE | S | 33 | 31.545 | 131.063 | 8.975 | 1.00 | 0.00 |
| ATOM | 263 | CE1 | PHE | S | 33 | 34.058 | 130.055 | 9.645 | 1.00 | 0.00 |
| ATOM | 264 | CE2 | PHE | S | 33 | 31.654 | 130.005 | 9.873 | 1.00 | 0.00 |
| ATOM | 265 | CZ | PHE | S | 33 | 32.910 | 129.498 | 10.206 | 1.00 | 0.00 |
| ATOM | 266 | N | ASN | S | 34 | 33.650 | 130.914 | 4.276 | 1.00 | 0.00 |
| ATOM | 267 | CA | ASN | S | 34 | 34.697 | 129.928 | 3.887 | 1.00 | 0.00 |
| ATOM | 268 | C | ASN | S | 34 | 34.514 | 128.668 | 4.739 | 1.00 | 0.00 |
| ATOM | 269 | O | ASN | S | 34 | 35.424 | 127.883 | 4.918 | 1.00 | 0.00 |
| ATOM | 270 | CB | ASN | S | 34 | 36.106 | 130.563 | 4.077 | 1.00 | 0.00 |
| ATOM | 271 | CG | ASN | S | 34 | 36.518 | 131.648 | 3.075 | 1.00 | 0.00 |
| ATOM | 272 | OD1 | ASN | S | 34 | 36.324 | 131.533 | 1.874 | 1.00 | 0.00 |
| ATOM | 273 | ND2 | ASN | S | 34 | 37.115 | 132.721 | 3.520 | 1.00 | 0.00 |
| ATOM | 274 | N | GLY | S | 35 | 33.328 | 128.478 | 5.271 | 1.00 | 0.00 |
| ATOM | 275 | CA | GLY | S | 35 | 33.056 | 127.286 | 6.120 | 1.00 | 0.00 |
| ATOM | 276 | C | GLY | S | 35 | 31.881 | 126.501 | 5.536 | 1.00 | 0.00 |
| ATOM | 277 | O | GLY | S | 35 | 31.116 | 125.886 | 6.251 | 1.00 | 0.00 |
| ATOM | 278 | N | SER | S | 36 | 31.723 | 126.528 | 4.239 | 1.00 | 0.00 |
| ATOM | 279 | CA | SER | S | 36 | 30.588 | 125.793 | 3.607 | 1.00 | 0.00 |
| ATOM | 280 | C | SER | S | 36 | 29.269 | 126.322 | 4.163 | 1.00 | 0.00 |
| ATOM | 281 | O | SER | S | 36 | 28.500 | 125.596 | 4.760 | 1.00 | 0.00 |
| ATOM | 282 | CB | SER | S | 36 | 30.744 | 124.264 | 3.804 | 1.00 | 0.00 |
| ATOM | 283 | OG | SER | S | 36 | 31.915 | 123.737 | 3.169 | 1.00 | 0.00 |
| ATOM | 284 | N | VAL | S | 37 | 29.020 | 127.595 | 3.969 | 1.00 | 0.00 |

| | | | | | | | | | | |
|---|---|---|---|---|---|---|---|---|---|---|
| ATOM | 285 | CA | VAL | S | 37 | 27.811 | 128.253 | 4.425 | 1.00 | 0.00 |
| ATOM | 286 | C | VAL | S | 37 | 27.367 | 127.743 | 5.797 | 1.00 | 0.00 |
| ATOM | 287 | O | VAL | S | 37 | 26.149 | 127.679 | 6.103 | 1.00 | 0.00 |
| ATOM | 288 | CB | VAL | S | 37 | 26.714 | 127.993 | 3.378 | 1.00 | 0.00 |
| ATOM | 289 | CG1 | VAL | S | 37 | 25.359 | 128.461 | 3.900 | 1.00 | 0.00 |
| ATOM | 290 | CG2 | VAL | S | 37 | 27.032 | 128.743 | 2.086 | 1.00 | 0.00 |
| ATOM | 291 | N | LEU | S | 38 | 28.366 | 127.332 | 6.620 | 1.00 | 0.00 |
| ATOM | 292 | CA | LEU | S | 38 | 28.052 | 126.711 | 7.913 | 1.00 | 0.00 |
| ATOM | 293 | C | LEU | S | 38 | 26.753 | 125.947 | 7.891 | 1.00 | 0.00 |
| ATOM | 294 | O | LEU | S | 38 | 26.705 | 124.711 | 8.147 | 1.00 | 0.00 |
| ATOM | 295 | CB | LEU | S | 38 | 27.925 | 127.809 | 8.970 | 1.00 | 0.00 |
| ATOM | 296 | CG | LEU | S | 38 | 29.126 | 128.735 | 9.025 | 1.00 | 0.00 |
| ATOM | 297 | CD1 | LEU | S | 38 | 28.777 | 130.105 | 9.613 | 1.00 | 0.00 |
| ATOM | 298 | CD2 | LEU | S | 38 | 30.247 | 128.170 | 9.896 | 1.00 | 0.00 |
| ATOM | 299 | N | ASN | S | 39 | 25.675 | 126.692 | 7.632 | 1.00 | 0.00 |
| ATOM | 300 | CA | ASN | S | 39 | 24.357 | 126.130 | 7.806 | 1.00 | 0.00 |
| ATOM | 301 | C | ASN | S | 39 | 23.279 | 126.989 | 7.185 | 1.00 | 0.00 |
| ATOM | 302 | O | ASN | S | 39 | 23.465 | 128.203 | 6.943 | 1.00 | 0.00 |
| ATOM | 303 | CB | ASN | S | 39 | 24.112 | 126.005 | 9.301 | 1.00 | 0.00 |
| ATOM | 304 | CG | ASN | S | 39 | 22.686 | 125.595 | 9.516 | 1.00 | 0.00 |
| ATOM | 305 | OD1 | ASN | S | 39 | 22.347 | 124.420 | 9.586 | 1.00 | 0.00 |
| ATOM | 306 | ND2 | ASN | S | 39 | 21.824 | 126.622 | 9.609 | 1.00 | 0.00 |
| ATOM | 307 | N | GLU | S | 40 | 22.111 | 126.468 | 6.900 | 1.00 | 0.00 |
| ATOM | 308 | CA | GLU | S | 40 | 21.022 | 127.276 | 6.398 | 1.00 | 0.00 |
| ATOM | 309 | C | GLU | S | 40 | 19.698 | 126.735 | 6.922 | 1.00 | 0.00 |
| ATOM | 310 | O | GLU | S | 40 | 19.501 | 125.521 | 6.855 | 1.00 | 0.00 |
| ATOM | 311 | CB | GLU | S | 40 | 20.992 | 127.237 | 4.894 | 1.00 | 0.00 |
| ATOM | 312 | CG | GLU | S | 40 | 19.960 | 128.182 | 4.298 | 1.00 | 0.00 |
| ATOM | 313 | CD | GLU | S | 40 | 19.931 | 128.272 | 2.794 | 1.00 | 0.00 |
| ATOM | 314 | OE1 | GLU | S | 40 | 19.218 | 129.105 | 2.210 | 1.00 | 0.00 |
| ATOM | 315 | OE2 | GLU | S | 40 | 20.658 | 127.492 | 2.172 | 1.00 | 0.00 |
| ATOM | 316 | N | THR | S | 41 | 18.841 | 127.619 | 7.441 | 1.00 | 0.00 |
| ATOM | 317 | CA | THR | S | 41 | 17.464 | 127.340 | 7.853 | 1.00 | 0.00 |
| ATOM | 318 | C | THR | S | 41 | 16.612 | 127.970 | 6.761 | 1.00 | 0.00 |
| ATOM | 319 | O | THR | S | 41 | 16.859 | 129.136 | 6.461 | 1.00 | 0.00 |
| ATOM | 320 | CB | THR | S | 41 | 17.194 | 127.906 | 9.288 | 1.00 | 0.00 |
| ATOM | 321 | OG1 | THR | S | 41 | 18.058 | 127.294 | 10.236 | 1.00 | 0.00 |
| ATOM | 322 | CG2 | THR | S | 41 | 15.776 | 127.683 | 9.857 | 1.00 | 0.00 |
| ATOM | 323 | N | SER | S | 42 | 15.627 | 127.286 | 6.201 | 1.00 | 0.00 |
| ATOM | 324 | CA | SER | S | 42 | 14.747 | 127.887 | 5.217 | 1.00 | 0.00 |
| ATOM | 325 | C | SER | S | 42 | 13.377 | 127.367 | 5.584 | 1.00 | 0.00 |
| ATOM | 326 | O | SER | S | 42 | 13.266 | 126.188 | 5.873 | 1.00 | 0.00 |
| ATOM | 327 | CB | SER | S | 42 | 15.220 | 127.555 | 3.779 | 1.00 | 0.00 |
| ATOM | 328 | OG | SER | S | 42 | 16.514 | 128.089 | 3.481 | 1.00 | 0.00 |
| ATOM | 329 | N | PHE | S | 43 | 12.369 | 128.209 | 5.531 | 1.00 | 0.00 |
| ATOM | 330 | CA | PHE | S | 43 | 11.048 | 127.921 | 6.037 | 1.00 | 0.00 |
| ATOM | 331 | C | PHE | S | 43 | 10.341 | 126.626 | 5.679 | 1.00 | 0.00 |
| ATOM | 332 | O | PHE | S | 43 | 10.229 | 125.847 | 6.621 | 1.00 | 0.00 |
| ATOM | 333 | CB | PHE | S | 43 | 10.046 | 129.023 | 5.575 | 1.00 | 0.00 |
| ATOM | 334 | CG | PHE | S | 43 | 8.606 | 128.898 | 6.093 | 1.00 | 0.00 |
| ATOM | 335 | CD1 | PHE | S | 43 | 8.324 | 129.188 | 7.433 | 1.00 | 0.00 |
| ATOM | 336 | CD2 | PHE | S | 43 | 7.583 | 128.438 | 5.261 | 1.00 | 0.00 |
| ATOM | 337 | CE1 | PHE | S | 43 | 7.041 | 129.003 | 7.937 | 1.00 | 0.00 |
| ATOM | 338 | CE2 | PHE | S | 43 | 6.297 | 128.263 | 5.763 | 1.00 | 0.00 |
| ATOM | 339 | CZ | PHE | S | 43 | 6.027 | 128.542 | 7.102 | 1.00 | 0.00 |
| ATOM | 340 | N | ILE | S | 44 | 9.835 | 126.212 | 4.530 | 1.00 | 0.00 |
| ATOM | 341 | CA | ILE | S | 44 | 9.139 | 124.919 | 4.536 | 1.00 | 0.00 |
| ATOM | 342 | C | ILE | S | 44 | 10.107 | 123.838 | 4.134 | 1.00 | 0.00 |
| ATOM | 343 | O | ILE | S | 44 | 9.842 | 123.092 | 3.206 | 1.00 | 0.00 |
| ATOM | 344 | CB | ILE | S | 44 | 7.844 | 124.957 | 3.627 | 1.00 | 0.00 |
| ATOM | 345 | CG1 | ILE | S | 44 | 8.119 | 125.214 | 2.111 | 1.00 | 0.00 |

| | | | | | | | | | | |
|---|---|---|---|---|---|---|---|---|---|---|
| ATOM | 346 | CG2 | ILE | S | 44 | 6.794 | 126.003 | 4.113 | 1.00 | 0.00 |
| ATOM | 347 | CD1 | ILE | S | 44 | 6.959 | 124.882 | 1.149 | 1.00 | 0.00 |
| ATOM | 348 | N | PHE | S | 45 | 11.226 | 123.703 | 4.810 | 1.00 | 0.00 |
| ATOM | 349 | CA | PHE | S | 45 | 12.233 | 122.765 | 4.373 | 1.00 | 0.00 |
| ATOM | 350 | C | PHE | S | 45 | 13.025 | 122.195 | 5.507 | 1.00 | 0.00 |
| ATOM | 351 | O | PHE | S | 45 | 12.819 | 122.593 | 6.650 | 1.00 | 0.00 |
| ATOM | 352 | CB | PHE | S | 45 | 13.228 | 123.464 | 3.397 | 1.00 | 0.00 |
| ATOM | 353 | CG | PHE | S | 45 | 12.619 | 124.092 | 2.134 | 1.00 | 0.00 |
| ATOM | 354 | CD1 | PHE | S | 45 | 12.159 | 125.413 | 2.170 | 1.00 | 0.00 |
| ATOM | 355 | CD2 | PHE | S | 45 | 12.462 | 123.341 | 0.966 | 1.00 | 0.00 |
| ATOM | 356 | CE1 | PHE | S | 45 | 11.533 | 125.970 | 1.060 | 1.00 | 0.00 |
| ATOM | 357 | CE2 | PHE | S | 45 | 11.845 | 123.902 | -0.148 | 1.00 | 0.00 |
| ATOM | 358 | CZ | PHE | S | 45 | 11.378 | 125.215 | -0.101 | 1.00 | 0.00 |
| ATOM | 359 | N | THR | S | 46 | 13.898 | 121.240 | 5.218 | 1.00 | 0.00 |
| ATOM | 360 | CA | THR | S | 46 | 14.725 | 120.690 | 6.282 | 1.00 | 0.00 |
| ATOM | 361 | C | THR | S | 46 | 16.076 | 121.403 | 6.180 | 1.00 | 0.00 |
| ATOM | 362 | O | THR | S | 46 | 16.491 | 121.794 | 5.089 | 1.00 | 0.00 |
| ATOM | 363 | CB | THR | S | 46 | 14.833 | 119.134 | 6.146 | 1.00 | 0.00 |
| ATOM | 364 | OG1 | THR | S | 46 | 15.519 | 118.784 | 4.951 | 1.00 | 0.00 |
| ATOM | 365 | CG2 | THR | S | 46 | 13.500 | 118.359 | 6.067 | 1.00 | 0.00 |
| ATOM | 366 | N | GLU | S | 47 | 16.680 | 121.725 | 7.327 | 1.00 | 0.00 |
| ATOM | 367 | CA | GLU | S | 47 | 18.008 | 122.306 | 7.440 | 1.00 | 0.00 |
| ATOM | 368 | C | GLU | S | 47 | 19.157 | 121.726 | 6.653 | 1.00 | 0.00 |
| ATOM | 369 | O | GLU | S | 47 | 19.285 | 120.524 | 6.462 | 1.00 | 0.00 |
| ATOM | 370 | CB | GLU | S | 47 | 18.437 | 122.270 | 8.933 | 1.00 | 0.00 |
| ATOM | 371 | CG | GLU | S | 47 | 17.611 | 123.139 | 9.939 | 1.00 | 0.00 |
| ATOM | 372 | CD | GLU | S | 47 | 16.261 | 122.605 | 10.424 | 1.00 | 0.00 |
| ATOM | 373 | OE1 | GLU | S | 47 | 15.865 | 121.475 | 10.175 | 1.00 | 0.00 |
| ATOM | 374 | OE2 | GLU | S | 47 | 15.547 | 123.506 | 11.156 | 1.00 | 0.00 |
| ATOM | 375 | N | PHE | S | 48 | 20.009 | 122.619 | 6.241 | 1.00 | 0.00 |
| ATOM | 376 | CA | PHE | S | 48 | 21.214 | 122.238 | 5.567 | 1.00 | 0.00 |
| ATOM | 377 | C | PHE | S | 48 | 22.243 | 122.515 | 6.644 | 1.00 | 0.00 |
| ATOM | 378 | O | PHE | S | 48 | 22.276 | 123.633 | 7.151 | 1.00 | 0.00 |
| ATOM | 379 | CB | PHE | S | 48 | 21.486 | 123.120 | 4.329 | 1.00 | 0.00 |
| ATOM | 380 | CG | PHE | S | 48 | 20.461 | 122.941 | 3.240 | 1.00 | 0.00 |
| ATOM | 381 | CD1 | PHE | S | 48 | 19.451 | 123.873 | 3.049 | 1.00 | 0.00 |
| ATOM | 382 | CD2 | PHE | S | 48 | 20.396 | 121.768 | 2.510 | 1.00 | 0.00 |
| ATOM | 383 | CE1 | PHE | S | 48 | 18.412 | 123.649 | 2.168 | 1.00 | 0.00 |
| ATOM | 384 | CE2 | PHE | S | 48 | 19.356 | 121.533 | 1.629 | 1.00 | 0.00 |
| ATOM | 385 | CZ | PHE | S | 48 | 18.360 | 122.471 | 1.457 | 1.00 | 0.00 |
| ATOM | 386 | N | LEU | S | 49 | 23.057 | 121.530 | 6.983 | 1.00 | 0.00 |
| ATOM | 387 | CA | LEU | S | 49 | 24.204 | 121.685 | 7.871 | 1.00 | 0.00 |
| ATOM | 388 | C | LEU | S | 49 | 25.370 | 121.144 | 7.051 | 1.00 | 0.00 |
| ATOM | 389 | O | LEU | S | 49 | 25.182 | 120.088 | 6.465 | 1.00 | 0.00 |
| ATOM | 390 | CB | LEU | S | 49 | 24.120 | 120.835 | 9.146 | 1.00 | 0.00 |
| ATOM | 391 | CG | LEU | S | 49 | 23.058 | 120.927 | 10.250 | 1.00 | 0.00 |
| ATOM | 392 | CD1 | LEU | S | 49 | 23.214 | 119.760 | 11.179 | 1.00 | 0.00 |
| ATOM | 393 | CD2 | LEU | S | 49 | 23.186 | 122.224 | 11.006 | 1.00 | 0.00 |
| ATOM | 394 | N | GLU | S | 50 | 26.526 | 121.769 | 6.935 | 1.00 | 0.00 |
| ATOM | 395 | CA | GLU | S | 50 | 27.653 | 121.249 | 6.174 | 1.00 | 0.00 |
| ATOM | 396 | C | GLU | S | 50 | 28.112 | 119.861 | 6.627 | 1.00 | 0.00 |
| ATOM | 397 | O | GLU | S | 50 | 28.510 | 119.040 | 5.816 | 1.00 | 0.00 |
| ATOM | 398 | CB | GLU | S | 50 | 28.830 | 122.257 | 6.285 | 1.00 | 0.00 |
| ATOM | 399 | CG | GLU | S | 50 | 29.569 | 122.348 | 7.662 | 1.00 | 0.00 |
| ATOM | 400 | CD | GLU | S | 50 | 30.762 | 123.300 | 7.784 | 1.00 | 0.00 |
| ATOM | 401 | OE1 | GLU | S | 50 | 30.776 | 124.418 | 7.286 | 1.00 | 0.00 |
| ATOM | 402 | OE2 | GLU | S | 50 | 31.803 | 122.785 | 8.497 | 1.00 | 0.00 |
| ATOM | 403 | N | PRO | S | 51 | 28.122 | 119.585 | 7.912 | 1.00 | 0.00 |
| ATOM | 404 | CA | PRO | S | 51 | 28.568 | 118.309 | 8.440 | 1.00 | 0.00 |
| ATOM | 405 | C | PRO | S | 51 | 27.907 | 118.154 | 9.800 | 1.00 | 0.00 |
| ATOM | 406 | O | PRO | S | 51 | 27.068 | 118.983 | 10.147 | 1.00 | 0.00 |

| | | | | | | | | | | |
|---|---|---|---|---|---|---|---|---|---|---|
| ATOM | 407 | CB | PRO | S | 51 | 30.082 | 118.404 | 8.562 | 1.00 | 0.00 |
| ATOM | 408 | CG | PRO | S | 51 | 30.249 | 119.892 | 8.916 | 1.00 | 0.00 |
| ATOM | 409 | CD | PRO | S | 51 | 29.235 | 120.596 | 8.008 | 1.00 | 0.00 |
| ATOM | 410 | N | ALA | S | 52 | 28.317 | 117.163 | 10.597 | 1.00 | 0.00 |
| ATOM | 411 | CA | ALA | S | 52 | 27.769 | 116.957 | 11.928 | 1.00 | 0.00 |
| ATOM | 412 | C | ALA | S | 52 | 28.283 | 117.934 | 12.969 | 1.00 | 0.00 |
| ATOM | 413 | O | ALA | S | 52 | 27.808 | 117.928 | 14.093 | 1.00 | 0.00 |
| ATOM | 414 | CB | ALA | S | 52 | 28.067 | 115.503 | 12.334 | 1.00 | 0.00 |
| ATOM | 415 | N | ALA | S | 53 | 29.238 | 118.789 | 12.642 | 1.00 | 0.00 |
| ATOM | 416 | CA | ALA | S | 53 | 29.828 | 119.729 | 13.593 | 1.00 | 0.00 |
| ATOM | 417 | C | ALA | S | 53 | 28.966 | 120.901 | 14.020 | 1.00 | 0.00 |
| ATOM | 418 | O | ALA | S | 53 | 29.248 | 121.555 | 15.025 | 1.00 | 0.00 |
| ATOM | 419 | CB | ALA | S | 53 | 31.100 | 120.269 | 12.997 | 1.00 | 0.00 |
| ATOM | 420 | N | ASN | S | 54 | 27.938 | 121.125 | 13.233 | 1.00 | 0.00 |
| ATOM | 421 | CA | ASN | S | 54 | 27.139 | 122.311 | 13.353 | 1.00 | 0.00 |
| ATOM | 422 | C | ASN | S | 54 | 25.720 | 121.889 | 13.574 | 1.00 | 0.00 |
| ATOM | 423 | O | ASN | S | 54 | 25.320 | 120.796 | 13.198 | 1.00 | 0.00 |
| ATOM | 424 | CB | ASN | S | 54 | 27.304 | 123.182 | 12.073 | 1.00 | 0.00 |
| ATOM | 425 | CG | ASN | S | 54 | 28.645 | 123.902 | 11.893 | 1.00 | 0.00 |
| ATOM | 426 | OD1 | ASN | S | 54 | 29.199 | 124.489 | 12.811 | 1.00 | 0.00 |
| ATOM | 427 | ND2 | ASN | S | 54 | 29.202 | 123.908 | 10.712 | 1.00 | 0.00 |
| ATOM | 428 | N | GLU | S | 55 | 24.957 | 122.785 | 14.131 | 1.00 | 0.00 |
| ATOM | 429 | CA | GLU | S | 55 | 23.562 | 122.559 | 14.366 | 1.00 | 0.00 |
| ATOM | 430 | C | GLU | S | 55 | 23.017 | 123.972 | 14.322 | 1.00 | 0.00 |
| ATOM | 431 | O | GLU | S | 55 | 23.695 | 124.903 | 14.755 | 1.00 | 0.00 |
| ATOM | 432 | CB | GLU | S | 55 | 23.446 | 121.921 | 15.712 | 1.00 | 0.00 |
| ATOM | 433 | CG | GLU | S | 55 | 22.134 | 121.248 | 15.915 | 1.00 | 0.00 |
| ATOM | 434 | CD | GLU | S | 55 | 21.996 | 120.761 | 17.336 | 1.00 | 0.00 |
| ATOM | 435 | OE1 | GLU | S | 55 | 22.809 | 119.900 | 17.738 | 1.00 | 0.00 |
| ATOM | 436 | OE2 | GLU | S | 55 | 21.076 | 121.241 | 18.045 | 1.00 | 0.00 |
| ATOM | 437 | N | THR | S | 56 | 21.838 | 124.153 | 13.769 | 1.00 | 0.00 |
| ATOM | 438 | CA | THR | S | 56 | 21.264 | 125.474 | 13.654 | 1.00 | 0.00 |
| ATOM | 439 | C | THR | S | 56 | 20.121 | 125.411 | 14.668 | 1.00 | 0.00 |
| ATOM | 440 | O | THR | S | 56 | 19.353 | 124.458 | 14.714 | 1.00 | 0.00 |
| ATOM | 441 | CB | THR | S | 56 | 20.837 | 125.761 | 12.175 | 1.00 | 0.00 |
| ATOM | 442 | OG1 | THR | S | 56 | 19.788 | 124.886 | 11.780 | 1.00 | 0.00 |
| ATOM | 443 | CG2 | THR | S | 56 | 21.927 | 125.569 | 11.099 | 1.00 | 0.00 |
| ATOM | 444 | N | VAL | S | 57 | 20.104 | 126.347 | 15.577 | 1.00 | 0.00 |
| ATOM | 445 | CA | VAL | S | 57 | 19.133 | 126.367 | 16.643 | 1.00 | 0.00 |
| ATOM | 446 | C | VAL | S | 57 | 17.815 | 127.012 | 16.227 | 1.00 | 0.00 |
| ATOM | 447 | O | VAL | S | 57 | 17.732 | 127.733 | 15.228 | 1.00 | 0.00 |
| ATOM | 448 | CB | VAL | S | 57 | 19.729 | 127.122 | 17.897 | 1.00 | 0.00 |
| ATOM | 449 | CGI | VAL | S | 57 | 20.993 | 126.492 | 18.545 | 1.00 | 0.00 |
| ATOM | 450 | CG2 | VAL | S | 57 | 20.106 | 128.598 | 17.619 | 1.00 | 0.00 |
| ATOM | 451 | N | ARG | S | 58 | 16.811 | 126.805 | 17.069 | 1.00 | 0.00 |
| ATOM | 452 | CA | ARG | S | 58 | 15.471 | 127.333 | 16.873 | 1.00 | 0.00 |
| ATOM | 453 | C | ARG | S | 58 | 15.379 | 128.801 | 16.474 | 1.00 | 0.00 |
| ATOM | 454 | O | ARG | S | 58 | 14.522 | 129.186 | 15.698 | 1.00 | 0.00 |
| ATOM | 455 | CB | ARG | S | 58 | 14.650 | 127.120 | 18.174 | 1.00 | 0.00 |
| ATOM | 456 | CG | ARG | S | 58 | 14.494 | 125.641 | 18.615 | 1.00 | 0.00 |
| ATOM | 457 | CD | ARG | S | 58 | 13.787 | 124.784 | 17.559 | 1.00 | 0.00 |
| ATOM | 458 | NE | ARG | S | 58 | 13.880 | 123.361 | 17.972 | 1.00 | 0.00 |
| ATOM | 459 | CZ | ARG | S | 58 | 13.382 | 122.330 | 17.304 | 1.00 | 0.00 |
| ATOM | 460 | NH1 | ARG | S | 58 | 12.751 | 122.426 | 16.171 | 1.00 | 0.00 |
| ATOM | 461 | NH2 | ARG | S | 58 | 13.536 | 121.159 | 17.815 | 1.00 | 0.00 |
| ATOM | 462 | N | HIS | S | 59 | 16.260 | 129.637 | 16.972 | 1.00 | 0.00 |
| ATOM | 463 | CA | HIS | S | 59 | 16.193 | 131.077 | 16.775 | 1.00 | 0.00 |
| ATOM | 464 | C | HIS | S | 59 | 17.168 | 131.530 | 15.718 | 1.00 | 0.00 |
| ATOM | 465 | O | HIS | S | 59 | 17.465 | 132.722 | 15.606 | 1.00 | 0.00 |
| ATOM | 466 | CB | HIS | S | 59 | 16.542 | 131.716 | 18.129 | 1.00 | 0.00 |
| ATOM | 467 | CG | HIS | S | 59 | 15.498 | 131.447 | 19.173 | 1.00 | 0.00 |

| | | | | | | | | | | |
|---|---|---|---|---|---|---|---|---|---|---|
| ATOM | 468 | ND1 | HIS | S | 59 | 15.546 | 130.438 | 20.132 | 1.00 | 0.00 |
| ATOM | 469 | CD2 | HIS | S | 59 | 14.338 | 132.201 | 19.287 | 1.00 | 0.00 |
| ATOM | 470 | CE1 | HIS | S | 59 | 14.384 | 130.673 | 20.771 | 1.00 | 0.00 |
| ATOM | 471 | NE2 | HIS | S | 59 | 13.606 | 131.698 | 20.330 | 1.00 | 0.00 |
| ATOM | 472 | N | GLY | S | 60 | 17.710 | 130.556 | 15.011 | 1.00 | 0.00 |
| ATOM | 473 | CA | GLY | S | 60 | 18.690 | 130.814 | 13.987 | 1.00 | 0.00 |
| ATOM | 474 | C | GLY | S | 60 | 20.126 | 130.874 | 14.458 | 1.00 | 0.00 |
| ATOM | 475 | O | GLY | S | 60 | 20.991 | 131.171 | 13.630 | 1.00 | 0.00 |
| ATOM | 476 | N | CYS | S | 61 | 20.463 | 130.621 | 15.715 | 1.00 | 0.00 |
| ATOM | 477 | CA | CYS | S | 61 | 21.841 | 130.709 | 16.186 | 1.00 | 0.00 |
| ATOM | 478 | C | CYS | S | 61 | 22.709 | 129.572 | 15.685 | 1.00 | 0.00 |
| ATOM | 479 | O | CYS | S | 61 | 22.215 | 128.442 | 15.408 | 1.00 | 0.00 |
| ATOM | 480 | CB | CYS | S | 61 | 21.818 | 130.701 | 17.718 | 1.00 | 0.00 |
| ATOM | 481 | SG | CYS | S | 61 | 20.269 | 131.378 | 18.384 | 1.00 | 0.00 |
| ATOM | 482 | N | LEU | S | 62 | 24.013 | 129.867 | 15.530 | 1.00 | 0.00 |
| ATOM | 483 | CA | LEU | S | 62 | 24.923 | 128.870 | 14.977 | 1.00 | 0.00 |
| ATOM | 484 | C | LEU | S | 62 | 25.707 | 128.127 | 16.036 | 1.00 | 0.00 |
| ATOM | 485 | O | LEU | S | 62 | 26.381 | 128.755 | 16.891 | 1.00 | 0.00 |
| ATOM | 486 | CB | LEU | S | 62 | 25.893 | 129.589 | 14.039 | 1.00 | 0.00 |
| ATOM | 487 | CG | LEU | S | 62 | 26.804 | 128.630 | 13.288 | 1.00 | 0.00 |
| ATOM | 488 | CD1 | LEU | S | 62 | 26.039 | 127.742 | 12.305 | 1.00 | 0.00 |
| ATOM | 489 | CD2 | LEU | S | 62 | 27.866 | 129.366 | 12.471 | 1.00 | 0.00 |
| ATOM | 490 | N | ARG | S | 63 | 25.721 | 126.828 | 16.123 | 1.00 | 0.00 |
| ATOM | 491 | CA | ARG | S | 63 | 26.508 | 126.175 | 17.209 | 1.00 | 0.00 |
| ATOM | 492 | C | ARG | S | 63 | 27.689 | 125.418 | 16.610 | 1.00 | 0.00 |
| ATOM | 493 | O | ARG | S | 63 | 27.584 | 124.833 | 15.556 | 1.00 | 0.00 |
| ATOM | 494 | CB | ARG | S | 63 | 25.569 | 125.241 | 18.020 | 1.00 | 0.00 |
| ATOM | 495 | CG | ARG | S | 63 | 26.160 | 124.717 | 19.355 | 1.00 | 0.00 |
| ATOM | 496 | CD | ARG | S | 63 | 25.201 | 123.777 | 20.095 | 1.00 | 0.00 |
| ATOM | 497 | NE | ARG | S | 63 | 25.845 | 123.357 | 21.365 | 1.00 | 0.00 |
| ATOM | 498 | CZ | ARG | S | 63 | 25.311 | 122.546 | 22.268 | 1.00 | 0.00 |
| ATOM | 499 | NH1 | ARG | S | 63 | 24.136 | 121.999 | 22.159 | 1.00 | 0.00 |
| ATOM | 500 | NH2 | ARG | S | 63 | 26.005 | 122.289 | 23.320 | 1.00 | 0.00 |
| ATOM | 501 | N | LEU | S | 64 | 28.815 | 125.420 | 17.269 | 1.00 | 0.00 |
| ATOM | 502 | CA | LEU | S | 64 | 29.982 | 124.680 | 16.701 | 1.00 | 0.00 |
| ATOM | 503 | C | LEU | S | 64 | 30.433 | 123.573 | 17.651 | 1.00 | 0.00 |
| ATOM | 504 | O | LEU | S | 64 | 30.865 | 123.817 | 18.759 | 1.00 | 0.00 |
| ATOM | 505 | CB | LEU | S | 64 | 31.149 | 125.660 | 16.388 | 1.00 | 0.00 |
| ATOM | 506 | CG | LEU | S | 64 | 30.904 | 126.777 | 15.339 | 1.00 | 0.00 |
| ATOM | 507 | CD1 | LEU | S | 64 | 32.068 | 127.778 | 15.352 | 1.00 | 0.00 |
| ATOM | 508 | CD2 | LEU | S | 64 | 30.711 | 126.226 | 13.916 | 1.00 | 0.00 |
| ATOM | 509 | N | ASN | S | 65 | 30.335 | 122.352 | 17.208 | 1.00 | 0.00 |
| ATOM | 510 | CA | ASN | S | 65 | 30.756 | 121.203 | 18.054 | 1.00 | 0.00 |
| ATOM | 511 | C | ASN | S | 65 | 31.594 | 120.243 | 17.214 | 1.00 | 0.00 |
| ATOM | 512 | O | ASN | S | 65 | 31.485 | 120.215 | 16.005 | 1.00 | 0.00 |
| ATOM | 513 | CB | ASN | S | 65 | 29.497 | 120.502 | 18.643 | 1.00 | 0.00 |
| ATOM | 514 | CG | ASN | S | 65 | 28.698 | 121.281 | 19.694 | 1.00 | 0.00 |
| ATOM | 515 | OD1 | ASN | S | 65 | 27.484 | 121.408 | 19.628 | 1.00 | 0.00 |
| ATOM | 516 | ND2 | ASN | S | 65 | 29.339 | 121.847 | 20.681 | 1.00 | 0.00 |
| ATOM | 517 | N | GLN | S | 66 | 32.431 | 119.459 | 17.836 | 1.00 | 0.00 |
| ATOM | 518 | CA | GLN | S | 66 | 33.277 | 118.504 | 17.064 | 1.00 | 0.00 |
| ATOM | 519 | C | GLN | S | 66 | 33.958 | 119.220 | 15.894 | 1.00 | 0.00 |
| ATOM | 520 | O | GLN | S | 66 | 34.271 | 118.629 | 14.880 | 1.00 | 0.00 |
| ATOM | 521 | CB | GLN | S | 66 | 32.388 | 117.343 | 16.569 | 1.00 | 0.00 |
| ATOM | 522 | CG | GLN | S | 66 | 31.824 | 116.384 | 17.671 | 1.00 | 0.00 |
| ATOM | 523 | CD | GLN | S | 66 | 32.803 | 115.543 | 18.500 | 1.00 | 0.00 |
| ATOM | 524 | OE1 | GLN | S | 66 | 33.757 | 114.990 | 17.976 | 1.00 | 0.00 |
| ATOM | 525 | NE2 | GLN | S | 66 | 32.607 | 115.388 | 19.785 | 1.00 | 0.00 |
| ATOM | 526 | N | PRO | S | 67 | 34.186 | 120.493 | 16.036 | 1.00 | 0.00 |
| ATOM | 527 | CA | PRO | S | 67 | 34.845 | 121.264 | 14.942 | 1.00 | 0.00 |
| ATOM | 528 | C | PRO | S | 67 | 36.275 | 120.760 | 14.725 | 1.00 | 0.00 |

| | | | | | | | | | | |
|---|---|---|---|---|---|---|---|---|---|---|
| ATOM | 529 | O | PRO | S | 67 | 37.129 | 120.891 | 15.579 | 1.00 | 0.00 |
| ATOM | 530 | CB | PRO | S | 67 | 34.873 | 122.715 | 15.401 | 1.00 | 0.00 |
| ATOM | 531 | CG | PRO | S | 67 | 35.050 | 122.534 | 16.918 | 1.00 | 0.00 |
| ATOM | 532 | CD | PRO | S | 67 | 34.127 | 121.358 | 17.252 | 1.00 | 0.00 |
| ATOM | 533 | N | THR | S | 68 | 36.537 | 120.186 | 13.583 | 1.00 | 0.00 |
| ATOM | 534 | CA | THR | S | 68 | 37.907 | 119.672 | 13.295 | 1.00 | 0.00 |
| ATOM | 535 | C | THR | S | 68 | 38.803 | 120.815 | 12.809 | 1.00 | 0.00 |
| ATOM | 536 | O | THR | S | 68 | 38.495 | 121.976 | 12.993 | 1.00 | 0.00 |
| ATOM | 537 | CB | THR | S | 68 | 37.849 | 118.510 | 12.247 | 1.00 | 0.00 |
| ATOM | 538 | OG1 | THR | S | 68 | 37.384 | 118.991 | 10.993 | 1.00 | 0.00 |
| ATOM | 539 | CG2 | THR | S | 68 | 36.909 | 117.332 | 12.583 | 1.00 | 0.00 |
| ATOM | 540 | N | HIS | S | 69 | 39.911 | 120.501 | 12.193 | 1.00 | 0.00 |
| ATOM | 541 | CA | HIS | S | 69 | 40.821 | 121.575 | 11.700 | 1.00 | 0.00 |
| ATOM | 542 | C | HIS | S | 69 | 40.398 | 122.034 | 10.300 | 1.00 | 0.00 |
| ATOM | 543 | O | HIS | S | 69 | 41.207 | 122.132 | 9.399 | 1.00 | 0.00 |
| ATOM | 544 | CB | HIS | S | 69 | 42.232 | 120.965 | 11.667 | 1.00 | 0.00 |
| ATOM | 545 | CG | HIS | S | 69 | 42.703 | 120.545 | 13.028 | 1.00 | 0.00 |
| ATOM | 546 | ND1 | HIS | S | 69 | 43.302 | 121.374 | 13.975 | 1.00 | 0.00 |
| ATOM | 547 | CD2 | HIS | S | 69 | 42.588 | 119.243 | 13.494 | 1.00 | 0.00 |
| ATOM | 548 | CE1 | HIS | S | 69 | 43.500 | 120.484 | 14.966 | 1.00 | 0.00 |
| ATOM | 549 | NE2 | HIS | S | 69 | 43.108 | 119.197 | 14.760 | 1.00 | 0.00 |
| ATOM | 550 | N | VAL | S | 70 | 39.139 | 122.318 | 10.111 | 1.00 | 0.00 |
| ATOM | 551 | CA | VAL | S | 70 | 38.668 | 122.773 | 8.772 | 1.00 | 0.00 |
| ATOM | 552 | C | VAL | S | 70 | 37.381 | 123.571 | 8.920 | 1.00 | 0.00 |
| ATOM | 553 | O | VAL | S | 70 | 36.420 | 123.376 | 8.202 | 1.00 | 0.00 |
| ATOM | 554 | CB | VAL | S | 70 | 38.468 | 121.526 | 7.822 | 1.00 | 0.00 |
| ATOM | 555 | CG1 | VAL | S | 70 | 37.249 | 120.613 | 8.131 | 1.00 | 0.00 |
| ATOM | 556 | CG2 | VAL | S | 70 | 38.333 | 121.894 | 6.325 | 1.00 | 0.00 |
| ATOM | 557 | N | ASN | S | 71 | 37.361 | 124.459 | 9.860 | 1.00 | 0.00 |
| ATOM | 558 | CA | ASN | S | 71 | 36.151 | 125.285 | 10.098 | 1.00 | 0.00 |
| ATOM | 559 | C | ASN | S | 71 | 36.556 | 126.725 | 10.427 | 1.00 | 0.00 |
| ATOM | 560 | O | ASN | S | 71 | 35.899 | 127.661 | 10.024 | 1.00 | 0.00 |
| ATOM | 561 | CB | ASN | S | 71 | 35.312 | 124.659 | 11.250 | 1.00 | 0.00 |
| ATOM | 562 | CG | ASN | S | 71 | 34.860 | 123.205 | 11.067 | 1.00 | 0.00 |
| ATOM | 563 | OD1 | ASN | S | 71 | 35.437 | 122.271 | 11.605 | 1.00 | 0.00 |
| ATOM | 564 | ND2 | ASN | S | 71 | 33.838 | 122.956 | 10.293 | 1.00 | 0.00 |
| ATOM | 565 | N | ASN | S | 72 | 37.630 | 126.888 | 11.162 | 1.00 | 0.00 |
| ATOM | 566 | CA | ASN | S | 72 | 38.123 | 128.248 | 11.557 | 1.00 | 0.00 |
| ATOM | 567 | C | ASN | S | 72 | 36.982 | 129.099 | 12.106 | 1.00 | 0.00 |
| ATOM | 568 | O | ASN | S | 72 | 35.836 | 128.696 | 12.127 | 1.00 | 0.00 |
| ATOM | 569 | CB | ASN | S | 72 | 38.786 | 128.938 | 10.329 | 1.00 | 0.00 |
| ATOM | 570 | CG | ASN | S | 72 | 40.146 | 128.392 | 9.878 | 1.00 | 0.00 |
| ATOM | 571 | OD1 | ASN | S | 72 | 41.034 | 128.119 | 10.673 | 1.00 | 0.00 |
| ATOM | 572 | ND2 | ASN | S | 72 | 40.368 | 128.233 | 8.601 | 1.00 | 0.00 |
| ATOM | 573 | N | GLY | S | 73 | 37.299 | 130.248 | 12.613 | 1.00 | 0.00 |
| ATOM | 574 | CA | GLY | S | 73 | 36.274 | 131.093 | 13.211 | 1.00 | 0.00 |
| ATOM | 575 | C | GLY | S | 73 | 35.613 | 132.016 | 12.232 | 1.00 | 0.00 |
| ATOM | 576 | O | GLY | S | 73 | 34.541 | 131.721 | 11.771 | 1.00 | 0.00 |
| ATOM | 577 | N | ASN | S | 74 | 36.203 | 133.166 | 12.011 | 1.00 | 0.00 |
| ATOM | 578 | CA | ASN | S | 74 | 35.600 | 134.226 | 11.123 | 1.00 | 0.00 |
| ATOM | 579 | C | ASN | S | 74 | 34.320 | 133.736 | 10.423 | 1.00 | 0.00 |
| ATOM | 580 | O | ASN | S | 74 | 34.345 | 133.328 | 9.285 | 1.00 | 0.00 |
| ATOM | 581 | CB | ASN | S | 74 | 36.675 | 134.574 | 10.090 | 1.00 | 0.00 |
| ATOM | 582 | CG | ASN | S | 74 | 37.814 | 135.372 | 10.736 | 1.00 | 0.00 |
| ATOM | 583 | OD1 | ASN | S | 74 | 37.577 | 136.278 | 11.507 | 1.00 | 0.00 |
| ATOM | 584 | ND2 | ASN | S | 74 | 39.051 | 135.085 | 10.430 | 1.00 | 0.00 |
| ATOM | 585 | N | TYR | S | 75 | 33.214 | 133.745 | 11.128 | 1.00 | 0.00 |
| ATOM | 586 | CA | TYR | S | 75 | 31.932 | 133.248 | 10.537 | 1.00 | 0.00 |
| ATOM | 587 | C | TYR | S | 75 | 30.924 | 134.381 | 10.416 | 1.00 | 0.00 |
| ATOM | 588 | O | TYR | S | 75 | 30.723 | 135.125 | 11.352 | 1.00 | 0.00 |
| ATOM | 589 | CB | TYR | S | 75 | 31.389 | 132.210 | 11.525 | 1.00 | 0.00 |

| | | | | | | | | | | |
|---|---|---|---|---|---|---|---|---|---|---|
| ATOM | 590 | CG | TYR | S | 75 | 31.029 | 132.854 | 12.841 | 1.00 | 0.00 |
| ATOM | 591 | CD1 | TYR | S | 75 | 31.928 | 132.852 | 13.914 | 1.00 | 0.00 |
| ATOM | 592 | CD2 | TYR | S | 75 | 29.768 | 133.434 | 12.988 | 1.00 | 0.00 |
| ATOM | 593 | CE1 | TYR | S | 75 | 31.555 | 133.433 | 15.133 | 1.00 | 0.00 |
| ATOM | 594 | CE2 | TYR | S | 75 | 29.395 | 134.018 | 14.201 | 1.00 | 0.00 |
| ATOM | 595 | CZ | TYR | S | 75 | 30.288 | 134.017 | 15.277 | 1.00 | 0.00 |
| ATOM | 596 | OH | TYR | S | 75 | 29.922 | 134.591 | 16.479 | 1.00 | 0.00 |
| ATOM | 597 | N | THR | S | 76 | 30.267 | 134.523 | 9.294 | 1.00 | 0.00 |
| ATOM | 598 | CA | THR | S | 76 | 29.263 | 135.622 | 9.197 | 1.00 | 0.00 |
| ATOM | 599 | C | THR | S | 76 | 27.880 | 135.051 | 8.903 | 1.00 | 0.00 |
| ATOM | 600 | O | THR | S | 76 | 27.708 | 133.862 | 8.764 | 1.00 | 0.00 |
| ATOM | 601 | CB | THR | S | 76 | 29.688 | 136.660 | 8.104 | 1.00 | 0.00 |
| ATOM | 602 | OG1 | THR | S | 76 | 29.678 | 136.060 | 6.816 | 1.00 | 0.00 |
| ATOM | 603 | CG2 | THR | S | 76 | 31.106 | 137.257 | 8.241 | 1.00 | 0.00 |
| ATOM | 604 | N | LEU | S | 77 | 26.895 | 135.893 | 8.806 | 1.00 | 0.00 |
| ATOM | 605 | CA | LEU | S | 77 | 25.515 | 135.387 | 8.507 | 1.00 | 0.00 |
| ATOM | 606 | C | LEU | S | 77 | 24.785 | 136.340 | 7.564 | 1.00 | 0.00 |
| ATOM | 607 | O | LEU | S | 77 | 24.383 | 137.418 | 7.948 | 1.00 | 0.00 |
| ATOM | 608 | CB | LEU | S | 77 | 24.714 | 135.178 | 9.824 | 1.00 | 0.00 |
| ATOM | 609 | CG | LEU | S | 77 | 23.375 | 134.397 | 9.749 | 1.00 | 0.00 |
| ATOM | 610 | CD1 | LEU | S | 77 | 22.522 | 134.691 | 10.990 | 1.00 | 0.00 |
| ATOM | 611 | CD2 | LEU | S | 77 | 22.569 | 134.718 | 8.478 | 1.00 | 0.00 |
| ATOM | 612 | N | LEU | S | 78 | 24.574 | 135.940 | 6.346 | 1.00 | 0.00 |
| ATOM | 613 | CA | LEU | S | 78 | 23.836 | 136.828 | 5.407 | 1.00 | 0.00 |
| ATOM | 614 | C | LEU | S | 78 | 22.340 | 136.546 | 5.563 | 1.00 | 0.00 |
| ATOM | 615 | O | LEU | S | 78 | 21.873 | 135.471 | 5.253 | 1.00 | 0.00 |
| ATOM | 616 | CB | LEU | S | 78 | 24.334 | 136.614 | 3.949 | 1.00 | 0.00 |
| ATOM | 617 | CG | LEU | S | 78 | 23.747 | 137.522 | 2.835 | 1.00 | 0.00 |
| ATOM | 618 | CD1 | LEU | S | 78 | 24.182 | 138.977 | 3.057 | 1.00 | 0.00 |
| ATOM | 619 | CD2 | LEU | S | 78 | 24.153 | 137.068 | 1.423 | 1.00 | 0.00 |
| ATOM | 620 | N | ALA | S | 79 | 21.586 | 137.484 | 6.062 | 1.00 | 0.00 |
| ATOM | 621 | CA | ALA | S | 79 | 20.136 | 137.228 | 6.264 | 1.00 | 0.00 |
| ATOM | 622 | C | ALA | S | 79 | 19.299 | 138.114 | 5.342 | 1.00 | 0.00 |
| ATOM | 623 | O | ALA | S | 79 | 19.414 | 139.320 | 5.360 | 1.00 | 0.00 |
| ATOM | 624 | CB | ALA | S | 79 | 19.827 | 137.450 | 7.755 | 1.00 | 0.00 |
| ATOM | 625 | N | ALA | S | 80 | 18.462 | 137.525 | 4.534 | 1.00 | 0.00 |
| ATOM | 626 | CA | ALA | S | 80 | 17.632 | 138.344 | 3.606 | 1.00 | 0.00 |
| ATOM | 627 | C | ALA | S | 80 | 16.162 | 138.328 | 4.028 | 1.00 | 0.00 |
| ATOM | 628 | O | ALA | S | 80 | 15.835 | 138.089 | 5.173 | 1.00 | 0.00 |
| ATOM | 629 | CB | ALA | S | 80 | 17.851 | 137.801 | 2.183 | 1.00 | 0.00 |
| ATOM | 630 | N | ASN | S | 81 | 15.273 | 138.588 | 3.109 | 1.00 | 0.00 |
| ATOM | 631 | CA | ASN | S | 81 | 13.826 | 138.599 | 3.447 | 1.00 | 0.00 |
| ATOM | 632 | C | ASN | S | 81 | 12.992 | 138.709 | 2.165 | 1.00 | 0.00 |
| ATOM | 633 | O | ASN | S | 81 | 13.512 | 138.953 | 1.094 | 1.00 | 0.00 |
| ATOM | 634 | CB | ASN | S | 81 | 13.647 | 139.840 | 4.330 | 1.00 | 0.00 |
| ATOM | 635 | CG | ASN | S | 81 | 12.241 | 139.858 | 4.940 | 1.00 | 0.00 |
| ATOM | 636 | OD1 | ASN | S | 81 | 11.527 | 138.878 | 4.881 | 1.00 | 0.00 |
| ATOM | 637 | ND2 | ASN | S | 81 | 11.813 | 140.943 | 5.527 | 1.00 | 0.00 |
| ATOM | 638 | N | PRO | S | 82 | 11.700 | 138.537 | 2.267 | 1.00 | 0.00 |
| ATOM | 639 | CA | PRO | S | 82 | 10.829 | 138.635 | 1.056 | 1.00 | 0.00 |
| ATOM | 640 | C | PRO | S | 82 | 11.132 | 139.908 | 0.270 | 1.00 | 0.00 |
| ATOM | 641 | O | PRO | S | 82 | 10.885 | 139.996 | -0.915 | 1.00 | 0.00 |
| ATOM | 642 | CB | PRO | S | 82 | 9.395 | 138.679 | 1.565 | 1.00 | 0.00 |
| ATOM | 643 | CG | PRO | S | 82 | 9.582 | 139.447 | 2.885 | 1.00 | 0.00 |
| ATOM | 644 | CD | PRO | S | 82 | 10.872 | 138.858 | 3.466 | 1.00 | 0.00 |
| ATOM | 645 | N | PHE | S | 83 | 11.666 | 140.888 | 0.925 | 1.00 | 0.00 |
| ATOM | 646 | CA | PHE | S | 83 | 11.997 | 142.161 | 0.236 | 1.00 | 0.00 |
| ATOM | 647 | C | PHE | S | 83 | 13.088 | 142.907 | 1.009 | 1.00 | 0.00 |
| ATOM | 648 | O | PHE | S | 83 | 12.984 | 144.091 | 1.260 | 1.00 | 0.00 |
| ATOM | 649 | CB | PHE | S | 83 | 10.727 | 143.061 | 0.136 | 1.00 | 0.00 |
| ATOM | 650 | CG | PHE | S | 83 | 9.587 | 142.538 | -0.750 | 1.00 | 0.00 |

| | | | | | | | | | | |
|---|---|---|---|---|---|---|---|---|---|---|
| ATOM | 651 | CD1 | PHE | S | 83 | 8.465 | 141.947 | -0.158 | 1.00 | 0.00 |
| ATOM | 652 | CD2 | PHE | S | 83 | 9.683 | 142.588 | -2.144 | 1.00 | 0.00 |
| ATOM | 653 | CE1 | PHE | S | 83 | 7.462 | 141.397 | -0.948 | 1.00 | 0.00 |
| ATOM | 654 | CE2 | PHE | S | 83 | 8.672 | 142.047 | -2.934 | 1.00 | 0.00 |
| ATOM | 655 | CZ | PHE | S | 83 | 7.564 | 141.449 | -2.337 | 1.00 | 0.00 |
| ATOM | 656 | N | GLY | S | 84 | 14.133 | 142.222 | 1.390 | 1.00 | 0.00 |
| ATOM | 657 | CA | GLY | S | 84 | 15.226 | 142.895 | 2.147 | 1.00 | 0.00 |
| ATOM | 658 | C | GLY | S | 84 | 16.530 | 142.112 | 1.980 | 1.00 | 0.00 |
| ATOM | 659 | O | GLY | S | 84 | 16.578 | 141.094 | 1.320 | 1.00 | 0.00 |
| ATOM | 660 | N | GLN | S | 85 | 17.591 | 142.583 | 2.579 | 1.00 | 0.00 |
| ATOM | 661 | CA | GLN | S | 85 | 18.899 | 141.876 | 2.463 | 1.00 | 0.00 |
| ATOM | 662 | C | GLN | S | 85 | 19.900 | 142.466 | 3.460 | 1.00 | 0.00 |
| ATOM | 663 | O | GLN | S | 85 | 20.044 | 143.668 | 3.562 | 1.00 | 0.00 |
| ATOM | 664 | CB | GLN | S | 85 | 19.404 | 142.004 | 1.009 | 1.00 | 0.00 |
| ATOM | 665 | CG | GLN | S | 85 | 20.641 | 141.124 | 0.627 | 1.00 | 0.00 |
| ATOM | 666 | CD | GLN | S | 85 | 20.483 | 139.601 | 0.566 | 1.00 | 0.00 |
| ATOM | 667 | OE1 | GLN | S | 85 | 21.199 | 138.866 | 1.227 | 1.00 | 0.00 |
| ATOM | 668 | NE2 | GLN | S | 85 | 19.587 | 139.067 | -0.225 | 1.00 | 0.00 |
| ATOM | 669 | N | ALA | S | 86 | 20.592 | 141.639 | 4.200 | 1.00 | 0.00 |
| ATOM | 670 | CA | ALA | S | 86 | 21.573 | 142.168 | 5.183 | 1.00 | 0.00 |
| ATOM | 671 | C | ALA | S | 86 | 22.594 | 141.092 | 5.513 | 1.00 | 0.00 |
| ATOM | 672 | O | ALA | S | 86 | 22.628 | 140.043 | 4.898 | 1.00 | 0.00 |
| ATOM | 673 | CB | ALA | S | 86 | 20.784 | 142.663 | 6.407 | 1.00 | 0.00 |
| ATOM | 674 | N | SER | S | 87 | 23.422 | 141.334 | 6.481 | 1.00 | 0.00 |
| ATOM | 675 | CA | SER | S | 87 | 24.434 | 140.314 | 6.849 | 1.00 | 0.00 |
| ATOM | 676 | C | SER | S | 87 | 25.161 | 140.709 | 8.132 | 1.00 | 0.00 |
| ATOM | 677 | O | SER | S | 87 | 25.118 | 141.842 | 8.570 | 1.00 | 0.00 |
| ATOM | 678 | CB | SER | S | 87 | 25.423 | 140.073 | 5.681 | 1.00 | 0.00 |
| ATOM | 679 | OG | SER | S | 87 | 26.219 | 141.224 | 5.379 | 1.00 | 0.00 |
| ATOM | 680 | N | ALA | S | 88 | 25.833 | 139.771 | 8.723 | 1.00 | 0.00 |
| ATOM | 681 | CA | ALA | S | 88 | 26.589 | 140.050 | 9.977 | 1.00 | 0.00 |
| ATOM | 682 | C | ALA | S | 88 | 27.936 | 139.346 | 9.899 | 1.00 | 0.00 |
| ATOM | 683 | O | ALA | S | 88 | 28.253 | 138.687 | 8.930 | 1.00 | 0.00 |
| ATOM | 684 | CB | ALA | S | 88 | 25.790 | 139.514 | 11.147 | 1.00 | 0.00 |
| ATOM | 685 | OXT | ALA | S | 88 | 28.690 | 139.488 | 10.891 | 1.00 | 0.00 |
| TER | | | | | | | | | | |

European Patent Office

# EUROPEAN SEARCH REPORT

Application Number
EP 05 02 3460

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | URFER R ET AL: "AN IMMUNOGLOBULIN-LIKE DOMAIN DETERMINES THE SPECIFICITY OF NEUROTROPHIN RECEPTORS" EMBO JOURNAL, vol. 14, no. 12, 15 June 1995 (1995-06-15), pages 2795-2805, XP000568880 ISSN: 0261-4189 * the whole document * ----- | 1-10 | C12N15/12 C12N5/10 C07K14/71 C07K14/48 C07K16/28 G01N33/50 G01N33/566 C12Q1/68 A61K38/17 A01K67/027 G06F17/30 G06F17/50 G06F19/00 |
| A | HOLDEN P.H. ET AL.: "Immunoglobilin-like domains define the Nerve Growth Factor binding site of the TrkA receptor" NATURE BIOTECHNOLOGY, vol. 15, July 1997 (1997-07), pages 668-672, XP002116514 * the whole document * ----- | 1-10 | |
| A | WO 95/25795 A (GENENTECH, INC) 28 September 1995 (1995-09-28) * the whole document * ----- | 1-10 | |
| | | | TECHNICAL FIELDS SEARCHED (IPC) |
| | | | C12N C07K G01N C12Q A61K A01K G06F |

The present search report has been drawn up for all claims

2

| Place of search | Date of completion of the search | Examiner |
|---|---|---|
| Munich | 22 March 2006 | Petri, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 05 02 3460

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2006

| Patent document cited in search report | Publication date | Patent family member(s) | Publication date |
|---|---|---|---|
| WO 9525795 A | 28-09-1995 | AU 707613 B2 | 15-07-1999 |
| | | AU 3245695 A | 09-10-1995 |
| | | CA 2184059 A1 | 28-09-1995 |
| | | EP 0750666 A1 | 02-01-1997 |
| | | JP 9510612 T | 28-10-1997 |
| | | NZ 300064 A | 26-08-1998 |
| | | NZ 330897 A | 27-03-2000 |
| | | US 6153189 A | 28-11-2000 |

EPO FORM P0459

For more details about this annex : see Official Journal of the European Patent Office, No. 12/82

Annex to the application documents - subsequently filed sequences listing

SEQUENCE LISTING

<110> The University of Bristol

<120> Therapeutic Agent for NGF

<130> P100125EP-PCTDIV1

<140> 05023460.8
<141> 1999-04-09

<160> 16

<170> PatentIn version 3.2

<210> 1
<211> 25
<212> DNA
<213> Artificial

<220>
<223> Primer sequence

<400> 1
atcatatgcc ggccagtgtg cagct 25

<210> 2
<211> 38
<212> DNA
<213> Artificial

<220>
<223> Primer sequence

<400> 2
ccactggcga gaaggagaca gggatggggt cctcgggg 38

<210> 3
<211> 38
<212> DNA
<213> Artificial

<220>
<223> Primer sequence

<400> 3
gtctccttct cgccagtgga cactaacagc acatctgg 38

<210> 4
<211> 19
<212> DNA
<213> Artificial

<220>
<223> Primer sequence

<400> 4
gctagttatt gctcagcgg 19

<210> 5
<211> 30
<212> DNA
<213> Artificial

<220>

```
<223>  Primer sequence

<400>  5
cgctcgagtt atcagaagga gacgttgacc                                        30


<210>  6
<211>  25
<212>  DNA
<213>  Artificial

<220>
<223>  Primer sequence

<400>  6
atcatatgcc ggccagtgtg cagct                                             25


<210>  7
<211>  26
<212>  DNA
<213>  Artificial

<220>
<223>  Primer sequence

<400>  7
ccgatctcga gggtgtgccc acgctg                                            26


<210>  8
<211>  41
<212>  DNA
<213>  Artificial

<220>
<223>  Primer sequence

<400>  8
ccgatctcga gttatcattc gtccttcttc tccaccgggt c                           41


<210>  9
<211>  734
<212>  DNA
<213>  Artificial

<220>
<223>  Nucleotide insert


<220>
<221>  CDS
<222>  (1)..(717)

<400>  9
atg ggc agc agc cat cat cat cat cat cac agc agc ggc ctg gtg ccg       48
Met Gly Ser Ser His His His His His His Ser Ser Gly Leu Val Pro
1               5                   10                  15

cgc ggc agc cat atg ctc gag ggt gtg ccc acg ctg aag gtc cag gtg       96
Arg Gly Ser His Met Leu Glu Gly Val Pro Thr Leu Lys Val Gln Val
            20                  25                  30

ccc aat gcc tcg gtg gat gtg ggg gac gac gtg ctg ctg cgg tgc cag      144
Pro Asn Ala Ser Val Asp Val Gly Asp Asp Val Leu Leu Arg Cys Gln
        35                  40                  45

gtg gag ggg cgg ggc ctg gag cag gcc ggc tgg atc ctc aca gag ctg      192
```

```
Val Glu Gly Arg Gly Leu Glu Gln Ala Gly Trp Ile Leu Thr Glu Leu
    50                  55                  60

gag cag tca gcc acg gtg atg aaa tct ggg ggt ctg cca tcc ctg ggg      240
Glu Gln Ser Ala Thr Val Met Lys Ser Gly Gly Leu Pro Ser Leu Gly
65                  70                  75                  80

ctg acc ctg gcc aat gtc acc agt gac ctc aac agg aag aac ttg acg      288
Leu Thr Leu Ala Asn Val Thr Ser Asp Leu Asn Arg Lys Asn Leu Thr
                85                  90                  95

tgc tgg gca gag aac gat gtg ggc cgg gca gag gtc tct gtt cag gtc      336
Cys Trp Ala Glu Asn Asp Val Gly Arg Ala Glu Val Ser Val Gln Val
            100                 105                 110

aac gtc tcc ttc ccg gcc agt gtg cag ctg cac acg gcg gtg gag atg      384
Asn Val Ser Phe Pro Ala Ser Val Gln Leu His Thr Ala Val Glu Met
        115                 120                 125

cac cac tgg tgc atc ccc ttc tct gtg gat ggg cag ccg gca ccg tct      432
His His Trp Cys Ile Pro Phe Ser Val Asp Gly Gln Pro Ala Pro Ser
    130                 135                 140

ctg cgc tgg ctc ttc aat ggc tcc gtg ctc aat gag acc agc ttc atc      480
Leu Arg Trp Leu Phe Asn Gly Ser Val Leu Asn Glu Thr Ser Phe Ile
145                 150                 155                 160

ttc act gag ttc ctg gag ccg gca gcc aat gag acc gtg cgg cac ggg      528
Phe Thr Glu Phe Leu Glu Pro Ala Ala Asn Glu Thr Val Arg His Gly
                165                 170                 175

tgt ctg cgc ctc aac cag ccc acc cac gtc aac aac ggc aac tac acg      576
Cys Leu Arg Leu Asn Gln Pro Thr His Val Asn Asn Gly Asn Tyr Thr
            180                 185                 190

ctg ctg gct gcc aac ccc ttc ggc cag gcc tcc gcc tcc atc atg gct      624
Leu Leu Ala Ala Asn Pro Phe Gly Gln Ala Ser Ala Ser Ile Met Ala
        195                 200                 205

gcc ttc atg gac aac cct ttc gag ttc aac ccc gag gac ccc atc cct      672
Ala Phe Met Asp Asn Pro Phe Glu Phe Asn Pro Glu Asp Pro Ile Pro
    210                 215                 220

gac act aac agc aca tct gga gac ccg gtg gag aag aag gac gaa          717
Asp Thr Asn Ser Thr Ser Gly Asp Pro Val Glu Lys Lys Asp Glu
225                 230                 235

tgataactcg agatcgg                                                   734


<210>  10
<211>  239
<212>  PRT
<213>  Artificial

<220>
<223>  Synthetic Construct

<400>  10

Met Gly Ser Ser His His His His His His Ser Ser Gly Leu Val Pro
1               5                   10                  15

Arg Gly Ser His Met Leu Glu Gly Val Pro Thr Leu Lys Val Gln Val
            20                  25                  30

Pro Asn Ala Ser Val Asp Val Gly Asp Asp Val Leu Leu Arg Cys Gln
```

```
        35                  40                  45

Val Glu Gly Arg Gly Leu Glu Gln Ala Gly Trp Ile Leu Thr Glu Leu
    50                  55                  60

Glu Gln Ser Ala Thr Val Met Lys Ser Gly Gly Leu Pro Ser Leu Gly
65                  70                  75                  80

Leu Thr Leu Ala Asn Val Thr Ser Asp Leu Asn Arg Lys Asn Leu Thr
                85                  90                  95

Cys Trp Ala Glu Asn Asp Val Gly Arg Ala Glu Val Ser Val Gln Val
            100                 105                 110

Asn Val Ser Phe Pro Ala Ser Val Gln Leu His Thr Ala Val Glu Met
        115                 120                 125

His His Trp Cys Ile Pro Phe Ser Val Asp Gly Gln Pro Ala Pro Ser
    130                 135                 140

Leu Arg Trp Leu Phe Asn Gly Ser Val Leu Asn Glu Thr Ser Phe Ile
145                 150                 155                 160

Phe Thr Glu Phe Leu Glu Pro Ala Ala Asn Glu Thr Val Arg His Gly
                165                 170                 175

Cys Leu Arg Leu Asn Gln Pro Thr His Val Asn Asn Gly Asn Tyr Thr
            180                 185                 190

Leu Leu Ala Ala Asn Pro Phe Gly Gln Ala Ser Ala Ser Ile Met Ala
        195                 200                 205

Ala Phe Met Asp Asn Pro Phe Glu Phe Asn Pro Glu Asp Pro Ile Pro
    210                 215                 220

Asp Thr Asn Ser Thr Ser Gly Asp Pro Val Glu Lys Lys Asp Glu
225                 230                 235


<210>  11
<211>  362
<212>  DNA
<213>  Artificial

<220>
<223>  Nucleotide insert


<220>
<221>  CDS
<222>  (1)..(348)

<400>  11
atg ggc agc agc cat cat cat cat cat cac agc agc ggc ctg gtg ccg        48
Met Gly Ser Ser His His His His His His Ser Ser Gly Leu Val Pro
1               5                   10                  15
```

```
cgc ggc agc cat atg ctc gag ggt gtg ccc acg ctg aag gtc cag gtg        96
Arg Gly Ser His Met Leu Glu Gly Val Pro Thr Leu Lys Val Gln Val
            20                  25                  30

ccc aat gcc tcg gtg gat gtg ggg gac gac gtg ctg ctg cgg tgc cag       144
Pro Asn Ala Ser Val Asp Val Gly Asp Asp Val Leu Leu Arg Cys Gln
        35                  40                  45

gtg gag ggg cgg ggc ctg gag cag gcc ggc tgg atc ctc aca gag ctg       192
Val Glu Gly Arg Gly Leu Glu Gln Ala Gly Trp Ile Leu Thr Glu Leu
    50                  55                  60

gag cag tca gcc acg gtg atg aaa tct ggg ggt ctg cca tcc ctg ggg       240
Glu Gln Ser Ala Thr Val Met Lys Ser Gly Gly Leu Pro Ser Leu Gly
65                  70                  75                  80

ctg acc ctg gcc aat gtc acc agt gac ctc aac agg aag aac ttg acg       288
Leu Thr Leu Ala Asn Val Thr Ser Asp Leu Asn Arg Lys Asn Leu Thr
                85                  90                  95

tgc tgg gca gag aac gat gtg ggc cgg gca gag gtc tct gtt cag gtc       336
Cys Trp Ala Glu Asn Asp Val Gly Arg Ala Glu Val Ser Val Gln Val
            100                 105                 110

aac gtc tcc ttc tgataactcg agcg                                       362
Asn Val Ser Phe
        115


<210>  12
<211>  116
<212>  PRT
<213>  Artificial

<220>
<223>  Synthetic Construct

<400>  12

Met Gly Ser Ser His His His His His His Ser Ser Gly Leu Val Pro
1               5                   10                  15


Arg Gly Ser His Met Leu Glu Gly Val Pro Thr Leu Lys Val Gln Val
            20                  25                  30


Pro Asn Ala Ser Val Asp Val Gly Asp Asp Val Leu Leu Arg Cys Gln
        35                  40                  45


Val Glu Gly Arg Gly Leu Glu Gln Ala Gly Trp Ile Leu Thr Glu Leu
    50                  55                  60


Glu Gln Ser Ala Thr Val Met Lys Ser Gly Gly Leu Pro Ser Leu Gly
65                  70                  75                  80


Leu Thr Leu Ala Asn Val Thr Ser Asp Leu Asn Arg Lys Asn Leu Thr
                85                  90                  95


Cys Trp Ala Glu Asn Asp Val Gly Arg Ala Glu Val Ser Val Gln Val
            100                 105                 110


Asn Val Ser Phe
        115
```

```
<210>  13
<211>  449
<212>  DNA
<213>  Artificial

<220>
<223>  Nucleotide insert


<220>
<221>  CDS
<222>  (1)..(432)

<400>  13
atg ggc agc agc cat cat cat cat cat cac agc agc ggc ctg gtg ccg        48
Met Gly Ser Ser His His His His His His Ser Ser Gly Leu Val Pro
1               5                   10                  15

cgc ggc agc cat atg ccg gcc agt gtg cag ctg cac acg gcg gtg gag        96
Arg Gly Ser His Met Pro Ala Ser Val Gln Leu His Thr Ala Val Glu
            20                  25                  30

atg cac cac tgg tgc atc ccc ttc tct gtg gat ggg cag ccg gca ccg        144
Met His His Trp Cys Ile Pro Phe Ser Val Asp Gly Gln Pro Ala Pro
        35                  40                  45

tct ctg cgc tgg ctc ttc aat ggc tcc gtg ctc aat gag acc agc ttc        192
Ser Leu Arg Trp Leu Phe Asn Gly Ser Val Leu Asn Glu Thr Ser Phe
    50                  55                  60

atc ttc act gag ttc ctg gag ccg gca gcc aat gag acc gtg cgg cac        240
Ile Phe Thr Glu Phe Leu Glu Pro Ala Ala Asn Glu Thr Val Arg His
65                  70                  75                  80

ggg tgt ctg cgc ctc aac cag ccc acc cac gtc aac aac ggc aac tac        288
Gly Cys Leu Arg Leu Asn Gln Pro Thr His Val Asn Asn Gly Asn Tyr
                85                  90                  95

acg ctg ctg gct gcc aac ccc ttc ggc cag gcc tcc gcc tcc atc atg        336
Thr Leu Leu Ala Ala Asn Pro Phe Gly Gln Ala Ser Ala Ser Ile Met
            100                 105                 110

gct gcc ttc atg gac aac cct ttc gag ttc aac ccc gag gac ccc atc        384
Ala Ala Phe Met Asp Asn Pro Phe Glu Phe Asn Pro Glu Asp Pro Ile
        115                 120                 125

cct gac act aac agc aca tct gga gac ccg gtg gag aag aag gac gaa        432
Pro Asp Thr Asn Ser Thr Ser Gly Asp Pro Val Glu Lys Lys Asp Glu
    130                 135                 140

tgataactcg agatcgg                                                     449


<210>  14
<211>  144
<212>  PRT
<213>  Artificial

<220>
<223>  Synthetic Construct

<400>  14

Met Gly Ser Ser His His His His His His Ser Ser Gly Leu Val Pro
1               5                   10                  15
```

```
Arg Gly Ser His Met Pro Ala Ser Val Gln Leu His Thr Ala Val Glu
            20                  25                  30

Met His His Trp Cys Ile Pro Phe Ser Val Asp Gly Gln Pro Ala Pro
        35                  40                  45

Ser Leu Arg Trp Leu Phe Asn Gly Ser Val Leu Asn Glu Thr Ser Phe
    50                  55                  60

Ile Phe Thr Glu Phe Leu Glu Pro Ala Ala Asn Glu Thr Val Arg His
65                  70                  75                  80

Gly Cys Leu Arg Leu Asn Gln Pro Thr His Val Asn Asn Gly Asn Tyr
                85                  90                  95

Thr Leu Leu Ala Ala Asn Pro Phe Gly Gln Ala Ser Ala Ser Ile Met
            100                 105                 110

Ala Ala Phe Met Asp Asn Pro Phe Glu Phe Asn Pro Glu Asp Pro Ile
        115                 120                 125

Pro Asp Thr Asn Ser Thr Ser Gly Asp Pro Val Glu Lys Lys Asp Glu
    130                 135                 140


<210>  15
<211>  467
<212>  DNA
<213>  Artificial

<220>
<223>  Nucleotide insert


<220>
<221>  CDS
<222>  (1)..(450)

<400>  15
atg ggc agc agc cat cat cat cat cat cac agc agc ggc ctg gtg ccg       48
Met Gly Ser Ser His His His His His His Ser Ser Gly Leu Val Pro
1               5                   10                  15

cgc ggc agc cat atg ccg gcc agt gtg cag ctg cac acg gcg gtg gag       96
Arg Gly Ser His Met Pro Ala Ser Val Gln Leu His Thr Ala Val Glu
            20                  25                  30

atg cac cac tgg tcg atc ccc ttc tct gtg gat ggg cag ccg gca ccg      144
Met His His Trp Ser Ile Pro Phe Ser Val Asp Gly Gln Pro Ala Pro
        35                  40                  45

tct ctg cgc tgg ctc ttc aat ggc tcc gtg ctc aat gag acc agc ttc      192
Ser Leu Arg Trp Leu Phe Asn Gly Ser Val Leu Asn Glu Thr Ser Phe
    50                  55                  60

atc ttc act gag ttc ctg gag ccg gca gcc aat gag acc gtg cgg cac      240
Ile Phe Thr Glu Phe Leu Glu Pro Ala Ala Asn Glu Thr Val Arg His
65                  70                  75                  80

ggg tgt ctg cgc ctc aac cag ccc acc cac gtc aac aac ggc aac tac      288
Gly Cys Leu Arg Leu Asn Gln Pro Thr His Val Asn Asn Gly Asn Tyr
                85                  90                  95
```

```
acg ctg ctg gct gcc aac ccc ttc ggc cag gcc tcc gcc tcc atc atg        336
Thr Leu Leu Ala Ala Asn Pro Phe Gly Gln Ala Ser Ala Ser Ile Met
            100                 105                 110

gct gcc ttc atg gac aac cct ttc gag ttc aac ccc gag gac ccc atc        384
Ala Ala Phe Met Asp Asn Pro Phe Glu Phe Asn Pro Glu Asp Pro Ile
        115                 120                 125

cct gtc tcc ttc tcg cca gtg gac act aac agc aca tct gga gac ccg        432
Pro Val Ser Phe Ser Pro Val Asp Thr Asn Ser Thr Ser Gly Asp Pro
    130                 135                 140

gtg gag aag aag gac gaa tgataactcg agatcgg                             467
Val Glu Lys Lys Asp Glu
145                 150


<210>  16
<211>  150
<212>  PRT
<213>  Artificial

<220>
<223>  Synthetic Construct

<400>  16

Met Gly Ser Ser His His His His His His Ser Ser Gly Leu Val Pro
1               5                   10                  15

Arg Gly Ser His Met Pro Ala Ser Val Gln Leu His Thr Ala Val Glu
            20                  25                  30

Met His His Trp Ser Ile Pro Phe Ser Val Asp Gly Gln Pro Ala Pro
        35                  40                  45

Ser Leu Arg Trp Leu Phe Asn Gly Ser Val Leu Asn Glu Thr Ser Phe
    50                  55                  60

Ile Phe Thr Glu Phe Leu Glu Pro Ala Ala Asn Glu Thr Val Arg His
65                  70                  75                  80

Gly Cys Leu Arg Leu Asn Gln Pro Thr His Val Asn Asn Gly Asn Tyr
                85                  90                  95

Thr Leu Leu Ala Ala Asn Pro Phe Gly Gln Ala Ser Ala Ser Ile Met
            100                 105                 110

Ala Ala Phe Met Asp Asn Pro Phe Glu Phe Asn Pro Glu Asp Pro Ile
        115                 120                 125

Pro Val Ser Phe Ser Pro Val Asp Thr Asn Ser Thr Ser Gly Asp Pro
    130                 135                 140

Val Glu Lys Lys Asp Glu
145                 150
```